# EUROPEAN PATENT SPECIFICATION

(11) **EP 1 996 591 B1**
(45) Date of publication and mention of the grant of the patent: **12.01.2011**
(21) Application number: 07726742.5
(22) Date of filing: 09.03.2007
(51) Int. Cl.: C07D 471/10, C07D 471/20, A61P 35/04

(54) **SPIROINDOLINONE DERIVATIVES**
SPIROINDOLINON-DERIVATE
DÉRIVÉS DE SPIROINDOLINONE

(30) Priority: 13.03.2006 US 781958 P; 07.02.2007 US 899987 P
(43) Date of publication of application: 03.12.2008
(73) Proprietor: F.Hoffmann-La Roche AG, 4070 Basel (CH)
(72) Inventor: DING, Qingjie, Bridgewater, New Jersey 08807 (US); LIU, Jin-Jun, Warren, New Jersey 07059 (US); ZHANG, Zhuming, Hillsborough, New Jersey 08844 (US)
(74) Representative: Klein, Thomas
(86) International application number: PCT/EP2007/052220
(87) International publication number: WO 2007/104714

(56) References cited:
- WO-A1-01/05790

## Description

The present invention relates to spiroindolinone derivatives of the formula 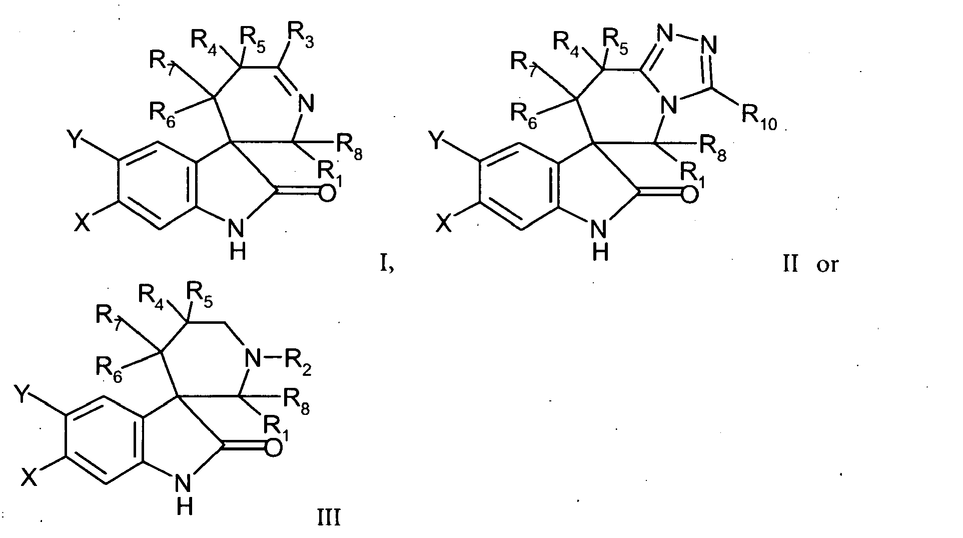 and enantiomers and pharmaceutically acceptable salts and esters thereof wherein R₁, R_{2,} R₃, R₄, R₅, R₆, R₇, R₈, R₉, R₉, R₁₀, are as herein described.

The compounds have utility as antiproliferative agents, especially, as anticancer agents.

p53 is a tumor suppresser protein that plays a central role in protection against development of cancer. It guards cellular integrity and prevents the propagation of permanently damaged clones of cells by the induction of growth arrest or apoptosis. At the molecular level, p53 is a transcription factor that can activate a panel of genes implicated in the regulation of cell cycle and apoptosis. p53 is a potent cell cycle inhibitor which is tightly regulated by MDM2 at the cellular level. MDM2 and p53 form a feedback control loop. MDM2 can bind p53 and inhibit its ability to transactivate p53-regulated genes. In addition, MDM2 mediates the ubiquitin-dependent degradation of p53. p53 can activate the expression of the MDM2 gene, thus raising the cellular level of MDM2 protein. This feedback control loop insures that both MDM2 and p53 are kept at a low level in normal proliferating cells. MDM2 is also a cofactor for E2F, which plays a central role in cell cycle regulation.

The ratio of MDM2 to p53 (E2F) is dysregulated in many cancers. Frequently occurring molecular defects in the p16INK4/p19ARF locus, for instance, have been shown to affect MDM2 protein degradation. Inhibition of MDM2-p53 interaction in tumor cells with wild-type p53 should lead to accumulation of p53, cell cycle arrest and/or apoptosis. MDM2 antagonists, therefore, can offer a novel approach to cancer therapy as single agents or in combination with a broad spectrum of other antitumor therapies. The feasibility of this strategy has been shown by the use of different macromolecular tools for inhibition of MDM2-p53 interaction (e.g. antibodies, antisense oligonucleotides, peptides). MDM2 also binds E2F through a conserved binding region as p53 and activates E2F-dependent transcription of cyclin A, suggesting that MDM2 antagonists might have effects in p53 mutant cells.

A series of spiroindolinone as antagonists of MDM2 has previously been disclosed in J. Am Chem. Soc., 2005, 127, 10130.

The present invention provides spiroindolinone derivatives which are small molecule inhibitors of the MDM2-p53 interaction. In cell-free and cell-based assays, compounds of the present invention are shown to inhibit the interaction of MDM2 protein with a p53-like peptide. In cell-based assays, these compounds demonstrate mechanistic activity. Incubation of cancer cells with wild-type p53 leads to accumulation of p53 protein, induction of p53-regulated p21 gene, and cell cycle arrest in G1 and G2 phase, resulting in potent antiproliferative activity against wild-type p53 cells in vitro. In contrast, these activities were not observed in cancer cells with mutant p53 at comparable compound concentrations. Therefore, the activity of MDM2 antagonists is likely linked to its mechanism of action. These compounds can be potent and selective anticancer agents.

The present invention relates to 3,3'-spiroindolinones of the formulas 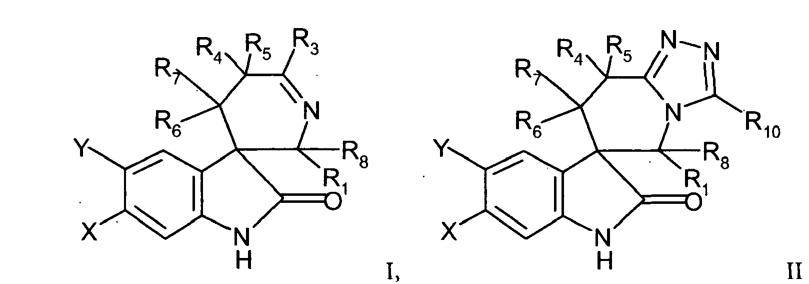 or 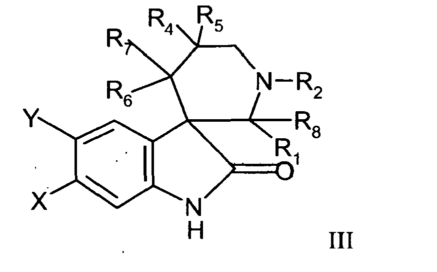 wherein
X is selected from the group consisting of hydrogen, halogen, cyano, nitro, ethynyl, cyclopropyl, methyl, ethyl, isopropyl, methoxy, and vinyl,
Y is hydrogen or fluorine,
R₄, R₅ are selected from the group consisting of hydrogen and lower alkyl
one of R₁ and R₈ is selected from the group consisting of lower alkyl,
substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl, and substituted cycloalkenyl and the other is hydrogen,
one of R₆ and R₇ is selected from the group consisting of lower alkyl, substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl, and substituted cycloalkenyl and the other is hydrogen, lower alkyl or cyano,
R₂ is selected from the group consisting of hydrogen, lower alkyl, substituted lower alkyl, C(=O)R₉, C(=O)NHR₉, C(=O)NR₉R_{9'} and C(=O)OR₉,
R₃ is selected from the group consisting of NHR₉, SR₉, and NR₉R₉,
R₉ is selected from the group consisting of lower alkyl, substituted lower alkyl, aryl, substituted aryl, heteroaryl, substituted heteroaryl, heterocycle, substituted heterocycle, cycloalkyl and substituted cycloalkyl,
R₉ is selected from the group consisting of lower alkyl and substituted lower alkyl and in the case of R₉ and R₉ they may independently link to form a cyclic structure selected from heterocycle or substituted heterocycle,
R₁₀ is selected from the group consisting of hydrogen, hydroxyl and lower alkyl and the pharmaceutically acceptable salts and esters thereof.

Preferred are compounds of formula I having a stereochemical structure shown as formula IV 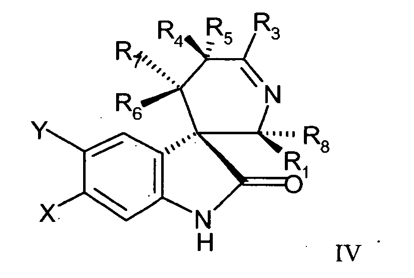 wherein
X is Cl or Br,
Y is hydrogen,
R₁ is hydrogen,
R₄ and R₅ are both hydrogen,
R₆ is hydrogen,
R₇ is a substituted phenyl or substituted heteroaryl with the substituted phenyl or substituted heteroaryl selected from group consisting of 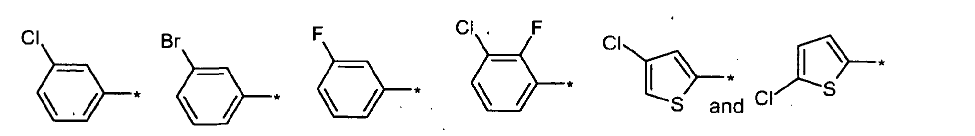
R₈ is selected from the group consisting of lower alkyl, substituted lower alkyl, lower alkenyl, substituted lower alkenyl, aryl, substituted aryl, heteroaryl, substituted heteroaryl, cycloalkyl, substituted cycloalkyl, cycloalkenyl and substituted cycloalkenyl,
R₃ is selected from the group consisting of NHR₉ and NR₉R_{9,}
R₉ is selected from the group consisting of lower alkyl, substituted lower alkyl, aryl, substituted aryl, heteroaryl, substituted heteroaryl, heterocycle, substituted heterocycle, cycloalkyl and substituted cycloalkyl,
R_{9'} is selected from the group consisting of lower alkyl and substituted lower alkyl and in the case of R₉ and R₉ they may independently link to form a cyclic structure selected from heterocycle or substituted heterocycle
and the pharmaceutically acceptable salts and esters thereof.

Also preferred are compounds of formula II having a stereochemical structure shown as formula V 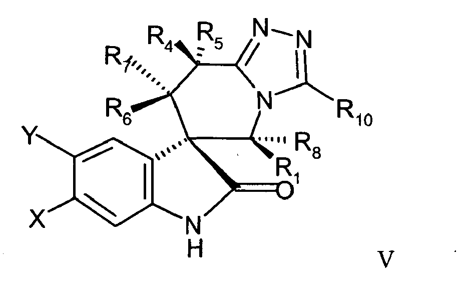 wherein
X is Cl or Br,
Y is hydrogen,
R₁ is hydrogen,
R₄ and R₅ are both hydrogen,
R₆ is hydrogen,
R₇ is a substituted phenyl or substituted heteroaryl with the substituted phenyl or substituted heteroaryl selected from group consisting of 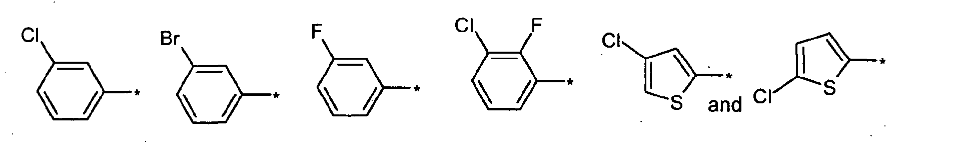
R₈ is selected from the group consisting of lower alkyl, substituted lower alkyl, lower alkenyl, substituted lower alkenyl, aryl, substituted aryl, heteroaryl, substituted heteroaryl, cycloalkyl, substituted cycloalkyl, cycloalkenyl and substituted cycloalkenyl,
R₁₀ is hydrogen, hydroxyl, and methyl
and the pharmaceutically acceptable salts and esters thereof.

Also preferred are compounds of formula III having a stereochemical structure shown as formula VI 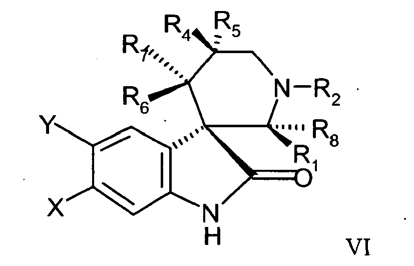 wherein
X is Cl or Br,
is hydrogen,
R₁ is hydrogen,
R₄ and R₅ are both hydrogen,
R₆ is hydrogen,
R₇ is a substituted phenyl or substituted heteroaryl with the substituted phenyl or substituted heteroaryl selected from group consisting of 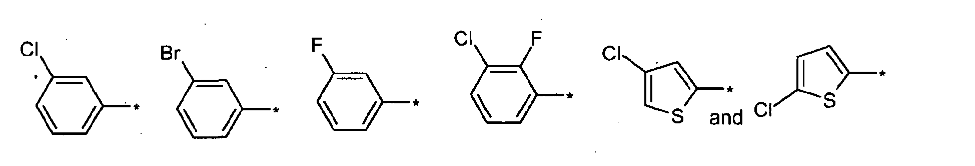
R₈ is selected from the group consisting of lower alkyl, substituted alkyl, lower alkenyl, substituted lower alkenyl, aryl, substituted aryl, heteroaryl, substituted heteroaryl, cycloalkyl, substituted cycloalkyl, cycloalkenyl and substituted cycloalkenyl,
R₂ is selected from the group consisting of hydrogen, lower alkyl, substituted lower alkyl, C(=O)R₉, C(=O)NHR₉, C(=O)NR₉R_{9'} and C(=O)OR₉,
R₉ is selected from the group consisting of lower alkyl, substituted lower alkyl, aryl, substituted aryl, heteroaryl, substituted heteroaryl, heterocycle, cycloalkyl and substituted cycloalkyl,
R₉ is selected from the group consisting of lower alkyl and substituted lower alkyl and in the case of R₉ and R_{9'} they may independently link to form a cyclic structure selected from heterocycle and substituted heterocycle
and the pharmaceutically acceptable salts and esters thereof.

Other preferred are compounds of formula III having a stereochemical structure shown as formula VII 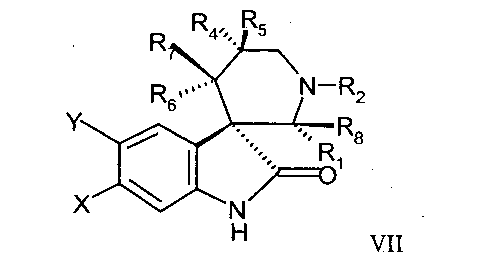 wherein
X is Cl or Br,
Y is hydrogen,
R₁ is hydrogen,
R₄ and R₅ are both hydrogen,
R₆ is hydrogen,
R₈ is a substituted phenyl with the substituted phenyl selected from group consisting of 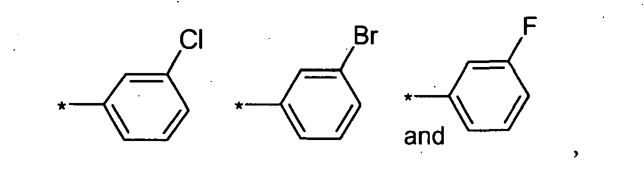
R₇ is selected from the group consisting of lower alkyl, substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl and substituted cycloalkenyl,
R₂ is selected from the group consisting of hydrogen, lower alkyl, substituted lower alkyl, C(=O)R₉, C(=O)NHR₉, C(=O)NR₉R_{9'} and C(=O)OR₉,
R₉ is selected from the group consisting of lower alkyl, substituted lower alkyl, aryl, substituted aryl, heteroaryl, substituted heteroaryl, heterocycle, cycloalkyl and substituted cycloalkyl,
R₉ is selected from the group consisting of lower alkyl and substituted lower alkyl and in the case of R₉ and R_{9'} they may independently link to form a cyclic structure selected from heterocycle and substituted heterocycle
and the pharmaceutically acceptable salts and esters thereof.

Especially preferred compounds are those of the formula
racemic (2'S, 3S, 4'S)-6-chloro-2'-(3-chlorophenyl)-4'-(2,2-dimethylpropyl) spiro[3H-indole-3,3'-piperidine]-2(1H)-one,
racemic (2'S, 3R)-6-chloro-2'-(3-chlorophenyl)-2',3',4',5'-tetrahydro-4'-isopropyl-6'-(methylthio) spiro[3H-indole-3,3'-pyridin]-2(1H)-one,
racemic (2'S, 3S, 4'S)-6-chloro-2'-(3-chlorophenyl)-4'-ethyl spiro[3H-indole-3,3'-piperidine]-2(1H)-one,
racemic (2'R, 3R, 4'S)-6-chloro-4'-(3-chlorophenyl)-2'-(3-methylphenyl) spiro[3H-indole-3,3'-piperidine]-2(1H)-one,
racemic (2'R, 3R, 4'S)-6-chloro-4'-(3-chlorophenyl)-2'-(3-fluorophenyl)spiro[3H-indole-3,3'-piperidine]-2(1H)-one,
racemic (2'S, 3S, 4'R)-6-chloro-2'-(3-chlorophenyl)-4'-phenyl spiro[3H-indole-3,3'-piperidine]-2(1H)-one,
racemic (2'R, 3R, 4'S, 5'S)-6-chloro-4'-(3-chlorophenyl)-5'-methyl-2'-(2-methylphenyl) spiro[3H-indole-3,3'-piperidine]-2(1H)-one,
racemic (2'R, 3R, 4'S)-6-chloro-4'-(3-chlorophenyl)-2'-(3-methoxyphenyl) spiro[3H-indole-3,3'-piperidine]-2(1H)-one,
racemic (2'R, 3R, 4'S)-6-chloro-4'-(3-chlorophenyl)-2'-(3-cyanophenyl) spiro[3H-indole-3,3'-piperidine]-2(1H)-one,
racemic (2'R, 3R, 4'S)-6-chloro-4'-(3-chlorophenyl)-2'-(2, 3-dimethylphenyl) spiro[3H-indole-3,3'-piperidine]-2(1H)-one,
racemic (2'R, 3R, 4'S)-6-chloro-4'-(3-chlorophenyl)-2'-[2-(trifluoromethyl)-phenyl)] spiro [3H-indole-3,3'-piperidine]-2(1H)-one,
racemic (2'S, 3R)-6-chloro-4'-(3-chlorophenyl)-2',3',4',5'-tetrahydro-6'-(methylthio)-2'-[2-(trifluoromethyl)-phenyl] spiro[3H-indole-3,3'-pyridin]-2(1H)-one,
racemic (2'R, 3R, 4'S)-6-chloro-4'-(3-chlorophenyl)-2'-[5-fluoro-2-(trifluoromethyl)-phenyl] spiro[3H-indole-3,3'-piperidine]-2(1H)-one,
racemic (2'R, 3R, 4'S)-6-chloro-4'-(3-chlorophenyl)-2'-(5-fluoro-2-methylphenyl) spiro [3H-indole-3,3'-piperidine]-2(1H)-one,
racemic (2'R, 3R, 4'S)-6-chloro-4'-(3-chlorophenyl)-2'-(2, 4-difluorophenyl) spiro[3H-indole-3,3'-piperidine]-2(1H)-one,
racemic (2'R, 3R, 4'S)-6-chloro-4'-(3-chlorophenyl)-2'-(5-fluoro-2-methoxyphenyl) spiro [3H-indole-3,3'-piperidine]-2(1H)-one,
racemic (2'R, 3R, 4'S)-6-chloro-4'-(3-chlorophenyl)-2'-(1-naphthalenyl) spiro[3H-indole-3,3'-piperidine]-2(1H)-one,
racemic (2'R, 3R, 4'S)-6-chloro-4'-(3-chlorophenyl)-2'-(3-pyridinyl) spiro[3H-indole-3,3'-piperidine]-2(1H)-one,
racemic (2'R, 3R, 4'S)-6-chloro-4'-(3-chlorophenyl)-2'-(3, 4-difluoro-phenyl) spiro[3H-indole-3,3'-piperidine]-2(1H)-one,
racemic (5'R, 3R, 7'S)-6-chloro-7'-(3-chlorophenyl)-5'-(3, 4-difluorophenyl)-5',6',7',8'-tetrahydro-3'-hydroxyspiro[3H-indole-3,6'-(1,2,4-triazolo[4,3-a]pyridine)]-2(1H)- one,
racemic (5'R, 3R, 7'S)-6-chloro-7'-(3-chlorophenyl)-5'-(5-fluoro-2-methylphenyl)-5',6',7',8'-tetrahydro-3'-hydroxyspiro[3H-indole-3,6'-(1,2,4- triazolo[4,3-a]pyridine)]-2(1H)-one,
racemic (2'R, 3R, 4'S)-6-chloro-4'-(3-chlorophenyl)-2'-(2,3-difluoro-6-methylphenyl) spiro[3H-indole-3,3'-piperidine]-2(1H)-one,
racemic (2'R, 3R, 4'S)-6-chloro-4'-(3-chlorophenyl)-5-fluoro-2'-(5-fluoro-2-methylphenyl) spiro[3H-indole-3,3'-piperidine]-2(1H)-one,
racemic (2'R, 3R, 4'S)-6-chloro-4'-(3-chlorophenyl)-2'-(1-ethylpropyl) spiro[3H-indole-3,3'-piperidine]-2(1H)-one,
racemic (2'R, 3R, 4'S)-6-chloro-4'-(3-chloro-phenyl)-2'-(2,5-dimethyl-phenyl) spiro [3H-indole-3,3'-piperidine]-2(1H)-one,
racemic (2'R, 3R, 4'S)-6-chloro-4'-(3-chlorophenyl)-2'-(2,5-dimethyl-2H-pyrazole-3-yl) spiro[3H-indole-3,3'-piperidine] -2(1H)-one,
racemic (2'R, 3R, 4'S)-6-chloro-4'-(3-chlorophenyl)-1'-(morpholin-4-carbonyl)-2'-(1-naphthalenyl) spiro[3H-indole-3,3'-piperidine]-2(1H)-one,
racemic (2'S, 3S, 4'R)-6-chloro-4'-(4-chloro-phenyl)-2'-(3-fluoro-phenyl) spiro[3H-indole-3,3'-piperidine]-2(1H)-one,
racemic (2'S, 3S, 4'R)-1'-tert-butylaminocarbonyl-6-chloro-4'-(4-chloro-phenyl)-2'-(3-fluoro-phenyl) spiro[3H-indole-3,3'-piperidine]-2(1H)-one,
racemic (2'S, 3S, 4'R)-6-chloro-4'-(4-chloro-phenyl)-1'-(3-cyanophenylaminocarbonyl)-2'-(3-fluoro-phenyl) spiro[3H-indole-3,3'-piperidine]-2(1H)-one,
racemic (2'R, 3R, 4'S)-6-chloro-4'-(3-chloro-phenyl)-1'-(3-cyano= phenylaminocarbonyl)-2'-(3-fluoro-phenyl) spiro[3H-indole-3,3'-piperidine]-2(1H)-one,
racemic (2'R, 3R, 4'S)-1'-tert-butylaminocarbonyl-6-chloro-4'-(3-chloro-phenyl)-2'-(3-fluoro-phenyl) spiro[3H-indole-3,3'-piperidine]-2(1H)-one,
racemic (2'R, 3R, 4'S)-1'-benzoyl-6-chloro-4'-(3-chloro-phenyl)-2'-(3-fluoro-phenyl) spiro[3H-indole-3,3'-piperidine]-2(1H)-one,
racemic (2'R, 3R, 4'S)-1'-acetyl-6-chloro-4'-(3-chloro-phenyl)-2'-(3-fluoro-phenyl) spiro [3H-indole-3,3'-piperidine]-2(1H)-one,
racemic (2'R, 3R, 4'S)-1'-[4-(amonocarbonylmethyl)-piperazine-1-carbonyl]-6-chloro-4'-(3-chloro-phenyl)-2'-,(3-fluoro-phenyl) spiro[3H-indole-3,3'-piperidirie]-2(1H)-one,
racemic (2'R, 3R, 4'S)-6-chloro-4'-(3-chloro-phenyl)-2'-(3-fluoro-phenyl)-1'-[4-(3-methanesulfonyl-propyl)-piperazine-1-carbonyl] spiro[3H-indole-3,3'-piperidine]-2(1H)-one,
racemic (2'R, 3R, 4'S)-6-chloro-4'-(3-chloro-phenyl)-2'-(3-fluoro-phenyl)-1'-[4-(2-oxo-2-pyrrolidin-1-yl-ethyl)-piperazine-1-carbonyl] spiro[3H-indole-3,3'-piperidine]-2(1H)-one,
racemic (2'R, 3R, 4'S)-1'-[4-(2-acetylamino-ethyl)-piperazine-1-carbonyl]-6-chloro-4'-(3-chloro-phenyl)-2'-(3-fluoro-phenyl) spiro[3H-indole-3,3'-piperidine]-2(1H)-one,
racemic (2'R, 3R, 4'S)-6-chloro-4'-(3-chloro-phenyl)-2'-(3-fluoro-phenyl)-1'-[4-(2-hydroxy-ethyl)-piperazine-1-carbonyl] spiro[3H-indole-3,3'-piperidine]-2(1H)-one,
racemic (2'R, 3R, 4'S)-6-chloro-4'-(3-chloro-phenyl)-1'-cyclohexylaminocarbonyl-2'-(3-fluoro-phenyl) spiro[3H-indole-3,3'-piperidine]-2(1H)-one,
racemic (2'R, 3R, 4'S)-1'-benzylaminocarbonyl-6-chloro-4'-(3-chloro-phenyl)-2'-(3-fluoro-phenyl) spiro[3H-indole-3,3'-piperidine]-2(1H)-one,
racemic (5'R, 3R, 7'S)-6-chloro-7'-(3-chlorophenyl)-5'-(2,3-difluoro-6-isopropoxyphenyl)-5',6',7',8'-tetrahydro-3'-hydroxyspiro[3H-indole-3,6'-(1,2,4-triazolo [4,3-a]pyridine)]-2(1H)-one,
chiral (5'R, 3R, 7'S)-6-chloro-7'-(3-chlorophenyl)-5'-(2,3-difluoro-6-isopropoxyphenyl)-5',6',7',8'-tetrahydro-3'-hydroxyspiro[3H-indole-3,6'-(1,2,4-triazolo [4,3-a] pyridine)]-2(1H)-one,
racemic (2'R, 3R, 4'S)-1'-hydroxycarbonylmethyl-6-chloro-4'-(3-chloro-phehyl)-2'-(3-fluoro-phenyl) spiro[3H-indole-3,3'-piperidine]-2(1H)-one,
racemic (5'R, 3R, 7'S)-6-chloro-7'-(3-chlorophenyl)-5'-(1-methylene-butyl)-5',6',7',8'-tetrahydro-spiro[3H-indole-3,6'-(1,2,4-triazolo[4,3-a]pyridine)]-2(1H)-one,
chiral (5'R, 3R, 7'S)-6-chloro-7'-(3-chlorophenyl)-5'-(1-methylene-butyl)-5',6',7',8'-tetrahydro-spiro[3H-indole-3,6'-(1,2,4-triazolo[4,3-a]pyridine)]-2(1H)-one,
racemic (5'R, 3R, 7'S)-6-chloro-7'-(3-chlorophenyl)-3'-methyl-5'-(1-methylene-butyl)-5',6',7',8'-tetrahydro-spiro[3H-indole-3,6'-(1,2,4-triazolo[4,3-a]pyridine)] -2(1H)-one,
racemic (2'R, 3'R,4'S)-6-chloro-4'-(3-chlorophenyl)-2'-(5-fluoro-2-methyl-phenyl)-6'-(1,4-piperazinyl)-spiro[3H-indole-3,3'-2',3',4',5'-tetrahydro-pyridine]-2-one,
racemic (2'R, 3'R,4'S)-6-chloro-4'-(3-chlorophenyl)-2'-(5-fluoro-2-methyl-phenyl)-6'-[4-[1,1-dimethylethoxy-carbonyl]amino]ethylamino-spiro[3H-indole-3,3'-2',3',4',5'-tetrahydro-pyridine]-2-one,
racemic (2'R, 3'R,4'S)-6-chloro-4'-(3-chlorophenyl)-2'-(5-fluoro-2-methyl-phenyl)-6'-[4-amino]ethylamino-spiro[3H-indole-3,3'-2',3',4',5'-tetrahydro-pyridine] -2-one, trifluoroacetic acid,
racemic (2'R, 3'R,4'S)-6-chloro-4'-(3-chlorophenyl)-2'-(5-fluoro-2-methyl-phenyl)-6'-(4-methyl-1-piperazinyl)-spiro[3H-indole-3,3'-2',3',4',5'-tetrahydro-pyridine]-2-one,
racemic (2'R, 3'R,4'S)-6-chloro-4'-(3-chlorophenyl)-2'-(5-fluoro-2-methyl-phenyl)-6'-[[tetrahydro-1,1-dioxido-2H-thiopyran-4-yl]piperazinyl-spiro[3H-indole-3,3'-2',3',4',5'-tetrahydro-pyridine]-2-one,
racemic (2'R, 3'R,4'S)-6-chloro-4'-(3-chlorophenyl)-2'-(5-fluoro-2-methyl-phenyl)-6'-[4-(3-methylsulphonyl)-propyl]piperazinyl-spiro[3H-indole-3,3'-2',3',4',5'-tetrahydro-pyridine]-2-one and
racemic (2'R, 3'R,4'S)-6-chloro-4'-(3-chlorophenyl)-2'-(5-fluoro-2-methyl-phenyl)-6'- . [1-methylsulphonyl-4-piperidinyl]amino-spiro[3H-indole-3,3'-2',3',4',5'-tetrahydro-pyridine]-2-one.

In the specification where indicated the various groups may be substituted by 1-5 or, preferably, 1-3 substituents independently selected from the group consisting of lower alkyl, lower-alkenyl, lower-alkynyl, dioxo-lower-alkylene (forming e.g. a benzodioxyl group), halogen, hydroxy, CN; CF₃, NH₂, N(H, lower-alkyl), N(lower-alkyl)₂, aminocarbonyl, carboxy, NO₂, lower-alkoxy, thio-lower-alkoxy, lower-alkylsufonyl, aminosulfonyl, lower-alkylcarbonyl, lower-alkylcarbonyloxy, lower-alkoxycarbonyl, lower-alkyl-carbonyl-NH, fluoro-lower-alkyl, fluoro-lower-alkoxy, lower-alkoxycarbonyl-lower-alkoxy, carboxy-lower-alkoxy, carbamoyl-lower-alkoxy, hydroxy-lower-alkoxy, NH₂-lower-alkoxy, N(H, lower-alkyl)-lower-alkoxy, N(lower- alkyl)₂- lower-alkoxy, benzyloxy-lower-alkoxy, mono- or di-lower alkyl substituted amino-sulfonyl and lower-alkyl which can optionally be substituted with halogen, hydroxy, NH₂, N(H, lower-alkyl) or N(lower-alkyl)₂. Preferred substituents for the aryl, heteroaryl and heterocycle rings are halogen, lower alkoxy, lower alkyl and amino.

If alkyl, alkenyl, alkynyl or similar groups are linked with both ends to the same moiety, cyclic structures may result, where two hydrogens of said moiety are being replaced by the two ends of the alkyl, alkenyl, alkynyl or similar group, thus creating cyclic structures, as in tetralin, macrocycles or spiro compounds.

The term "alkyl" refers to straight- or branched-chain saturated hydrocarbon groups having from 1 to about 20 carbon atoms, including groups having from 1 to about 7 carbon atoms. In certain embodiments, alkyl substituents may be lower alkyl substituents. The term "lower alkyl" refers to alkyl groups having from 1 to 6 carbon atoms, and in certain embodiments from 1 to 4 carbon atoms. Examples of alkyl groups include, but are not limited to, methyl, ethyl, n-propyl, i-propyl, n-butyl, s-butyl, t-butyl, n-pentyl, and s-pentyl.

As used herein, "cycloalkyl" is intended to refer to any stable monocyclic or polycyclic system which consists of carbon atoms only, any ring of which being saturated, and the term "cycloalkenyl" is intended to refer to any stable monocyclic or polycyclic system which consists of carbon atoms only, with at least one ring thereof being partially unsaturated. Examples of cycloalkyls include, but are not limited to, cyclopropyl, cyclobutyl, cyclopentyl, cyclohexyl, cycloheptyl, adamantyl, cyclooctyl, bicycloalkyls, including bicyclooctanes such as [2.2.2]bicyclooctane or [3.3.0]bicyclooctane, bicyclononanes such as [4.3.0]bicyclononane, and bicyclodecanes such as [4.4.0]bicyclodecane (decalin), or spiro compounds. Examples of cycloalkenyls include, but are not limited to, cyclopentenyl or cyclohexenyl.

The term "alkenyl" as used herein means an unsaturated straight-chain or branched aliphatic hydrocarbon group containing one double bond and having 2 to 6, preferably 2 to 4 carbon atoms. Examples of such "alkenyl group" are vinyl (ethenyl), allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl and 5-hexenyl.

The term "alkynyl" as used herein means an unsaturated straight-chain or branched aliphatic hydrocarbon group containing one triple bond and having 2 to 6, preferably 2 to 4 carbon atoms. Examples of such "alkynyl group" are ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl and 5-hexynyl.

The term "halogen" as used in the definitions means fluorine, chlorine or bromine, preferably fluorine and chlorine.

"Aryl" means a monovalent, monocyclic or bicyclic, aromatic carbocyclic hydrocarbon radical, preferably a 6-10 member aromatic ring system. Preferred aryl groups include, but are not limited to, phenyl, naphthyl, tolyl, and xylyl.

"Heteroaryl" means an aromatic heterocyclic ring system containing up to two rings. Preferred heteroaryl groups include, but are not limited to, thienyl, furyl, indolyl, pyrrolyl, pyridinyl, pyrazinyl, oxazolyl, thiaxolyl, quinolinyl, pyrimidinyl, imidazole and tetrazolyl.

In the case of aryl or heteroaryl which are bicyclic it should be understood that one ring may be aryl while the other is heteroaryl and both being substituted or unsubstituted.

"Heterocycle" means a substituted or unsubstituted 5 to 8 membered, mono- or bicyclic, aromatic or non-aromatic hydrocarbon, wherein 1 to 3 carbon atoms are replaced by a hetero atom selected from nitrogen,oxygen or sulfur atom. Examples include pyrrolidin-2-yl; pyrrolidin-3-yl; piperidinyl; morpholin-4-yl and the like.

"Hetero atom" means an atom selected from N, O and S.

"Alkoxy, alkoxyl or lower alkoxy" refers to any of the above lower alkyl groups attached to an oxygen atom. Typical lower alkoxy groups include methoxy, ethoxy, isopropoxy or propoxy, butyloxy and the like. Further included within the meaning of alkoxy are multiple alkoxy side chains, e.g. ethoxy ethoxy, methoxy ethoxy, methoxy ethoxy ethoxy and the like and substituted alkoxy side chains,e.g., dimethylamino ethoxy, diethylamino ethoxy, dimethoxy-phosphoryl methoxy and the like.

"Pharmaceutically acceptable," such as pharmaceutically acceptable carrier, excipient, etc., means pharmacologically acceptable and substantially non-toxic to the subject to which the particular compound is administered.

"Pharmaceutically acceptable salt" refers to conventional acid-addition salts or base-addition salts that retain the biological effectiveness and properties of the compounds of the present invention and are formed from suitable non-toxic organic or inorganic acids or organic or inorganic bases. Sample acid-addition salts include those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, trifluoro acetic acid and the like. Sample base-addition salts include those derived from ammonium, potassium, sodium and, quaternary ammonium hydroxides, such as for example, tetramethylammonium hydroxide. Chemical modification of a pharmaceutical compound (i.e. drug) into a salt is a technique well known to pharmaceutical chemists to obtain improved physical and chemical stability, hygroscopicity, flowability and solubility of compounds. *See, e.g.,* Ansel et al., Pharmaceutical Dosage Forms and Drug Delivery Systems (6th Ed. 1995) at pp. 196 and 1456-1457.

The compounds of formulas I- VII as well as their salts have at least one asymmetric carbon atom and therefore may be present as racemic mixtures or different stereoisomers. The various isomers can be isolated by known separation methods, e.g., chromatography.

The compounds of the present invention are useful in the treatment or control of cell proliferative disorders, in particular oncological disorders. These compounds and formulations containing said compounds may be useful in the treatment or control of solid tumors, such as, for example, breast, colon, lung and prostate tumors.

A therapeutically effective amount of a compound in accordance with this invention means an amount of compound that is effective to prevent, alleviate or ameliorate symptoms of disease or prolong the survival of the subject being treated. Determination of a therapeutically effective amount is within the skill in the art.

The therapeutically effective amount or dosage of a compound according to this invention can vary within wide limits and may be determined in a manner known in the art. Such dosage will be adjusted to the individual requirements in each particular case including the specific compound(s) being administered, the route of administration, the condition being treated, as well as the patient being treated. In general, in the case of oral or parenteral administration to adult humans weighing approximately 70 Kg, a daily dosage of about 10 mg to about 10,000 mg, preferably from about 200 mg to about 1,000 mg, should be appropriate, although the upper limit may be exceeded when indicated. The daily dosage can be administered as a single dose or in divided doses, or for parenteral administration, it may be given as continuous infusion.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, as well as the particular mode of administration. The amount of active ingredient which can be combined with a carrier material to produce a single dosage form will generally be that amount of a formula I-VII compound which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, sachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

"Effective amount" means an amount that is effective to prevent, alleviate or ameliorate symptoms of disease or prolong the survival of the subject being treated.

" IC₅₀" refers to the concentration of a particular compound required to inhibit 50% of a specific measured activity. IC₅₀ can be measured, *inter alia,* as is described subsequently.

"Pharmaceutically acceptable ester" refers to a conventionally esterified compound of formulas I-VII having a carboxyl group or hydroxy group, which esters retain the biological effectiveness and properties of the compounds of formulas I-IV and are cleaved *in vivo* (in the organism) to the corresponding active carboxylic acid or alcohol respectively.

### Synthesis

The invention concerns a process to produce a compound according to the invention, preferably of the formula 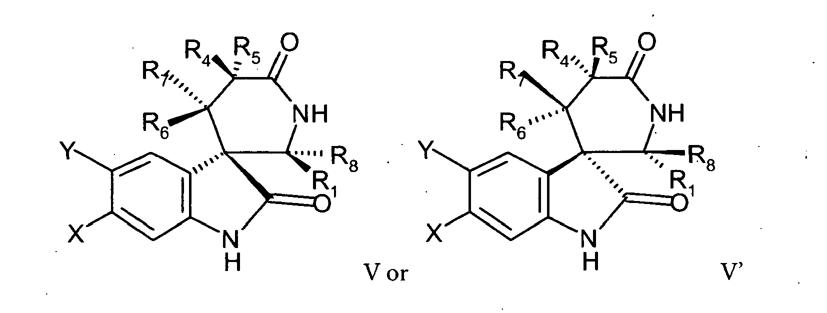 wherein
X is selected from the group consisting of hydrogen, halogen, cyano, nitro, cyclopropyl, methyl, ethyl, and isopropyl;
Y is hydrogen or fluorine;
R₁ is hydrogen;
R₄ and R₅ are hydrogen or lower alkyl;
R₈ is selected from the group consisting of lower alkyl, substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl, and substituted cycloalkenyl;
one of R₆ and R₇ are selected from the group consisting of lower alkyl, substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl, and substituted cycloalkenyl and the other is hydrogen, cyano or lower alkyl
   which comprises reacting a compound of the formula 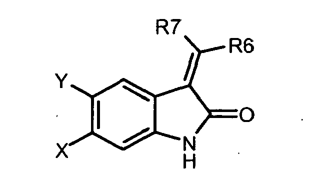 with a compound of the formula 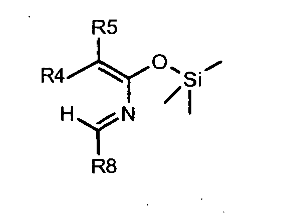 at about 110-160°C and under anhydrous conditions to produce a compound of formula VorV'.

The invention also concerns a process to produce a compound according to the invention, preferably of the formula 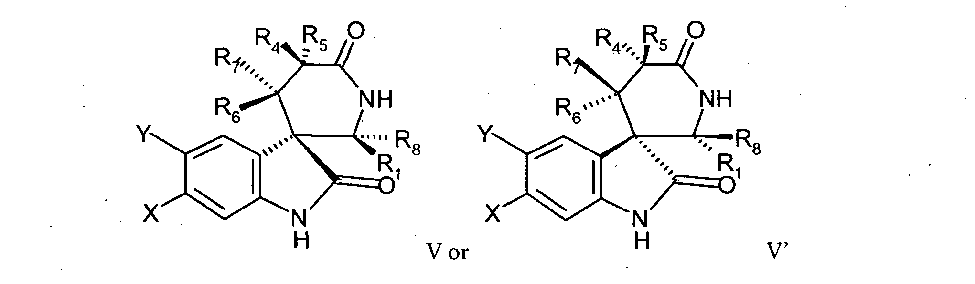 wherein
X is selected from the group consisting of hydrogen, halogen, cyano, nitro, cyclopropyl, methyl, ethyl, and isopropyl;
Y is hydrogen or fluorine;
R₁ is hydrogen;
R₄ and R₅ are hydrogen or lower alkyl;
R₈ is selected from the group consisting of lower alkyl, substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl, and substituted cycloalkenyl;
one of R₆ and R₇ are selected from the group consisting of lower alkyl, substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl, and substituted cycloalkenyl and the other is hydrogen, cyano or lower alkyl
which comprises reacting a compound of the formula 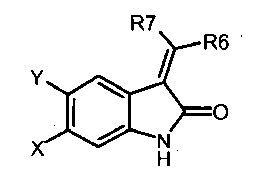 with a suitable protecting group (Pg) to provide a compound of the formula 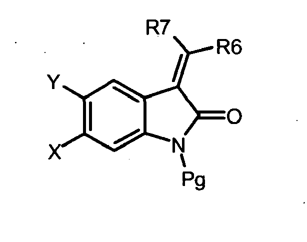 which is thereafter reacted with a compound of the formula 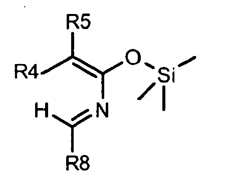 at about 110-160°C under anhydrous conditions and thereafter deprotecting the resultant product to produce a compound of formula V and V'.

Compounds of this invention in formula I-VII can be synthesized according to the following general schemes. It will be readily apparent to those of ordinary skill in the art that compounds in formula I-VII can be prepared by substitution of the reagents or agents in the general synthesis routes. The starting materials are either commercially available or can be synthesized by well-established literature methods known to those of ordinary skill in the art.

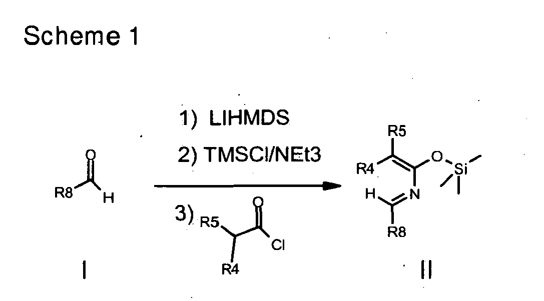

In general an appropriately selected aldehyde **I** can be reacted with lithium hexamethyldisilamide, chlorotrialkylsilane and a selectively substituted acyl chloride in a one-pot, multi-steps manner to generate 2-aza-1,3-butadiene **II (Scheme I)** and can be used as a crude product. Ghosez, L. and others have reported the preparation of 2-aza-1,3-butadienes and their use in aza Diels-Alder reaction to form heterocycle (Ref: Tetrahedron 1995, 11021; J. Am. Chem. Soc. 1999, 2617; and literatures cited therein). The appropriately selected aldehyde I are either commercially available or can be synthesized by well-established multiple literature methods.

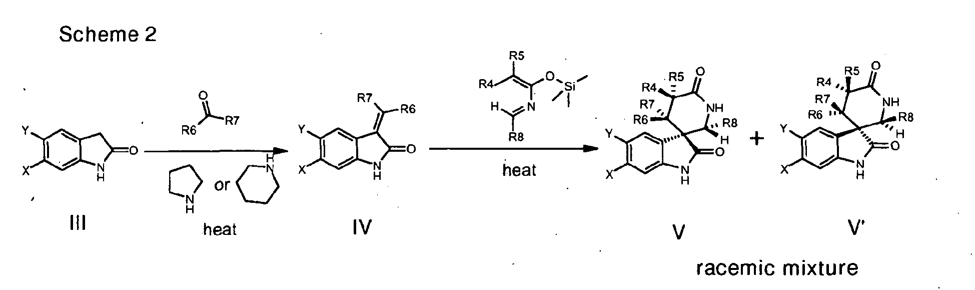

Oxindole **III** can be reacted with an appropriately substituted aldehyde or ketone in the presence of base under heated condition in either a protic solvent like methanol, ethanol or an aprotic solvent like toluene, o-xylene to give intermediate **IV.** The commonly used base is either pyrrolidine or piperidine. Intermediate **IV** can then be reacted with 2-aza-1,3-butadiene **II** in toluene or o-xylene under heating from about 110 °C to 160°C and anhydrous condition to afford a racemic mixture of siproindolinone **V** and **V'** as the major products shown together with other minor stereoisomers. 6-substituted or 5,6-disubstituted oxindole **III** starting materials are either commercially available or prepared according to literature methods, for examples, Kraynack, E. A.; Dalgard, J. E.; Gaeta, F. C. A. Tetrahedron Letters, 1998, 39, 7679-7682, EP153818 for 5-fluro-6-chlorooxindole, etc.

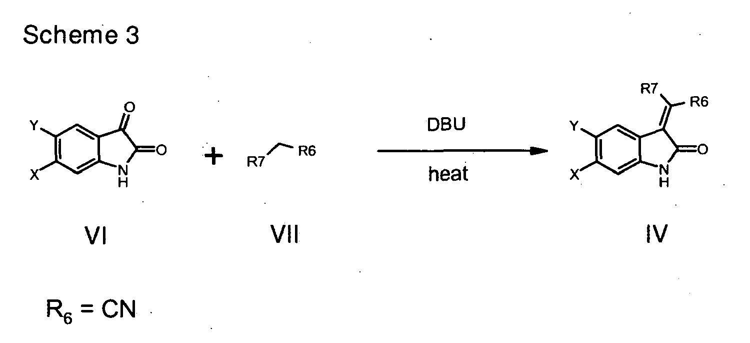

When R₆ is a strong electron withdrawing group in reagent **VII,** intermediate **IV** can be prepared alternatively from Isatin **VI** and reagent **VII.** For example, when R₆ is cyano and R₇ is a substituted aryl, hetereoaryl, Isatin **VI** can react with various R₇ substituted cyanide **VII** in the presence of a base like DBU in methanol under heated condition to form **IV** (Scheme 3). 6-substituted or 5,6-disubstituted isatin **VI** starting materials are either commercially available or prepared according to literature methods

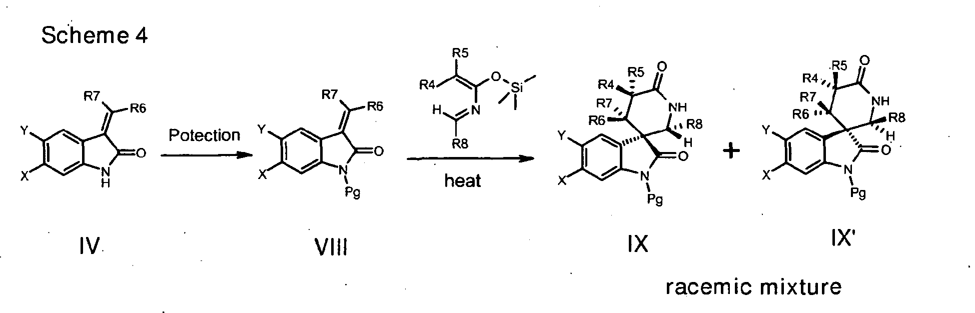

Intermediate **IV** can be protected to give intermediate **VIII.** The protective group can be attached by using ethyl chloroformate, di-tert-butyl dicarbonate, SEM-Cl, benzyl bromide, and a base like 4-(dimethylamine)pyridine (DMAP), triethylamine, NaH, or LiH according to well established literature procedures. Examples of protective group formation and their deprotection have been described and reviewed comprehensively by Greene, T.W. et al in "Protective Groups in Organic Synthesis, 2nd Edition. John Wiley & Sons Inc. In a similar manner intermediate **VIII** can be reacted with a selected 2-aza-butadiene **II** prepared in Scheme 1 in toluene or o-xylene under heating from 110°C to 160 °C and anhydrous condition to form intermediate **IX** and **IX'** as the major products shown as a racemic mixture of two enantiomers together with other minor stereoisomers (Scheme 4). Intermediate **IX** can be converted into **V** by a deprotection reaction (Scheme 5). A useful Pg can be ethyl carbamate, tert-butyl carbamate (BOC), or trimethylsilylethoxymethyl (SEM). Ethyl carbamate can be removed easily by treatment of **IX** with a base like NaOH in methanol or ethanol at room temperature, tert-butyl carbamate (BOC) can be readily removed by treatment of **IX** with trifluoroacetic acid at room temperature. Deprotection of trimethylsilylethoxymethyl (SEM) can be achieved by treatment with trifluoroacetic acid in dichloromethane at room temperature first, followed by heating with diisopropylethylamine in methanol.

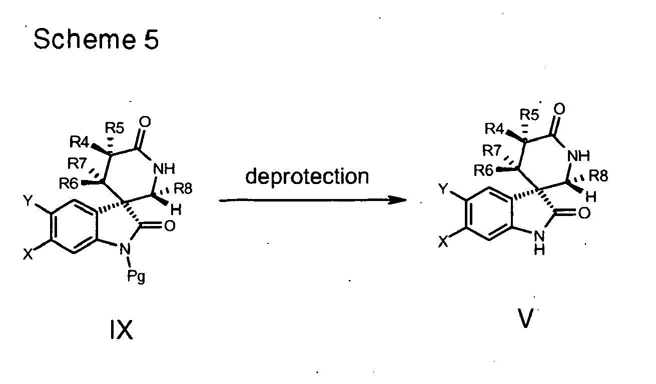

When R₈ is selected from a certain group such as lower alkyl, substituted lower alkyl, cycloalkyl, substituted cycloalkyl, alternative synthetic methods can be used to gain access to compounds **V** or intermediate **IX.** Typically, the compounds of **V** or intermediate **IX** with R₈ selected from a related lower alkenyl, or substituted alkenyl, or cycloalkenyl, or substituted cycloalkenyl, would be prepared first according to the methods in scheme 2 or scheme 4, followed by a catalytic hydrogenation reaction to give those **V** or **IX** with a **R₈** as the corresponding lower alkyl, or substituted lower alkyl, or cycloalkyl, or substituted cycloalkyl. Treatment of the compounds of **V** or intermediate **IX** with R₈ selected from a related lower alkenyl, or substituted alkenyl with Simmons-Smith reagent (CH₂I₂-Et₂Zn) will lead to those **V** or **IX** with a R₈ as the corresponding substituted cyclopropyl group.

V can be selectively protected to give **IX** under controlled conditions. In this case, a useful protective group Pg here can be ethyl carbamate, or tert-butyl carbamate (BOC) **(Scheme 6).** The protective group can be attached by using ethyl chloroformate, or di-tert-butyl dicarbonate, and a base like 4-(dimethylamine)pyridine (DMAP) in dichloromethane at room or lowered temperature similar to the transformation from **IV** to **VIII** in **Scheme 4.**

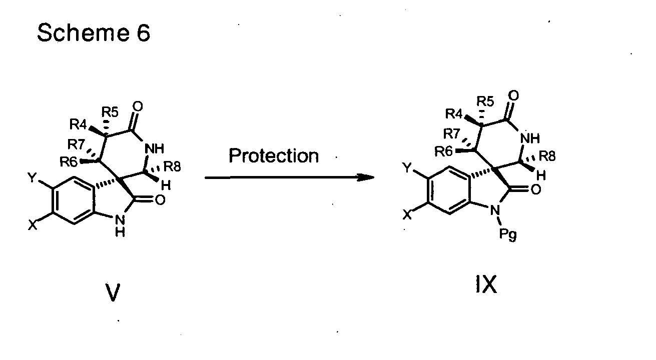

Compound **IX** can be selectively converted into thioamide analogue **X** by using Lawesson reagent or other similarly related reagent (**Scheme 7**). Compound **XI** can be prepared by the reaction of **X** with iodomethane.

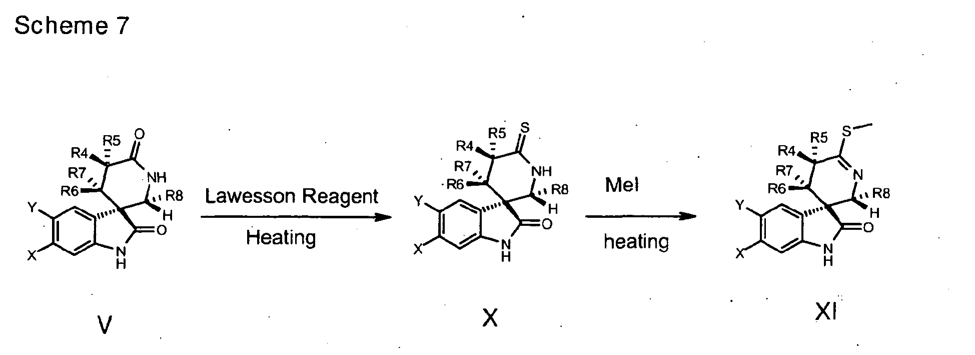

Compound **X** can also react with amine R₉-NH₂, or R₉R₉NH in the presence of a mercuric reagent like HgCl₂ or Hg(OAc)₂ to form analogues **XII** (**Scheme 8**).

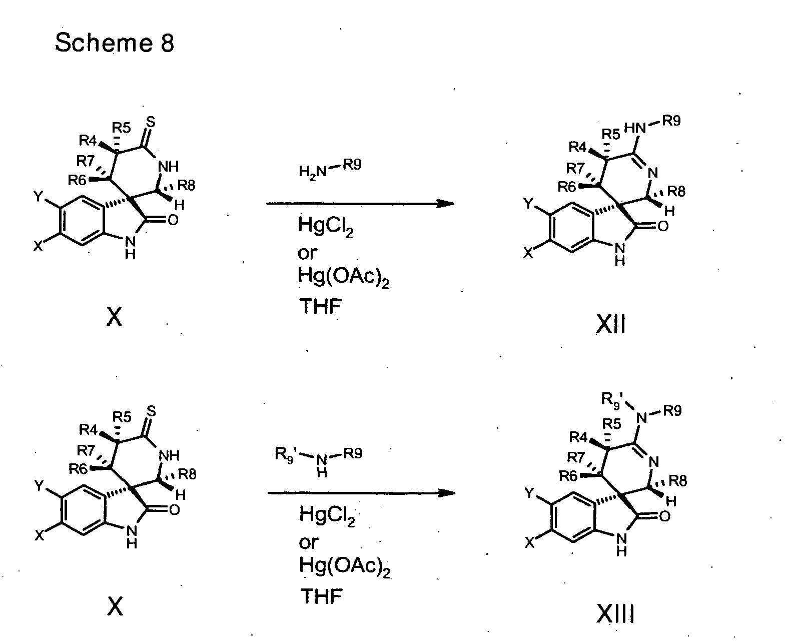

Thioamide compound **X** can also be a useful intermediate to prepare fused triazole analogues. For example, compound **X** can be reacted with substituted hydrazide EtO(O=C)NHNH2, and a mercuric reagent like HgCl₂ or Hg(OAc)₂ to form analogues **XIV** (**Scheme 9**). Heating in a sealed tube in toluene led to the formation of triazole analogue **XV**.

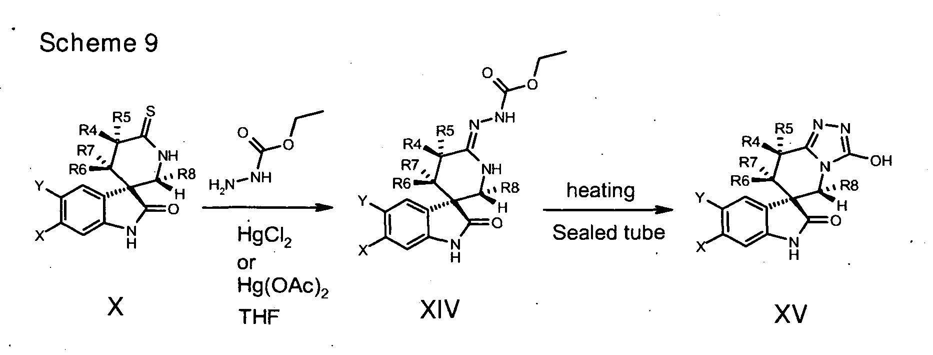

Similarly compound **XVI** can be prepared in **Scheme 10.** Treatment with trifluoroacetic acid to remove t-Boc group led to the formation of intermediate **XVII.**

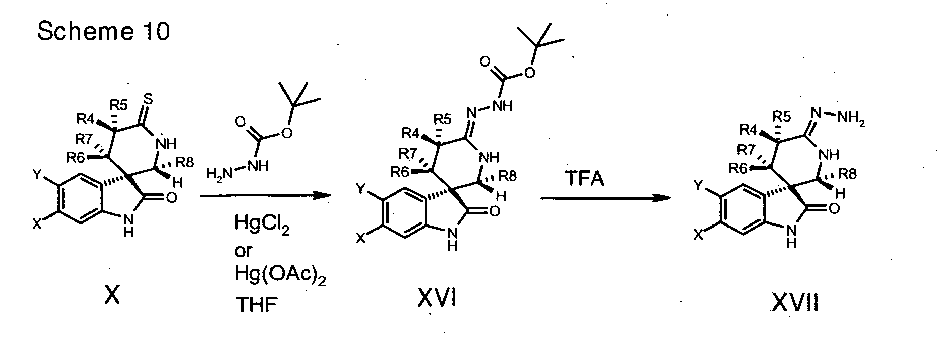

Refluxing of compound **XVII** in either formic acid or acetic acid led to compound **XVIII (Scheme 11).** R₁₀ is hydrogen or methyl.

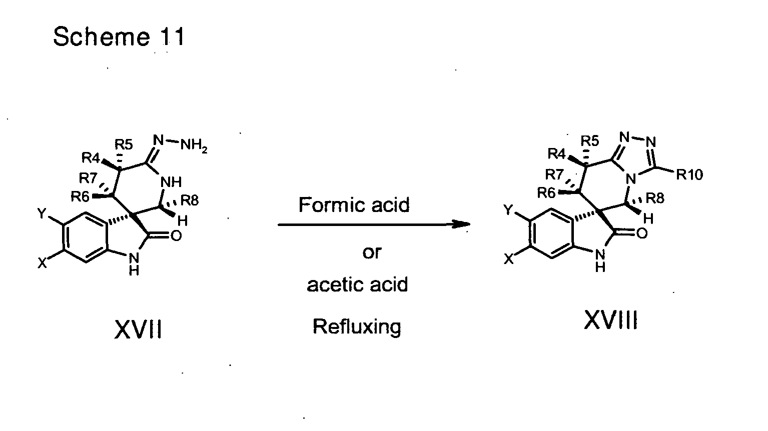

Compound **V** could also be selectively reduced by either sodium borohydride or other reducing reagents, such as, BH₃.THF to form compound **XVIII.** The compound **XVIII** reacted with appropriately selected alkylating reagent R₂-L to give N-alkylated compound **XIX (Scheme 12).** R₂ is a lower alkyl, or substituted lower alkyl, and L is a good leaving group like **I**, Br, Cl, OMs, OTs, OTf. In the cases R₂ is selected from C(=O)R₉, C(=O)NHR₉, C(=O)NR₉R₉; and C(=O)OR₉, the compound **XVIII** reacted with appropriately selected corresponding acyl chloride, isocyanate, aminocarbonyl chloride, or chloroformate, to give N-acylated compound **XIX.**

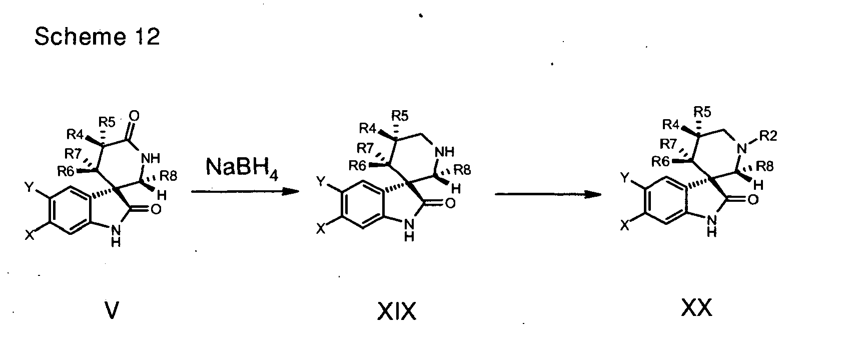

Similarly, intermediate **IX** can also be selectively reduced to intermediate **XXI,** Treatment of **XXI** with either a N-alkylation or a N-acylation reagent in the presence or absence of a base and deprotection reaction of Pg group in **XX** sequentially lead to R₂ derivatized analogue **XXII (Scheme 13)**

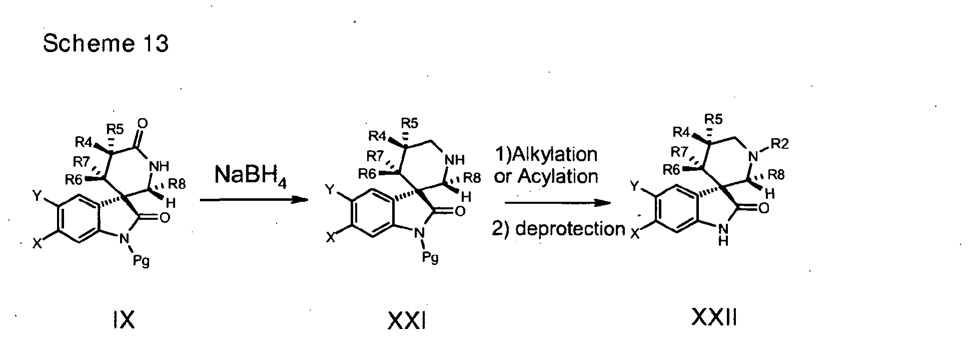

Compound **V** and **V',** or intermediate **IX** and **IX'**can be readily resolved into two optically pure or enriched chiral enantiomers by separation using chiral Super Fluid Chromatography (SFC) or chiral HPLC or chiral column chromatography. In a similar manner to the methods in the reaction schemes above, the chiral enantiomer of compounds **X-XXII** can be prepared by substitution of **V** with its enantiomer **V',** or substitution **of IX** with **IX'** as the corresponding starting material. The compounds **V** and **V',** intermediates **IX** and **IX'** were generated initially as a racemic mixture and subsequently reacted without chiral separation to give the corresponding racemic mixture of **X-XXII** together with their enantiomers . All these racemic mixtures of **X-XXII** and their corresponding enantiomers prepared in each reaction scheme above can also be readily separated into optically pure or enriched chiral enatiomeric pairs in a similar manner to the method for separation **V** and **V',** or **IX** and **IX'.**

The following examples and references are provided to aid the understanding of the present invention, the true scope of which is set forth in the appended claims.

### Example 1a

### Preparation of intermediate E/Z-6-chloro-3-(3,3-dimethyl-butylidene)-1,3-dihydroindol-2-one

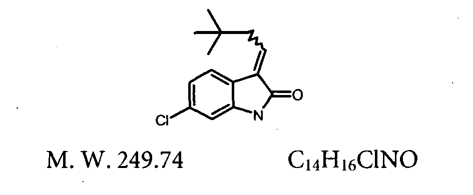

To the mixture of 6-chlorooxindole (0.26 g, 1.49 mmol) (Crescent) and 3,3-dimethylbutyraldehyde (0.21 g, 2.09 mmol) (Aldrich) in methanol (20 mL) was added pyrrolidine (0.15 g, 2.09 mmol) (Aldrich) dropwise. The mixture was then heated at 100 °C for 1 h. The mixture was concentrated, and the residue was partitioned between ethyl acetate and water. The organic layer was separated, dried over Na₂SO₄, concentrated, and dried in *vacuo* to give the crude E/Z-6-chloro-3-(3,3-dimethyl-butylidene)-1,3-dihydro-indol-2-one as a white solid (Yield 0.37 g, 100%).

### Example 1b

### Preparation of intermediate 1-(3-chlorophenyl)-3-trimethylsilyoxy-2-aza-1,3-butadiene

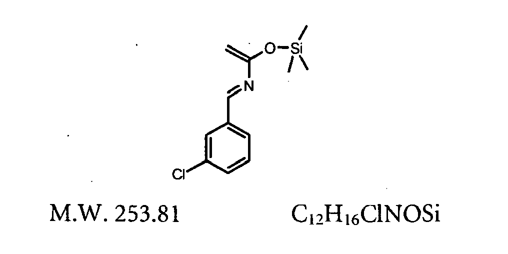

To 1,1,3,3,3-hexamethyldisilazane (2.18 mL, 10.5 mmol) (Aldrich) under nitrogen at room temperature was added n-butyllithium (2.5 M, 4.2 mL, 10.5 mmol) (Aldrich). The reaction mixture was stirred at room temperature for 10 minutes. Then dry tetrahydrofuran (30 mL) was added, followed by the addition of 3-chloro-benzaldehyde (1.19 mL, 10.5 mmol) (Aldrich). After the mixture was stirred at room temperature for 0.5 h, trimethylsilyl chloride (1.33 mL, 10.5 mmol) (Aldrich) was added dropwise. Then the temperature of the mixture was lowered to 0 °C on a cooling ice bath. To this mixture was added triethylamine (1.9 mL, 13.6 mmol) in one portion, followed by the dropwise addition of a solution of acetyl chloride (0.97 mL, 13.6 mmol) in diethyl ether (50 mL). The cooling bath was removed, and the mixture was stirred at room temperature for 1 h. The mixture was quickly filtered on celite under nitrogen, and filtrate was concentrated under reduced pressure to give crude 1-(3-chlorophenyl)-3-trimethylsilyoxy-2-aza-1,3-butadiene as a yellow gum and used for the next step without further purification.

Similar transformation has been reported by Ghosez, L., Bayard, Ph., Nshimyumukiza, P., Gouverneur, V., Sainte, F., Beaudegnies, R., Rivers, M., Frique-Hesbain, A.-M. and Wynants, C. in Tetrahedron 1995, 11021-11042.

### Example 1c

### Preparation of intermediate racemic (2'S, 3S, 4'S)-6-chloro-2'-(3-chlorophenyl)-4'-(2,2-dimethylpropyl) spiro[3H-indole-3,3'-piperidine]-2,6'(1H)-dione

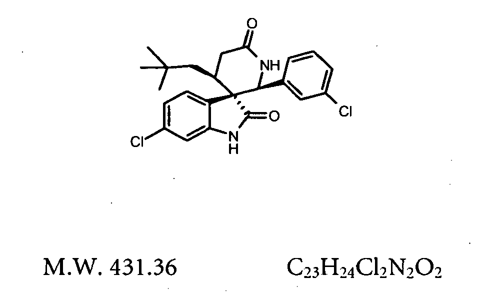

To a mixture of 1-(3-chlorophenyl)-3-trimethylsilyoxy-2-aza-1,3-butadiene (0.25 g, 1 mmol) prepared in example 1b, and toluene (4 mL) was added E/Z-6-chloro-3-(3,3-dimethyl-butylidene)-1,3-dihydro-indol-2-one (0.25 g, 1 mmol) prepared in example 1a. The reaction mixture was heated in a sealed tube under nitrogen at 110 °C for 18 h. The mixture was cooled to room temperature, and methanol (10 mL) was added. The mixture was concentrated and the residue was purified by chromatography (EtOAc/hexanes=2:1) to give racemic (2'S, 3S, 4'S)-6-chloro-2'-(3-chlorophenyl)-4'-(2,2-dimethylpropyl) spiro[3H-indole-3;3'-piperidine]-2,6'(1H)-dione as a brown solid (Yield 0.15 g, 35%). HRMS(ES⁺) *m*/*z* Calcd for C₂₃H₂₄Cl₂N₂O₂ + H [(M+H)⁺]: 431.1288. Found: 431.1285

Similar transformation has been reported by Ghosez, L. and Jnoff, E. in J. Am. Chem. Soc 1999, 2617-2618.

### Example 1d

### Preparation of racemic (2'S, 3S, 4'S)-6-chloro-2'-(3-chlorophenyl)-4'-(2,2-dimethylpropyl) spiro[3H-indole-3,3'-piperidine]-2(1H)-one

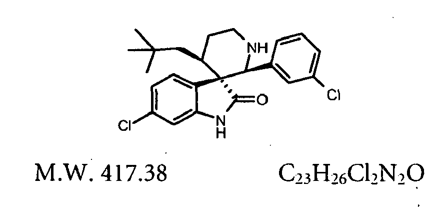

To a solution of racemic (2'S, 3S, 4'S)-6-chloro-2'-(3-chlorophenyl)-4'-(2,2-dimethylpropyl) spiro[3H-indole-3,3'-piperidine]-2,6'(1H)-dione (0.028 g, 0.065 mmol) prepared in example 1c in methanol (2 mL) was added NaBH₄ (0.42 g, 11 mmol) in portions over 1 h at room temperature. After the reaction mixture was stirred at room temperature for 1 h, the reaction mixture was diluted with ethyl acetate and washed with water, brine and concentrated. The residue was purified by chromatography (EtOAc:hexanes = 1:1) to give racemic racemic (2'S, 3S, 4'S)-6-chloro-2'-(3-chlorophenyl)-4'-(2,2-dimethylpropyl) spiro[3H-indole-3,3'-piperidine]-2(1H)-one as a white solid (Yield 0.006 g, 21%)
HRMS(ES⁺) *m*/*z* Calcd for C₂₃H₂₆Cl₂N₂O + H [(M+H)⁺]: 417.1495. Found: 417.1493.

### Example 2a

### Preparation of intermediate E/Z-6-chloro-3-isobutylidene-1,3-dihydro-indol-2-one

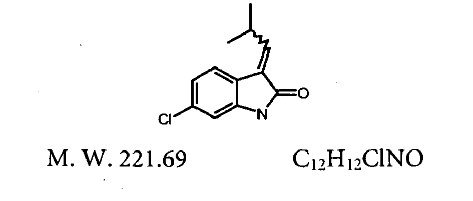

In a manner similar to the method described in example 1a, 6-chlorooxindole (0.85 g, 4.8 mmol) was reacted with 2-methyl-propionaldehyde (0.42 g, 5.8 mmol) (Aldrich), pyrrolidine (0.41 g, 5.8 mmol) in methanol (40 mL) to give a mixture of E/Z-6-chloro-3-isobutylidene 1,3-dihydro-indol-2-one as a brown foam (Yield 1.0 g, 100%).

### Example 2b

### Preparation of intermediate racemic (2'S, 3S, 4'R)-6-chloro-2'-(3-chlorophenyl)-4'-isopropyl spiro[3H-indole-3,3'-piperidine]-2,6'(1H)-dione

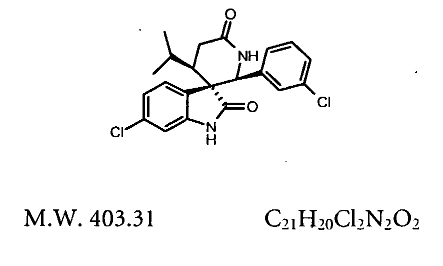

In a manner similar to the method described in example 1c, E/Z-6-chloro-3-isobutylidene-1,3-dihydro-indol-2-one (0.25 g, 1.1 mmol) was reacted with 1-(3-chlorophenyl)-3-trimethylsilyoxy-2-aza-1,3-butadiene (1.2 g, 4.7 mmol) prepared in example1b, in toluene to give racemic (2'S, 3S, 4'R)-6-chloro-2'-(3-chlorophenyl)-4'-isopropyl spiro[3H-indole-3,3'-piperidine]-2,6'(1H)-dione as a white solid (Yield 0.25 g, 56%).
HRMS(ES⁺) *m*/*z* Calcd for C₂₁H₂₀Cl₂N₂O₂ + H [(M+H)⁺]: 403.0975. Found: 403.0975.

### Example 2c

### Preparation of intermediate racemic (2'S, 3R)-6-chloro-2'-(3-chlorophenyl)-4'-isopropyl-6'-thioxo spiro[3H-indole-3,3'-piperidine]-2(1H)-one

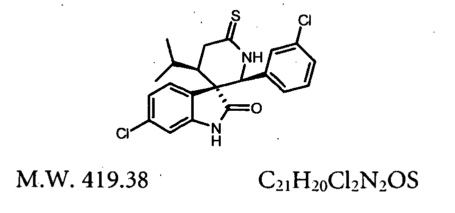

The mixture of racemic (2'S, 3R)-6-chloro-2'-(3-chlorophenyl)-4'-isopropyl spiro[3H-indole-3,3'-piperidine]-2,6'(1H)-dione (60 mg, 0.15 mmol) prepared in example 2b and 2,4-bis-(4-methoxyphenyl)-1,3-dithia-2,4-diphosphetane 2,4-disulfide (100 mg, 0.25 mmol) (Aldrich) in toluene (20 mL) was heated at 120 °C for 0.5 h. The mixture was cooled to room temperature and then concentrated. The residue was purified by chromatography (EtOAc:hexanes=1:1) to give racemic (2'S, 3R)-6-chloro-2'-(3-chlorophenyl)-4'-isopropyl-6'-thioxo spiro[3H-indole-3,3'-piperidine]-2(1H)-one as a white solid (Yield 60 mg, 92%).
HRMS(ES⁺) *m*/*z* Calcd for C₂₁H₂₀Cl₂N₂OS + H [(M+H)⁺]: 419.0746. Found: 419.0744.

### Example 2d

### Preparation of racemic (2'S, 3R)-6-chloro-2'-(3-chlorophenyl)-2',3',4',5'-tetrahydro-4'-isopropyl-6'-(methylthio) spiro[3H-indole-3,3'-pyridin]-2(1H)-one

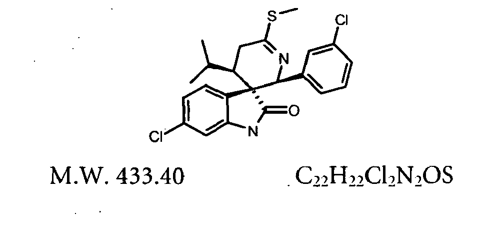

The mixture of racemic (2'S, 3R)-6-chloro-2'-(3-chlorophenyl)-4'-isopropyl-6'-thioxo spiro[3H-indole-3,3'-piperidine]-2(1H)-one (55 mg, 0.13 mmol) prepared in example 2c and iodomethane (1.7 g, 12 mmol) (Aldrich) In dichloroethane (20 mL) was heated at reflux for 2 h. The mixture was cooled to room temperature and then concentrated. The residue was purified by chromatography (EtOAc:hexanes=1:2) to give racemic (2'S, 3R)-6-chloro-2'-(3-chlorophenyl)-2',3',4',5'-tetrahydro-4'-isopropyl-6'-(methylthio) spiro[3H-indole-3,3'-pyridin]-2(1H)-one as a white solid (Yield 40 mg, 70%). HRMS(ES⁺) *m*/*z* Calcd for C₂₂H₂₂Cl₂N₂OS + H [(M+H)⁺]: 433.0933. Found: 433.0902.

### Example 3a

### Preparation of intermediate E-6-chloro-3-propylidene-1,3-dihydro-indol-2-one

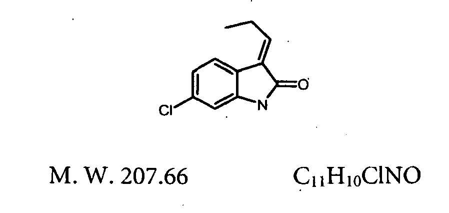

The mixture of 6-chlorooxindole (2.25 g, 12.8 mmol) (Crescent) and propionaldehyde (2.56g, 44 mmol) (Aldrich) and N,N-dimethylaniline (10.9 g, 90 mmol) (Aldrich) was heated at 100 °C for 18 h. The mixture was cooled to room temperature, then poured into aqueous HCl solution (1 N). The mixture was extracted with ethyl acetate. The organic layer was separated, dried over Na₂SO₄, concentrated, and the residue was purified by chromatography (EtOAc:hexanes=1:2) to give E-6-chloro-3-propylidene-1,3-dihydro-indol-2-one as a grey solid (Yield 1.3 g, 49%).

### Example 3b

### Preparation of intermediateracemic (2'S, 3S, 4'S)-6-chloro-2'-(3-chlorophenyl)-4'-ethyl spiro[3H-indole-3,3'-piperidine]-2,6'(1H)-dione

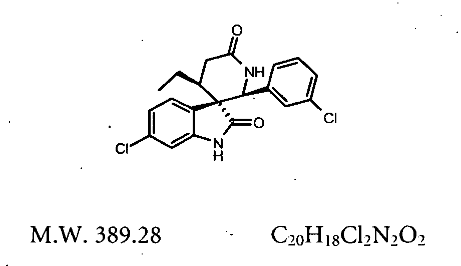

In a manner similar to the method described in example 1c, E-6-chloro-3-propylidene-1,3-dihydro-indol-2-one (0.23 g, 1 mmol) was reacted with 1-(3-chlorophenyl)-3-trimethylsilyoxy-2-aza-1,3-butadiene (1.2 g, 4.7 mmol) prepared in example 1b, in toluene to give racemic (2'S, 3S, 4'S)-6-chloro-2'-(3-chlorophenyl)-4'-ethyl spiro[3H-indole-3,3'-piperidine]-2,6'(1H)-dione as a white solid (Yield 0.21 g, 50%). HRMS(ES⁺) *m*/*z* Calcd for C₂₀H₁₈Cl₂N₂O₂ + H [(M+H)⁺]: 389.0818. Found: 389.0816.

### Example 3c

### Preparation of racemic (2'S, 3S, 4'S)-6-chloro-2'-(3-chlorophenyl)-4'-ethyl spiro[3H-indole-3,3'-piperidine]-2(1H)-one

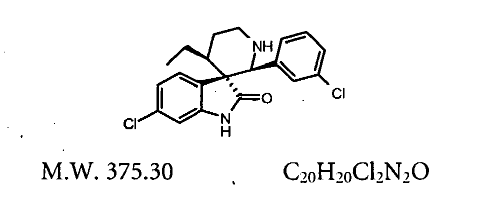

In a manner similar to the method described in example 1d, racemic (2'S, 3S, 4'S)-6-chloro-2'-(3-chlorophenyl)-4'-ethyl spiro[3H-indole-3,3'-piperidine]-2,6'(1H)-dione (0.086 g, 0.22 mmol) prepared in example 3b was reacted with NaBH₄ in methanol to give racemic (2'S, 3S, 4'S)-6-chloro-2'-(3-chlorophenyl)-4'-ethyl spiro[3H-indole-3,3'-piperidine]-2(1H)-one as a white form (Yield 0.018 g, 22%).
HRMS(ES⁺) *m*/*z* Calcd for C₂₀H₂₀Cl₂N₂O + H [(M+H)⁺]: 375.1026. Found: 375.1023.

### Example 4a

### Preparation of intermediate E/Z-6-chloro-3-(3-chloro-benzylidene)-1,3-dihydro-indol-2-one

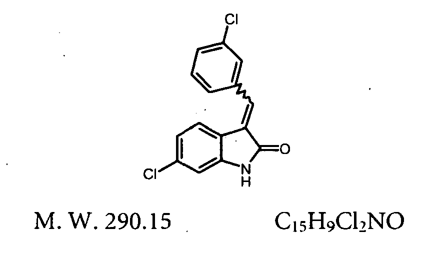

To the mixture of 6-chlorooxindole (16.2 g, 92 mmol) (Crescent) and 3-chloro-benzaldehyde (12.9 g, 92 mmol) (Aldrich) in methanol (109 mL) was added pyrrolidine (6.55 g, 92 mmol) (Aldrich) dropwise. The mixture was then heated at 70 °C for 3 h. After cooled to 4 °C, the mixture was filtered and resulting precipitate was collected, dried to give a mixture of E/Z-6-chloro-3-(3-chloro-benzylidene)-1,3-dihydro-indol-2-one as a bright yellow solid (Yield 25.2 g, 95 %).

### Example 4b

### Preparation of intermediate E/Z-6-chloro-3-(3-chloro-benzylidene)-2-oxo-2,3-dihydroindole-1-carboxylic acid ethyl ester

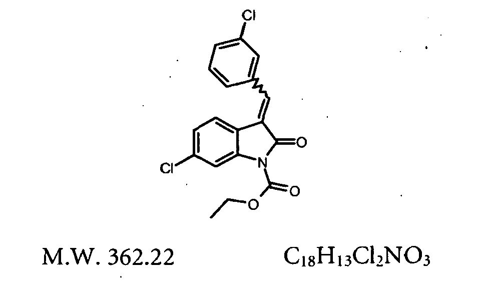

To a solution of E/Z-6-chloro-3-(3-chloro-benzylidene)-1,3-dihydro-indol-2-one prepared in example 6a (1.33 g, 4.6 mmol) in dichloromethane (50 mL) at 0 °C was added ethyl chloroformate (0.66 mL, 6.9 mmol) (Aldrich), followed by the addition of triethylamine (0.93 g, 9.2 mmol). The reaction mixture was stirred at 0 °C for 30 minutes. The mixture was then poured into aqueous HCl solution (1 N). The organic layer was separated and the aqueous layer was extracted with ethyl acetate. The organic layers were combined and dried over Na₂SO₄, and concentrated to give E/Z-6-chloro-3-(3-chlorobenzylidene)-2-oxo-2,3-dihydro-indole-1-carboxylic acid ethyl ester as a yellow solid and used for the next step without further purification (Yield 1.7 g; 100%).

### Example 4c

### Preparation of intermediate 1-(3-methylphenyl)-3-trimethylsilyoxy-2-aza-1,3-butadiene

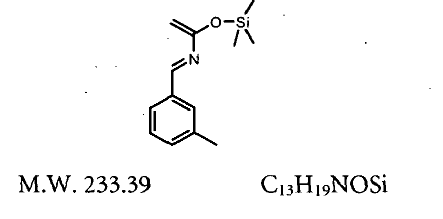

In a manner similar to the method described in example 1b, 3-methyl-benzaldehyde (1.30 g, 10.5 mmol) (Matrix) was used as the starting material in place of 3-chloro-benzaldehyde to react with 1,1,1,3,3,3-hexamethyldisilazane (2.18 mL, 10.5 mmol), n-butyllithium (2.5 M, 4.2 mL, 10.5 mmol), trimethylsilyl chloride (1.33 mL, 10.5 mmol), triethylamine (1.9 mL, 13.6 mmol) and acetyl chloride (0.97 mL, 13.6 mmol) to give 1-(3-methylphenyl)-3-trimethylsilyoxy-2-aza-1,3-butadiene as a yellow gum and used for the next step without further purification.

### Example 4d

### Preparation of intermediate racemic (2'R, 3R, 4'S)-6-chloro-4'-(3-chlorophenyl)-2,3-dihydro-2'-(3-methylphenyl)-2,6'-dioxo spiro[indole-3,3'-piperidine]-1-carboxylic acid ethyl ester

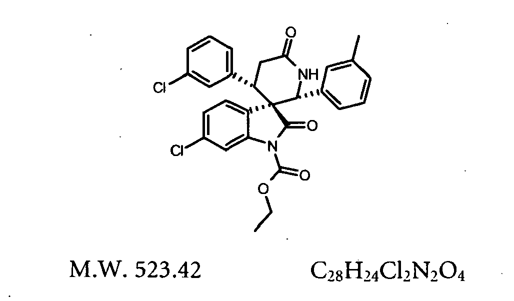

To a solution of 1-(3-methylphenyl)-3-trimethylsilyoxy-2-aza-1,3-butadiene prepared in example 4c in toluene (20 mL) was added E/Z-6-chloro-3-(3-chloro-benzylidene)-2-oxo-2,3-dihydro-indole-1-carboxylic acid ethyl ester prepared in example 4b (0.3 g, 0.83 mmol). The reaction mixture was stirred under nitrogen in a sealed tube at 135°C for 1 h. After the solution was cooled to room temperature, methanol (50 mL) was added, and then the mixture was concentrated. The residue was purified by chromatography (EtOAc:CH₂Cl₂ =1:3) to give racemic (2'R, 3R, 4'S)-6-chloro-4'-(3-chlorophenyl)-2,3-dihydro-2'-(3-methylphenyl)-2,6'-dioxo spiro[indole-3,3'-piperidine]-1-carboxylic acid ethyl ester as a yellow oil (Yield 0.5 g, 86%).

### Example 4e

### Preparation of intermediate racemic (2'R, 3R, 4'S)-6-chloro-4'-(3-chlorophenyl)-2'-(3-methylphenyl) spiro[3H-indole-3,3'-piperidine]-2,6'(1H)-dione

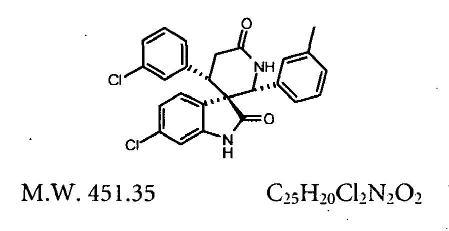

To a solution of racemic (2'R, 3R, 4'S)-6-chloro-4'-(3-chlorophenyl)-2,3-dihydro-2'-(3-methylphenyl)-2,6'-dioxo spiro[indole-3,3'-piperidine]-1-carboxylic acid ethyl ester (0.5 g, 0.96mmol) prepared in example 4d in methanol (30 mL) was added NaOH (69 mg, 1.72 mmol). The mixture was stirred at room temperature for 0.5 h. The solvent was removed and the residue was partitioned between ethyl acetate and aqueous HCl solution (1 N). The aqueous layer was extracted with ethyl acetate. The organic layers were combined and then concentrated. The residue was purified with chromatography (EtOAc:CH₂Cl₂= 1:3) to give racemic (2'R, 3R, 4'S)-6-chloro-4'-(3-chlorophenyl)-2'-(3-methylphenyl) spiro[3H-indole-3,3'-piperidine]-2,6'(1H)-dione as a white solid (Yield 0.15g, 35%).

### Example 4f

### Preparation of racemic (2'R, 3R, 4'S)-6-chloro-4'-(3-chlorophenyl)-2'-(3-methylphenyl) spiro[3H-indole-3,3'-piperidine]-2(1H)-one

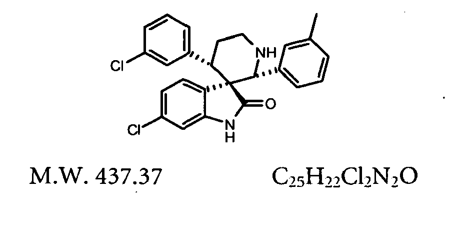

In a manner similar to the method described in example 1d, racemic (2'R, 3R, 4'S)-6-chloro-4'-(3-chlorophenyl)-2'-(3-methylphenyl) spiro[3H-indole-3,3'-piperidine]-2,6'(1H)-dione (0.045 g, 0.1 mmol) prepared in example 4d was reacted with NaBH₄ in methanol to give racemic (2'R, 3R, 4'S)-6-chloro-4'-(3-chlorophenyl)-2'-(3-methylphenyl) spiro[3H-indole-3,3'-piperidine]-2(1H)-one as a white sold (Yield 0.014 g, 31%).
HRMS(ES⁺) *m*/*z* Calcd for C₂₅H₂₂Cl₂N₂O + H [(M+H)⁺]: 437.1182. Found: 437.1178.

### Example 5a

### Preparation of intermediate 1-(3-fluorophenyl)-3-trimethylsilyoxy-2-aza-1,3-butadiene

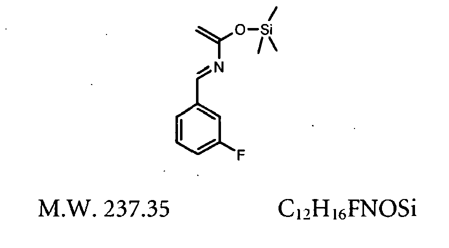

In a manner similar to the method described in example 1b, 3-fluoro-benzaldehyde (1.11 mL, 10.5 mmol) (Fluka) was used as the starting material in place of 3-chloro-benzaldehyde to react with 1,1,1,3,3,3-hexamethyldisilazane (2.18 mL, 10.5 mmol), n-butyllithium (2.5 M, 4.2 mL, 10.5 mmol), trimethylsilyl chloride (1.33 mL, 10.5 mmol), triethylamine (1.9 mL, 13.6 mmol) and acetyl chloride (0.97 mL, 13.6 mmol) to give 1-(3-fluorophenyl)-3-trimethylsilyoxy-2-aza-1,3-butadiene as a yellow gum and used for the next step without further purification.

### Example 5b

### Preparation of intermediate racemic (2'R, 3R, 4'S)-6-chloro-4'-(3-chlorophenyl)-2'-(3-fluorophenyl)-2,3-dihydro-2,6'-dioxo spiro[indole-3,3'-piperidine]-1-carboxylic acid ethyl ester

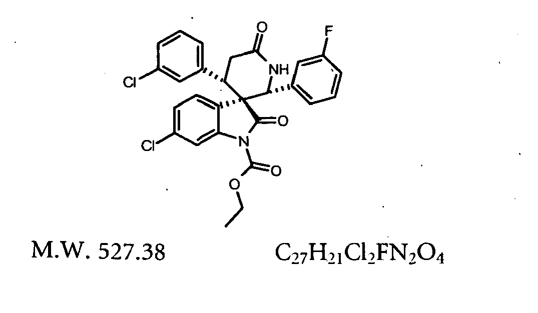

In a manner similar to the method described in example 4d, E/Z-6-chloro-3-(3-chlorobenzylidene)-2-oxo-2,3-dihydro-indole-1-carboxylic acid ethyl ester (0.25 g, 0.69 mmol) prepared in example 4b was reacted with 1-(3-fluorophenyl)-3-trimethylsilyoxy-2-aza-1,3-butadiene prepared in example 6a, in toluene to give racemic (2'R, 3R, 4'S)-6-chloro-4'-(3-chlorophenyl)-2'-(3-fluorophenyl)-2,3-dihydro-2,6'-dioxo spiro[indole-3,3'-piperidine]-1-carboxylic acid ethyl ester as a yellow oil (Yield 0.35 g, 97%).

### Example 5c

### Preparation of intermediate racemic (2'R, 3R, 4'S)-6-chloro-4'-(3-chlorophenyl)-2'-(3-fluorophenyl) spiro[3H-indole-3,3'-piperidine]-2,6'(1H)-dione

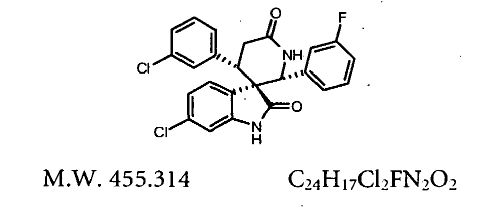

In a manner similar to the method described in example 4e, racemic (2'R, 3R, 4'S)-6-chloro-4'-(3-chlorophenyl)-2'-(3-fluorophenyl)-2,3-dihydro-2,6'-dioxo spiro[indole-3,3'-piperidine]-1-carboxylic acid ethyl ester (0.35 g, 0.66 mmol) was reacted with NaOH (48 mg, 1.19 mmol) to give racemic (2'R, 3R, 4'S)-6-chloro-4'-(3-chlorophenyl)-2'-(3-fluorophenyl) spiro[3H-indole-3,3'-piperidine]-2,6'(1H)-dione as a white solid (Yield 0.15 g, 50%).
HRMS(ES⁺) *m*/*z* Calcd for C₂₄H₁₇Cl₂FN₂O₂ + H [(M+H)⁺]: 451.0975. Found: 451.0976.

### Example 5d

### Preparation of racemic (2'R, 3R, 4'S)-6-chloro-4'-(3-chlorophenyl)-2'-(3-fluorophenyl) spiro[3H-indole-3,3'-piperidine]-2(1H)-one

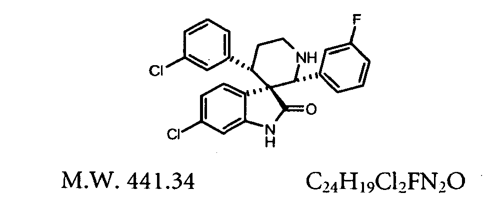

In a manner similar to the method described in example 1d, racemic (2'R, 3R, 4'S)-6-chloro-4'-(3-chlorophenyl)-2'-(3-fluorophenyl) spiro[3H-indole-3,3'-piperidine]-2,6'(1H)-dione (0.046 g, 0.1 mmol) prepared in example 5c was reacted with NaBH₄ in methanol to give racemic (2'R, 3R, 4'S)-6-chloro-4'-(3-chlorophenyl)-2'-(3-fluorophenyl) spiro[3H-indole-3,3'-piperidine]-2(1H)-one as a white solid (Yield 0.020 g, 46%).
HRMS(ES⁺) *m*/*z* Calcd for C₂₄H₁₉Cl₂FN₂O + H [(M+H)⁺]: 441.0931. Found: 441.0928.

### Example 6a

### Preparation of intermediate E/Z-3-benzylidene-6-chloro-1,3-dihydro-indol-2-one

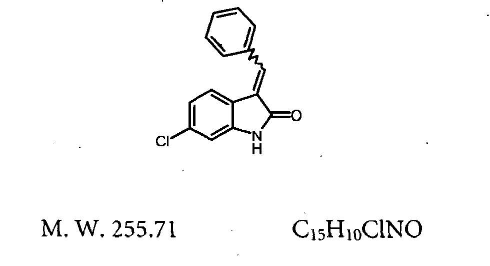

In a manner similar to the method described in example 1a, 6-chlorooxindole (1.0 g, 5.7 mmol) was reacted with benzaldehyde (0.6 g, 5.7 mmol) (Aldrich) and pyrrolidine (0.4 g, 5.7 mmol) in methanol to give a mixture of E-and Z-3-benzylidene-6-chloro-1,3-dihydro-indol-2-one as a yellow solid (Yield 1.5 g, 100%).

### Example 6b

### Preparation of intermediate E/Z-3-benzylidene-6-chloro-2-oxo-2,3-dihydro-indole-1-carboxylic acid ethyl ester

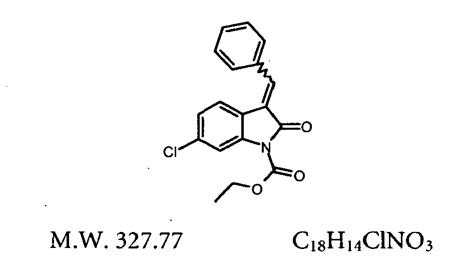

In a manner similar to the method described in example 4b, E/Z-3-benzylidene-6-chloro-1,3-dihydro-indol-2-one (1.5 g, 5.87 mmol) was reacted with ethyl chloroformate (0.83 mL, 8.8 mmol) and triethylamine (1.64 mL, 12 mmol.) in dichloromethane to give E/Z-3-benzylidene-6-chloro₋2-oxo-2,3-dihydro-indole-1-carboxylic acid ethyl ester as a yellow solid (Yield 2.0 g, 100%).

### Example 6c

### Preparation of intermediate racemic (2'S, 3S, 4'R)-6-chloro-2'-(3-chlorophenyl)-4'-phenyl spiro[3H-indole-3,3'-piperidine]-2,6'(1H)-dione

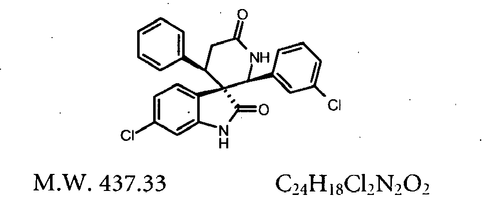

To a solution of 1-(3-chlorophenyl)-3-trimethylsilyoxy-2-aza-1,3-butadiene prepared in example 1b in toluene (30 mL) was added E/Z-3-benzylidene-6-chloro-2-oxo-2,3-dihydro-indole-1-carboxylic acid ethyl ester prepared in Example 6b (0.4 g, 1.22 mmol). The reaction mixture was stirred under nitrogen in a sealed tube at 140 °C for 1 h. After the solution was cooled to room temperature, methanol (40 mL) was added. The reaction mixture was filtered through a short pad of celite gel and washed with ethyl acetate. The filtrate was concentrated. The residue was dissolved in methanol (30 mL) and 1N of NaOH solution (5 mL, 5 mmol) was added. The reaction mixture was stirred at room temperature for 0.5 h, then the mixture was concentrated. The residue was purified by chromatography (EtOAc:CH₂Cl₂ =1:4) to give racemic (2'S, 3S, 4'R)-6-chloro-2'-(3-chlorophenyl)-4'-phenyl spiro[3H-indole-3,3'-piperidine]-2,6'(1H)-dione as a yellow oil (Yield 0.5 g, 100%).
HRMS(ES⁺) *m*/*z* Calcd for C₂₄H₁₈Cl₂NO₂ + H [(M+H)⁺]: 437.0818. Found: 437.0817.

### Example 6d

### Preparation of racemic (2'S, 3S, 4'R)-6-chloro-2'-(3-chlorophenyl)-4'-phenyl spiro[3H-indole-3,3'-piperidine]-2(1H)-one

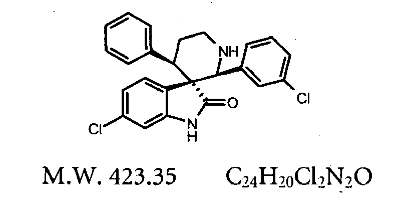

In a manner similar to the method described in example 1d, racemic (2'S, 3S, 4'R)-6-chloro-2'-(3-chlorophenyl)-4'-phenyl spiro[3H-indole-3,3'-piperidine]-2,6'(1H)-dione (52.1 mg, 0.12 mmol) prepared in example 6c was reacted with NaBH₄ (45.1 mg, 1.2 mmol) in methano (2 mL) to give racemic (2'S, 3S, 4'R)-6-chloro-2'-(3-chlorophenyl)-4'-phenyl spiro[3H-indole-3,3'-piperidine]-2(1H)-one as a white solid (Yield 15.8 mg, 31.1%).
HRMS(ES⁺) *m*/*z* Calcd for C₂₄H₂₀Cl₂N₂O + H [(M+H)⁺]: 423.1026. Found: 423.1025.

### Example 7a

### Preparation of intermediate 1-(3-chlorophenyl)-4-methyl-3-trimethylsilyoxy-2-aza-1,3-butadiene

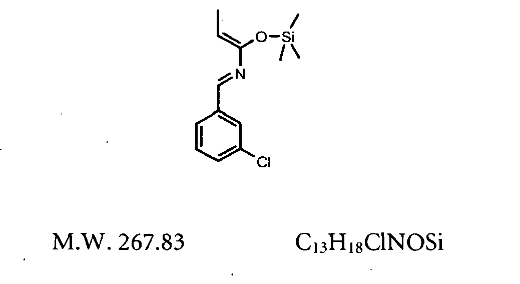

In a manner similar to the method described in example 1b, propionyl chloride (1.2 g, 13.mmol) (Aldrich) was used as the starting material in place of acetyl chloride to react with 1,1,3,3,3-hexamethyldisilazane (1.61 g, 10 mmol), n-butyllithium (2.5 M, 4 mL, 10 mmol), 3-chloro-benzaldehyde (1.4 g, 10 mmol) (Aldrich), trimethylsilyl chloride (1.1 g, 10 mmol) and triethylamine (1.36 g, 13 mmol) to give 1-(3-chlorophenyl)-4-methyl-3-trimethylsilyoxy-2-aza-1,3-butadiene as a yellow gum and used for the next step without further purification.

### Example 7b

### Preparation of intermediate racemic (2'S, 3S, 4'R, 5'R)-6-chloro-2'-(3-chlorophenyl)-5'-methyl-4'-phenyl spiro[3H-indole-3,3'-piperidine]-2,6'(1H)-dione

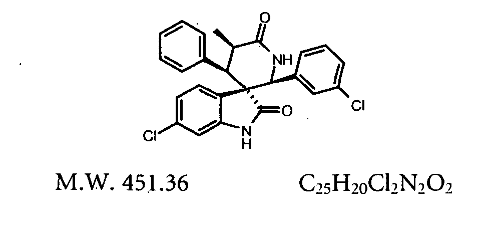

In a manner similar to the method described in example 6c, E/Z-3-benzylidene-6-chloro-2-oxo-2,3-dihydro-indole-1-carboxylic acid ethyl ester (0.32 g, 0.98 mmol) prepared in example 6b was reacted with 1-(3-chlorophenyl)-4-methyl-3-trimethylsilyoxy-2-aza-1,3-butadiene (1.5 g, 5.6 mmol) prepared in example 7a in toluene and then 2 N of NaOH solution (4 mL, 8 mmol) in methanol to give racemic (2'S, 3S, 4'R, 5'R)-6-chloro-2'-(3-chlorophenyl)-5'-methyl-4'-phenyl spiro[3H-indole-3,3'-piperidine]-2,6'(1H)-dione as a white foam (Yield 0.21 g, 48%).
HRMS(ES⁺) *m*/*z* Calcd for C₂₅H₂₀Cl₂N₂O₂ + H [(M+H)⁺]: 451.0975. Found: 451.0972.

### Example 7c

### Preparation of racemic (2'R, 3R, 4'S, 5'S)-6-chloro-4'-(3-chlorophenyl)-5'-methyl-2'-(2-methylphenyl) spiro[3H-indole-3,3'-piperidine]-2(1H)-one

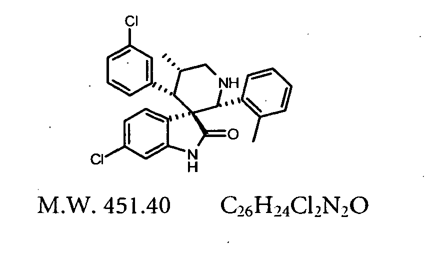

In a manner similar to the method described in example 1d, racemic (2'R, 3R, 4'S, 5'S)-6-chloro-4'-(3-chlorophenyl)-5'-methyl-2'-(2-methylphenyl) spiro[3H-indole-3,3'-piperidine]-2,6'(1H)-dione (0.21 g, 0.45 mmol) prepared in example 7b was reacted with NaBH₄ (170.2 mg, 4.5 mmol) in methanol (2 mL) to give racemic (2'R, 3R, 4'S, 5'S)-6-chloro-4'-(3-chlorophenyl)-5'-methyl-2'-(2-methylphenyl) spiro[3H-indole-3,3'-piperidine]-2(1H)-one as a white solid (Yield 13.6 mg, 6.7%).
HRMS(ES⁺) *m*/*z* Calcd for C₂₆H₂₄Cl₂N₂O + H [(M+H)⁺]: 451.1339. Found: 451.1337.

### Example 8a

### Preparation of intermediate 1-(3-methoxyphenyl)-3-trimethylsilyoxy-2-aza-1,3-butadiene

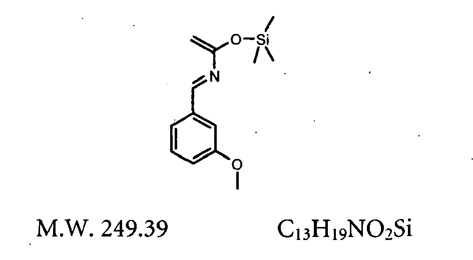

In a manner similar to the method described in example 1b, 3-methoxy-benzaldehyde (1.3 g, 9.5 mmol) (Aldrich) was used as the starting material in place of 3-chloro-benzaldehyde to react with 1,1,1,3,3,3-hexamethyldisilazane (1.53 g, 9.5 mmol), n-butyllithium (2.5 M, 3.8 mL, 9.5 mmol), trimethylsilyl chloride (1.2 mL, 9.5 mmol), triethylamine (1.72 mL, 12.4 mmol) and acetyl chloride (0.88 mL, 12.4 mmol) to give 1-(3-methoxyphenyl)-3-trimethylsilyoxy-2-aza-1,3-butadiene as a yellow gum and used for the next step without further purification.

### Example 8b

### Preparation of intermediate racemic (2'R, 3R, 4'S)-6-chloro-4'-(3-chlorophenyl)-2'-(3-methoxyphenyl) spiro[3H-indole-3,3'-piperidine]-2,6'(1H)-dione

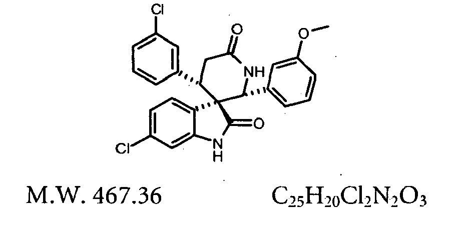

In a manner similar to the method described in example 6c, E/Z-6-chloro-3-(3-chlorobenzylidene)-2-oxo-2,3-dihydro-indole-1-carboxylic acid ethyl ester (0.23 g, 0.63 mmol) prepared in example 4b was reacted with 1-(3-methoxyphenyl)-3-trimethylsilyoxy-4-methyl-2-aza-1,3-butadiene (2 g, 8.0 mmol) prepared in example 8a in toluene and then 2 N of NaoH solution (4 mL, 8 mmol) in methanol to give racemic (2'R, 3R, 4'S)-6-chloro-4'-(3-chlorophenyl)-2'-(3-methoxyphenyl) spiro[3H-indole-3,3'-piperidine]-2,6'(1H)-dione (Yield 0.2 g, 69%).
HRMS(ES⁺) *m*/*z* Calcd for C₂₅H₂₀Cl₂N₂O₃ + H [(M+H)⁺]: 467.0924. Found: 467.0925.

### Example 8c

### Preparation of racemic (2'R, 3R, 4'S)-6-chloro-4'-(3-chlorophenyl)-2'-(3-methoxyphenyl) spiro[3H-indole-3,3'-piperidine]-2(1H)-one

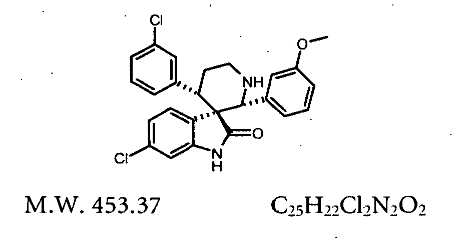

In a manner similar to the method described in example 1d, racemic (2'R, 3R, 4'S)-6-chloro-4'-(3-chlorophenyl)-2'-(3-methoxyphenyl) spiro[3H-indole-3,3'-piperidine]-2,6'(1H)-dione (0.11 g, 0.24 mmol) prepared in example 8b was reacted with NaBH₄ in methanol to give racemic (2'R, 3R, 4'S)-6-chloro-4'-(3-chlorophenyl)-2'-(3-methoxyphenyl) spiro[3H-indole-3,3'-piperidine]-2(1H)-one as a white solid (Yield 0.018 g, 17%).
HRMS(ES⁺) *m*/*z* Calcd for C₂₅H₂₂Cl₂N₂O₂ + H [(M+H)⁺]: 453.1131. Found: 453.1125.

### Example 9a

### Preparation of intermediate 1-(3-cyanophenyl)-3-trimethylsilyoxy-2-aza-1,3-butadiene

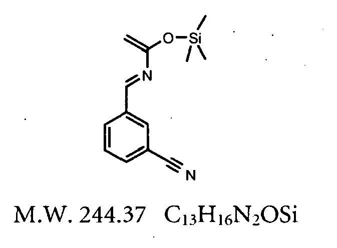

In a manner similar to the method described in example 1b, 3-cyano-benzaldehyde (1.2 g, 10 mmol) (Aldrich) was used as the starting material in place of 3-chloro-benzaldehyde to react with 1,1,1,3,3,3-hexamethyldisilazane (1.62 g, 10 mmol), n-butyllithium (2.5 M, 4 mL, 10 mmol), trimethylsilyl chloride (1.1 g, 10 mmol), triethylamine (1.3 g, 13 mmol) and acetyl chloride (1.0 g, 13 mmol) to give 1-(3-cyanophenyl)-3-trimethylsilyoxy-2-aza-1,3-butadiene as a yellow gum and used for the next step without further purification.

### Example 9b

### Preparation of intermediate racemic (2'R, 3R, 4'S)-6-chloro-4'-(3-chlorophenyl)-2'-(3-cyanophenyl) spiro[3H-indole-3,3'-piperidine]-2,6'(1H)-dione

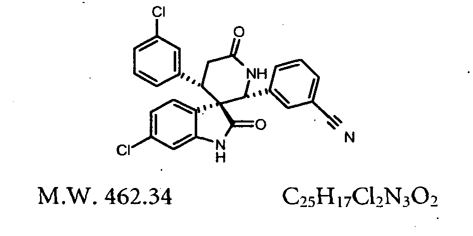

In a manner similar to the method described in example 6c, E/Z-6-chloro-3-(3-chlorobenzylidene)-2-oxo-2,3-dihydro-indole-1-carboxylic acid ethyl ester (0.42 g, 1.2 mmol) prepared in example 4b was reacted with 1-(3-cyanophenyl)-3-trimethylsilyoxy-2-aza-1,3-butadiene (2.0 g, 8.18 mmol) prepared in example 9a in toluene and then 2 N of NaOH solution (5 mL), 10 mmol) in methanol to give racemic (2'R, 3R, 4'S)-6-chloro-4'-(3-chlorophenyl)-2'-(3-cyanophenyl) spiro[3H-indole-3,3'-piperidine]-2,6'(1H)-dione as a white foam (Yield 0.11 g, 20%).
HRMS(ES⁺) *m*/*z* Calcd for C₂₅H₁₇Cl₂N3O₂ + H [(M+H)⁺]: 462.0771 Found: 462.0771.

### Example 9c

### Preparation of racemic (2'R, 3R, 4'S)-6-chloro-4'-(3-chlorophenyl)-2'-(3-cyanophenyl) spiro[3H-indole-3,3'-piperidine]-2(1H)-one

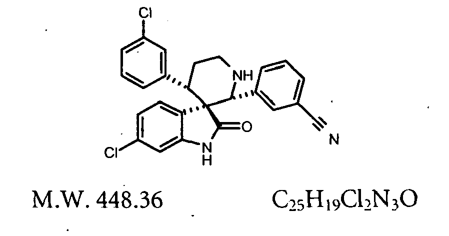

In a manner similar to the method described in example 1d, (2'R, 3R, 4'S)-6-chloro-4'-(3-chlorophenyl)-2'-(3-cyanophenyl) spiro[3H-indole-3,3'-piperidine]-2,6'(1H)-dione (0.05 g, 0.11 mmol) prepard in 9b was reacted with NaBH₄ in methanol to give racemic (2'R, 3R, 4'S)-6-chloro-4'-(3-chlorophenyl)-2'-(3-cyanophenyl) spiro[3H-indole-3,3'-piperidine]-2(1H)-one as a white form (Yield: 0.026 g, 53%).
HRMS(ES⁺) *m*/*z* Calcd for C₂₅H₁₉Cl₂N₃O + H [(M+H)⁺]: 448.0978. Found: 448.0978.

### Example 10a

### Preparation of intermediate 1-(2,3-dimethylphenyl)-3-trimethylsilyoxy-2-aza-1,3-butadiene

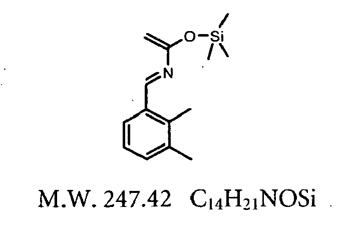

In a manner similar to the method described in example 1b, 2,3-dimethyl-benzaldehyde (1.34 g, 10 mmol) (Aldrich) was used as the starting material in place of 3-chlorobenzaldehyde to react with 1,1,3,3,3-hexamethyldisilazane (1.61 g, 10 mmol), *n-*butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(2,3-dimethylphenyl)-3-trimethylsilyoxy-2-aza-1,3-butadiene as a yellow gum and used for the next step without further purification.

### Example 10b

### Preparation of intermediate racemic (2'R, 3R, 4'S)-6-chloro-4'-(3-chlorophenyl)-2'-(2,3-dimethylphenyl) spiro[3H-indole-3,3'-piperidine]-2,6'(1H)-dione

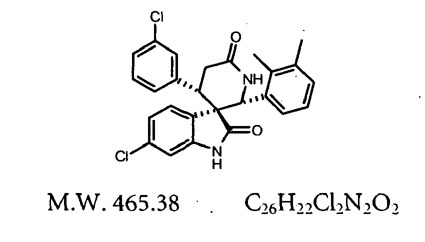

In a manner similar to the method described in example 6c, E/Z-6-chloro-3-(3-chlorobenzylidene)-2-oxo-2,3-dihydro-indole-1-carboxylic acid ethyl ester (0.3 g, 0.83 mmol) prepared in example 4b was reacted with 1-(2,3-dimethylphenyl)-3-trimethylsilyoxy-2-aza-1,3-butadiene (2.4 g, 9.72 mmol) prepared in example 10a in toluene and then NaOH (0.2 g, 5 mmol) in methanol to give racemic (2'R, 3R, 4'S)-6-chloro-4'-(3-chlorophenyl)-2'-(2,3-dimethylphenyl) spiro[3H-indoLe-3,3'-piperidine]-2,6'(1H)-dione (Yield 0.20 g, 53%).
HRMS(ES⁺) *m*/*z* Calcd for C₂₆H₂₂CL₂N₂O₂ + H [(M+H)⁺]: 465.1131. Found: 465.1131.

### Example 10c

### Preparation of racemic (2'R, 3R, 4'S)-6-chloro-4'-(3-chlorophenyl)-2'-(2, 3-dimethylphenyl) spiro[3H-indole-3,3'-piperidine]-2(1H)-one

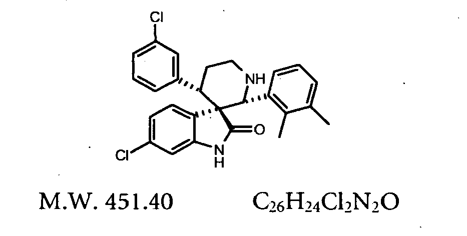

In a manner similar to the method described in example 1d, racemic (2'R, 3R, 4'S)-6-chloro-4'-(3-chlorophenyl)-2'-(2, 3-dimethylphenyl) spiro[3H-indole-3,3'-piperidine]-2,6'(1H)-dione (72.9 mg, 0.16 mmol) prepared in example 35b was reacted with NaBH₄ (68.1 mg, 1.6 mmol) in methanol (2 mL) to give racemic (2'R, 3R, 4'S)-6-chloro-4'-(3-chlorophenyl)-2'-(2, 3-dimethylphenyl) spiro[3H-indole-3,3'-piperidine]-2(1H)-one as a white solid (Yield 8.2 mg, 12%).
HRMS(ES⁺) *m*/*z* Calcd for C₂₆H₂₄Cl₂N₂O + H [(M+H)⁺]: 451.1339. Found: 451.1334.

### Example 11a

### Preparation of intermediate 1-[2-(trifluoromethyl)-phenyl]-3-trimethylsilyoxy-2-aza-1,3-butadiene

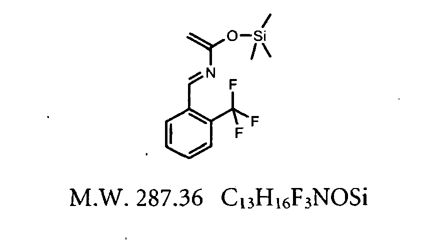

In a manner similar to the method described in example 1b, 2-(trifluoromethyl)-benzaldehyde (1.75 g, 10 mmol) (Aldrich) was used as the starting material in place of 3-chloro-benzaldehyde to react with 1,1,3,3,3-hexamethyldisilazane (1.6 g, 10 mmol), n-butyllithium (2.5 M, 4 mL, 10 mmol), trimethylsilyl chloride (1.1 g, 10-mmol), triethylamine (1.4 g, 13 mmol) and acetyl chloride (1.0 g, 13 mmol) to give 1-[2-(trifluoromethyl)-phenyl]-3-trimethylsilyoxy-2-aza-1,3-butadiene as a yellow gum and used for the next step without further purification.

### Example 11b

### Preparation of intermediate racemic (2'R, 3R, 4'S)-6-chloro-4'-(3-chlorophenyl)-2'-[2-(trifluoromethyl)-phenyl] spiro[3H-indole-3,3'-piperidine]-2,6'(1H)-dione

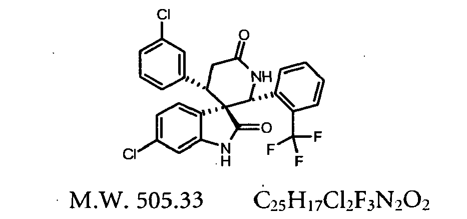

In a manner similar to the method described in example 6c, E/Z-6-chloro-3-(3-chlorobenzylidene)-2-oxo-2,3-dihydro-indole-1-carboxylic acid ethyl ester (0.5 g, 1.38 mmol) prepared in example 4b was reacted with 1-[2-(trifluoromethyl)-phenyl]-3-trimethylsilyoxy-2-aza-1,3-butadiene (3.2 g, 11.1 mmol) prepared in example 11a in toluene and then NaOH (0.2 g, 5 mmol) in methanol to give racemic (2'R, 3R, 4'S)-6-chloro-4'-(3-chlorophenyl)-2'-[2-(trifluoromethyl)-phenyl] spiro[3H-indole-3,3'-piperidine]-2,6'(1H)-dione as an off-white solid (Yield 0.45 g, 64%).
HRMS(ES⁺) *m*/*z* Calcd for C₂₅H₁₇Cl₂F₃N₂O₂+ H [(M+H)⁺]: 505.0692. Found: 505.0688.

### Example 11c

### Preparation of racemic (2'R, 3R, 4'S)-6-chloro-4'-(3-chlorophenyl)-2'-[2-(trifluoromethyl)-phenyl)] spiro[3H-indole-3,3'-piperidine]-2(1H)-one

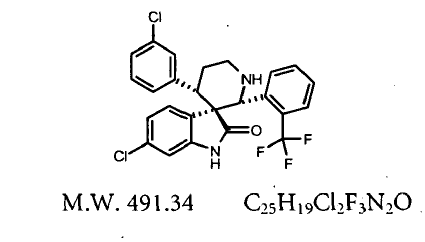

In a manner similar to the method described in example 1d, racemic (2'R, 3R, 4'S)-6-chloro-4'-(3-chlorophenyl)-2'-[2-(trifluoromethyl)-phenyl] spiro[3H-indole-3,3'-piperidine]-2,6'(1H)-dione (0.067 g, 0.13 mmol) prepared in example 11b was reacted with NaBH₄ in methanol to give racemic (2'R, 3R, 4'S)-6-chloro-4'-(3-chlorophenyl)-2'-[2-(trifluoromethyl)-phenyl)] spiro[3H-indole-3,3'-piperidine]-2(1H)-one as a white solid (Yield 0.008 g, 13%).
HRMS(ES⁺) *m*/*z* Calcd for C₂₅H₁₉Cl₂F₃N₂O + H [(M+H)⁺]: 491.0900. Found: 491.0894.

### Example 12a

### Preparation of intermediate racemic (2'R, 3R, 4'S)-6-chloro-4'-(3-chlorophenyl)-6'-thioxo-2'-[2-(trifluoromethyl)-phenyl] spiro[3H-indole-3,3'-piperidine]-2(1H)-one

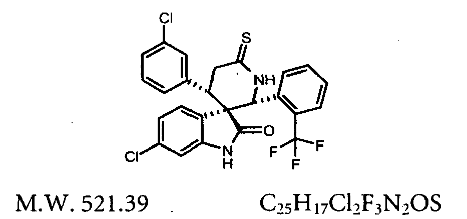

In a manner similar to the method described in example 2c, racemic (2'R, 3R, 4'S)-6-chloro-4'-(3-chlorophenyl)-2'-[2-(trifluoromethyl)-phenyl] spiro[3H-indole-3,3'-piperidine]-2,6'(1H)-dione (0.4 g, 0.79 mmol) prepared in example 11b was reacted with 2,4-bis-(4-methoxyphenyl)-1,3-dithia-2,4-diphosphetane 2,4-disulfide (0.4 g, 0.99 mmol) in toluene to give racemic (2'R, 3R, 4'S)-6-chloro-4'-(3-chlorophenyl)-6'-thioxo-2'-[2-(trifluoromethyl)-phenyl] spiro[3H-indole-3,3'-piperidine]-2(1H)-one (Yield 0.15 g, 36%).
HRMS(ES⁺) *m*/*z* Calcd for C₂₅H₁₇Cl₂F₃N₂OS+H [(M+H)⁺]: 521.0464. Found: 521.0458.

### Example 12b

### Preparation of racemic (2'S, 3R)-6-chloro-4'-(3-chlorophenyl)-2',3',4',5'-tetrahydro-6'-(methylthio)-2'-[2-(trifluoromethyl)-phenyl] spiro[3H-indole-3,3'-pyridin]-2(1H)-one

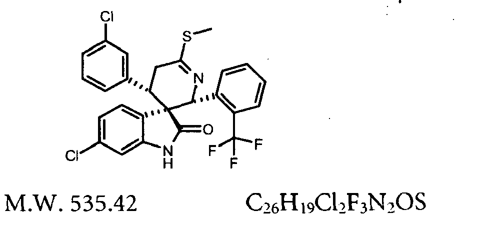

In a manner similar to the method described in example 38, racemic (2'R, 3R, 4'S)-6-chloro-4'-(3-chlorophenyl)-6'-thioxo-2'-[2-(trifluoromethyl)-phenyl] spiro[3H-indole-3,3'-piperidine]-2(1H)-one (0.15 g, 0.29 mmol) prepared in example 41 was reacted with iodomethane (2.5 g, 17.6 mmol) in dichloroethane to give racemic (2'S, 3R)-6-chloro-4'-(3-chlorophenyl)-2',3',4',5'-tetrahydro-6'-(methylthio)-2'-[2-(trifluoromethyl)-phenyl] spiro[3H-indole-3,3'-pyridin]-2(1H)-one as a white solid (Yield 0.11 g, 73%). HRMS(ES⁺) *m*/*z* Calcd for C₂₆H₁₉Cl₂F₃N₂OS + H [(M+H)⁺]: 535.0620. Found: 535.0611.

### Example 13a

### Preparation of intermediate 1-[5-fluoro-2-(trifluoromethyl)-phenyl]-3-trimethylsilyoxy-2-aza-1,3-butadiene

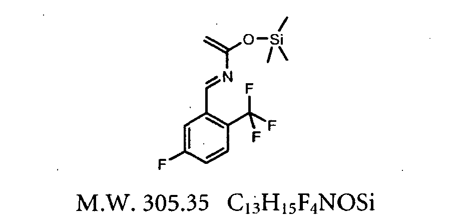

In a manner similar to the method described in example 1b, 5-fLuoro-2-(trifluoromethy)-benzaldehyde (1.9 g, 10 mmol) (Matrix) was used as the starting material in place of 3-chloro-benzaldehyde to react with 1,1,3,3,3-hexamethyldisilazane (1.61 g, 10 mmol), n-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-[5-fluoro-2-(trifluoromethyl)-phenyl]-3-trimethylsilyoxy-2-aza-1,3-butadiene as a yellow gum and used for the next step without further purification.

### Example 13b

### Preparation of intermediate racemic (2'R, 3R, 4'S)-6-chloro-4'-(3-chlorophenyl)-2'-[5-fluoro-2-(trifluoromethyl)-phenyl] spiro[3H-indole-3,3'-piperidine]-2,6'(1H)-dione

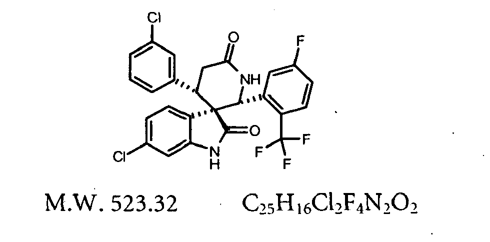

In a manner similar to the method described in example 6c, E/Z-6-chloro-3-(3-chlorobenzylidene)-2-oxo-2,3-dihydro-indole-1-carboxylic acid ethyl ester (0.41 g, 1.13 mmol) prepared in example 4b was reacted 1-[5-fluoro-2-(trinuoromethyl)-phenyl]-3-trimethylsilyoxy-2-aza-1,3-butadiene (2.9 g, 9.5 mmol) prepared in example 13a in toluene and then NaOH (0.2 g, 5 mmol) in methanol to give racemic (2'R, 3R, 4'S)-6-chloro-4'-(3-chlorophenyl)-2'-[5-fluoro-2-(trifluoromethyl)-phenyl] spiro[3H-indole-3,3'-piperidine]-2,6'(1H)-dione as an off-white soild (Yield 0.31 g, 52%). HRMS(ES⁺) *m*/*z* Calcd for C₂₅H₁₆Cl₂F₄N₂O₂ + H [(M+H)⁺]: 523.0598. Found: 523.0593.

### Example 13c

### Preparation of racemic (2'R, 3R, 4'S)-6-chloro-4'-(3-chlorophenyl)-2'-[5-fluoro-2-(trifluoromethyl)-phenyl] spiro[3H-indole-3,3'-piperidine]-2(1H)-one

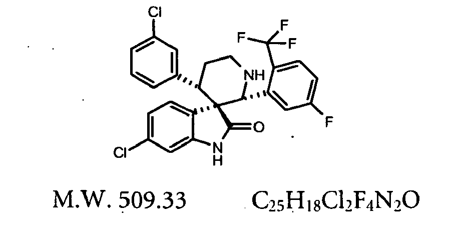

In a manner similar to the method described in example 6c, racemic (2'R, 3R, 4'S)-6-chloro-4'-(3-chlorophenyl)-2'-[5-fluoro-2-(trifluoromethyl)-phenyl] spiro[3H-indole-3,3'-piperidine]-2,6'(1H)-dione (104.2 mg, 0.20 mmol) prepared in example 13b was reacted with NaBH₄ (117 mg, 3.1 mmol) in methanol (2 mL) to give racemic (2'R, 3R, 4'S)-6-chloro-4'-(3-chlorophenyl)-2'-[5-fluoro-2-(trifluoromethyl)-phenyl] spiro[3H-indole-3,3'-piperidine]-2(1H)-one as a white solid (Yield 30.1 mg, 29.7%). HRMS(ES⁺) *m*/*z* Calcd for C₂₅H₁₈Cl₂F₄N₂O + H [(M+H)⁺]: 509.0805. Found: 509.0798.

### Example 14a

### Preparation of intermediate 1-(5-fluoro-2-methylphenyl)-3-trimethylsilyoxy-2-aza-1,3-butadiene

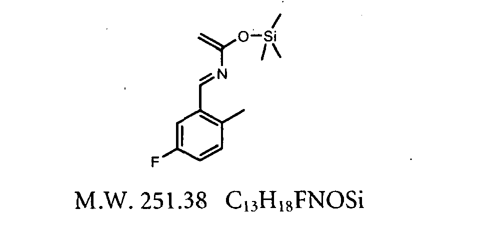

In a manner similar to the method described in example 1b, 5-fluoro-2-methybenzaldehyde (1.38 g, 10 mmol) (Platte) was used as the starting material in place of 3-chloro-benzaldehyde to react with 1,1,1,3,3,3-hexamethyldisilazane (1.61 g, 10 mmol), *n-*butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(5-fluoro-2-methylphenyl)-3-trimethylsilyoxy-2-aza-1,3-butadiene as a yellow gum and used for the next step without further purification.

### Example 14b

### Preparation of intermediate racemic (2'R, 3R, 4'S)-6-chloro-4'-(3-chlorophenyl)-2'-(5-fluoro-2-methylphenyl) spiro[3H-indole-3,3'-piperidine]-2,6'(1H)-dione

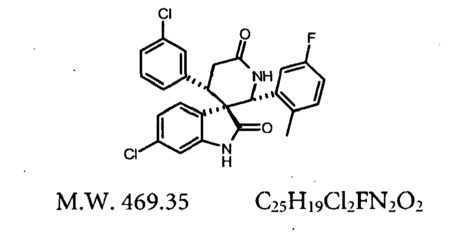

In a manner similar to the method described in example 6c, E/Z-6-chloro-3-(3-chlorobenzylidene)-2-oxo-2,3-dihydro-indole-1-carboxylic acid ethyl ester (0.25 g, 0.69 mmol) prepared in example 4b was reacted with 1-(5-fluoro-2-methylphenyl)-3-trimethylsilyoxy-2-aza-1,3-butadiene (2.5 g, 9.9 mmol) prepared in example 14a in toluene and then NaOH (1N, 5 mL, 5 mmol) in methanol to give racemic (2'R, 3R, 4'S)-6-chloro-4'-(3-chlorophenyl)-2'-(5-fluoro-2-methylphenyl) spiro[3H-indole-3,3'-piperidine]-2,6'(1H)-dione (Yield 0.13 g, 41%).
HRMS(ES⁺) *m*/*z* Calcd for C₂₅H₁₉Cl₂FN₂O₂+ H [(M+H)⁺]: 469.0881. Found: 469.0881.

### Example 14c

### Preparation of racemic (2'R, 3R, 4'S)-6-chloro-4'-(3-chlorophenyl)-2'-(5-fluoro-2-methylphenyl) spiro[3H-indole-3,3'-piperidine]-2(1H)-one

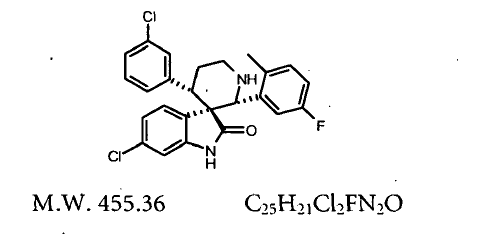

In a manner similar to the method described in example 1d, racemic (2'R, 3R, 4'S)-6-chloro-4'-(3-chlorophenyl)-2'-(5-fluoro-2-methylphenyl) spiro[3H-indole-3,3'-piperidine]-2,6'(1H)-dione (80.1 mg, 0.17 mmol) prepared in example 14b was reacted with NaBH₄ (117 mg, 3.1 mmol) in methanol (2 mL) to give racemic (2'R, 3R, 4'S)-6-chloro-4'-(3-chlorophenyl)-2'-(5-fluoro-2-methylphenyl) spiro[3H-indole-3,3'-piperidine]-2(1H)-one as a white solid (Yield 23.1 mg, 29.7%).
HRMS(ES⁺) *m*/*z* Calcd for C₂₅H₂₁Cl₂FN₂O + H [(M+H)⁺]: 455.1088. Found: 455.1091.

### Example 15a

### Preparation of intermediate E/Z-6-chloro-3-(3-chloro-benzylidene)-2-oxo-2,3-dihydroindole-1-caiboxylic acid tert-butyl ester

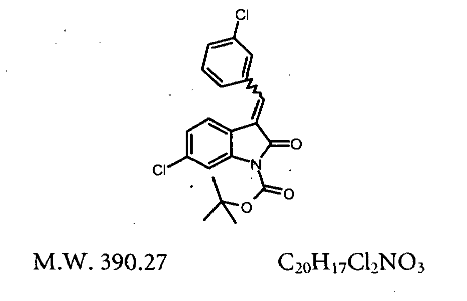

To a solution of E/Z-6-chloro-3-(3-chloro-benzylidene)-1,3-dihydro-indol-2-one prepared in example 4a (1g, 3.4 mmol) in dichloromethane (50 mL) at room temperature was added Di-*tert*-butyl-dicarbonate (1.5 g, 6.9 mmol) (Aldrich), followed by the addition of 4-dimethylaminopyridine (1g, 8.2 mmol). The reaction mixture was stirred at room temperature for 1 h. The mixture was then concentrated and the residue was purified by chromatography to give E/Z-6-chloro-3-(3-chloro-benzylidene)-2-oxo-2,3-dihydro-indole-1-carboxylic acid *tert*-butyl ester as an orange solid (Yield 1.3 g, 96%).

### Example 15b

### Preparation of intermediate 1-(2,4-difluorophenyl)-3-trimethylsilyoxy-2-aza-1,3-butadiene

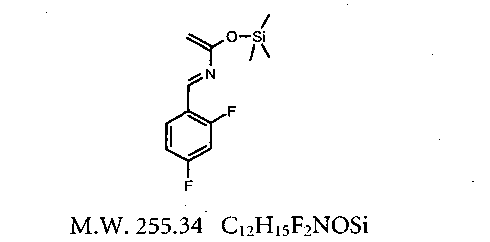

In a manner similar to the method described in example 1b, 2,4-difluoro-benzaldehyde (1.4 g, 10 mmol) (Aldrich) was used as the starting material in place of 3-chloroben-zaldehyde to react with 1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(2,4-difluorophenyl)-3-trimethylsilyoxy-2-aza-1,3-butadiene as a yellow gum and used for the next step without further purification.

### Example 15c

### Preparation of intermediate racemic (2'R, 3R, 4'S)-6-chloro-4'-(3-chlorophenyl)-2'-(2,4-difluorophenyl) spiro[3H-indole-3,3'-piperidine]-2,6'(1H)-dione

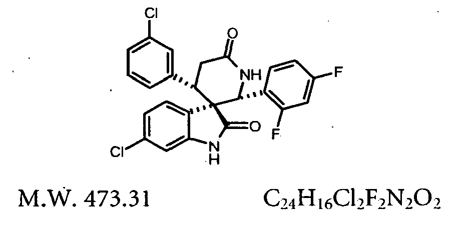

To a solution of 1-(2,4-difluorophenyl)-3-trimethylsilyoxy-2-aza-1,3-butadiene prepared in example 15b (2.4 g, 9.40 mmol) in toluene (30 mL) was added E/Z-6-chloro-3-(3-chlorobenzylidene)-2-oxo-2,3-dihydro-indole-1-carboxylic acid *tert*-butyl ester prepared in Example 15a (0.3 g, 0.77 mmol). The reaction mixture was stirred under nitrogen in a sealed tube at 140 °C for 0.5 h. After the solution was cooled to room temperature, methanol (10 mL) was added. The reaction mixture was filtered through a short pad of celite gel and washed with ethyl acetate. The filtrate was concentrated. The residue was dissolved in dichloromethane (20 mL) and trifluoroactic acid (15 mL) was added. After the reaction mixture was stirred at room temperature for 1h, the mixture was concentrated. The residue was partitioned between saturated NaHCO₃ solution and ethyl acetate. The aqueous layer was extracted with ethyl acetate. The combined organic layer was dried over Na₂SO₄ and concentrated. The residue was purified by chromatography (EtOAc:hexanes =2:1) to give racemic (2'R, 3R, 4'5)-6-chloro-4'-(3-chlorophenyl)-2'-(2,4-difluorophenyl) spiro[3H-indole-3,3'-piperidine]-2,6'(1H)-dione as an off-white solid (Yield 0.23 g, 63.9%).
HRMS(ES⁺) *m*/*z* Calcd for C₂₄H₁₆Cl₂F₂N₂O₂ + H [(M+H)⁺]: 473.0630. Found: 473.0630.

### Example 15d

### Preparation of racemic (2'R, 3R, 4'S)-6-chloro-4'-(3-chlorophenyl)-2'-(2,'4-difluorophenyl) spiro[3H-indole-3,3'-piperidine]-2(1H)-one

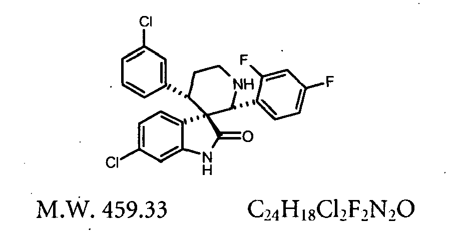

In a manner similar to the method described in example 1d, racemic (2'R, 3R, 4'S)-6-chloro-4'-(3-chlorophenyl)-2'-(2,4-difluorophenyl) spiro[3H-indole-3,3'-piperidine]-2,6'(1H)-dione (211.2 mg, 0.45 mmol) prepared in example 15c was reacted with NaBH₄ (170 mg, 4.5 mmol) in methanol (2 mL) to give racemic ₍2'R, 3R, 4'S)-6-chloro-4'-(3-chlorophenyl)-2'-(2, 4-difluorophenyl) spiro[3H-indole-3,3'-piperidine]-2(1H)-one as a white solid (Yield 30.6 mg, 14.9 %).
HRMS(ES⁺) *m*/*z* Calcd for C₂₄H₁₈Cl₂F₂N₂O + H [(M+H)⁺]: 459.0837. Found: 459.0835.

### Example 16a

### Preparation of intermediate 1-(5-fluoro-2-methoxyphenyl)-3-trimethylsilyoxy-2-aza-1,3-butadiene

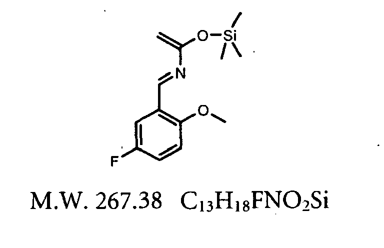

In a manner similar to the method described in example 1b, 5-fluoro-2-methoxy-benzaldehyde (1.5 g, 10 mmol) (Aldrich) was used as the starting material in place of 3-chloro-benzaldehyde to react with 1,1,1,3,3,3-hexamethyldisilazane (1.6 g, 10 mmol), n-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(5-fluoro-2-methoxyphenyl)-3-trimethylsilyoxy-2-aza-1,3-butadiene as a yellow gum and used for the next step without further purification.

### Example 16b

### Preparation of intermediate racemic (2'R, 3R, 4'S)-6-chloro-4'-(3-chlorophenyl)-2'-(5-fluoro-2-methoxyphenyl) spiro[3H-indole-3,3'-piperidine]-2,6'(1H)-dione

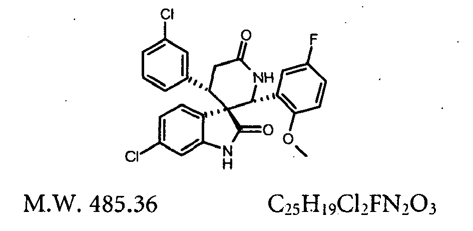

In a manner similar to the method described in example 15c, E/Z-6-chloro-3-(3-chlorobenzylidene)-2-oxo-2,3-dihydro-indole-1-carboxylic acid tert-butyl ester prepared in example 15a (0.3 g, 0.77 mmol) was reacted with 1-(5-fluoro-2-methoxyphenyl)-3-trimethylsilyoxy-2-aza-1,3-butadiene (2.4 g, 9.0 mmol) prepared in example 16a in toluene and then trifluoroacetic acid (15 mL) in dichloromethane to give racemic (2'R, 3R, 4'S)-6-chloro-4'-(3-chlorophenyl)-2'-(5-fluoro-2-methoxyphenyl) spiro[3H-indole-3,3'-piperidine]-2,6'(1H)-dione (Yield 0.18 g, 49%).
HRMS(ES⁺) *m*/*z* Calcd for C₂₅H₁₉Cl₂FN₂O₃+ H [(M+H)⁺]: 485.0830. Found: 485.0827.

### Example 16c

### Preparation of racemic (2'R, 3R, 4'S)-6-chloro-4'-(3-chlorophenyl)-2'-(5-ftuoro-2-methoxyphenyl) spiro[3H-indole-3,3'-piperidine]-2(1H)-one

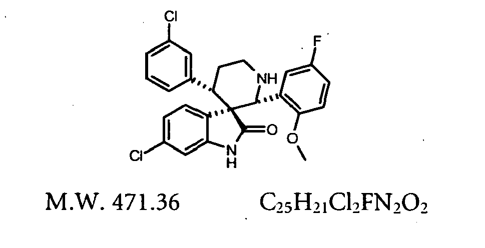

In a manner similar to the method described in example 1d, racemic (2'R, 3R, 4'S)-6-chloro-4'-(3-chlorophenyl)-2'-(5-fluoro-2-methoxyphenyl) spiro[3H-indole-3,3'-piperidine]-2,6'(1H)-dione (154.3 mg, 0.32 mmol) prepared in example 16b was reacted with NaBH₄ (121.0 mg, 3.2 mmol) in methanol (2 mL) to give racemic (2'R, 3R, 4'S)-6-chloro-4'-(3-chlorophenyl)-2'-(5-fluoro-2-methoxyphenyl) spiro[3H-indole-3,3'-piperidine]-2(1H)-one as a white solid (Yield 21.5 mg, 14.3 %).
HRMS(ES⁺) *m*/*z* Calcd for C₂₅H₂₁Cl₂FN₂O₂ + H [(M+H)⁺]: 471.1037. Found: 471.1036.

### Example 17a

### Preparation of intermediate 1-(1-naphthalenyl)-3-trimethylsilyoxy-2-aza-1,3-butadiene

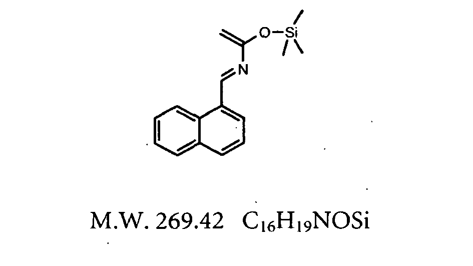

In a manner similar to the method described in example 1b, naphthalene-1-carbaldehyde (1.6 g, 10 mmol) (Lancaster) was used as the starting material in place of 3-chloro-benzaldehyde to react with 1,1,1,3,3,3-hexamethyldisilazane (1.6 g, 10 mmol), n-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(1-naphthalenyl)-3-trimethylsilyoxy-2-aza-1,3-butadiene as a yellow gum and used for the next step without further purification.

### Example 17b

### Preparation of intermediate racemic (2'R, 3R, 4'S)-6-chloro-4'-(3-chlorophenyl)-2'-(1-naphthalenyl) spiro[3H-indole-3,3'-piperidine]-2,6'(1H)-dione

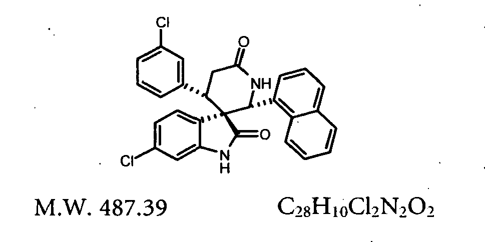

In a manner similar to the method described in example 15c, E/Z-6-chloro-3-(3-chlorobenzylidene)-2-oxo-2,3-dihydro-indole-1-carboxylic acid *tert-*butyl ester prepared in example 15a (0.3 g, 0.77 mmol) was reacted with 1-(1-naphthalenyl)-3-trimethylsilyoxy-2-aza-1,3-butadiene (2.7 g, 10.0 mmol) prepared in example 17a in toluene and then trifluoroacetic acid (15 mL) in dichloromethane to give racemic (2'R, 3R, 4'S)-6-chloro-4'-(3-chlorophenyl)-2'-(1-naphthalenyl) spiro[3H-indole-3,3'-piperidine]-2,6'(1H)-dione (Yield 0.21 g, 57%).
HRMS(ES⁺) *m*/*z* Calcd for C₂₈H₁₀Cl₂N₂O₂ + H [(M+H)⁺]: 487.0975. Found: 487.0975.

### Example 17c

### Preparation of racemic (2'R, 3R, 4'S)-6-chloro-4'-(3-chlorophenyl)-2'-(1-naphthalenyl) spiro [3H-indole-3,3'-piperidine]-2(1H)-one

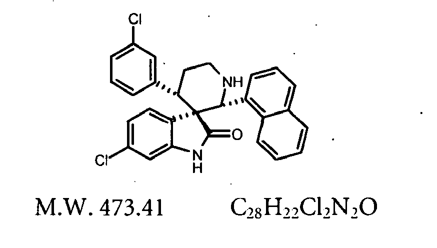

In a manner similar to the method described in example 1d, racemic (2'R, 3R, 4'S)-6-chloro-4'-(3-chlorophenyl)-2'-(1-naphthalenyl) spiro[3H-indole-3,3'-piperidine]-2,6'(1H)-dione (201.3 mg, 0.41 mmol) prepared in example 17b was reacted with NaBH₄ (155.0 mg, 4.1 mmol) in methanol (2 mL) to give racemic (2'R, 3R, 4'S)-6-chloro-4'-(3-chlorophenyl)-2'-(1-naphthalenyl) spiro[3H-indole-3,3'-piperidine]-2(1H)-one as a white solid (Yield 51.5 mg, 26.3 %).
HRMS(ES⁺) *m*/*z* Calcd for C₂₈H₂₂Cl₂N₂O + H [(M+H)⁺]: 473.1182. Found: 473.1182.

### Example 18a

### Preparation of intermediate 1-(3-pyridinyl)-3-trimethylsilyoxy-2-aza-1,3-butadiene

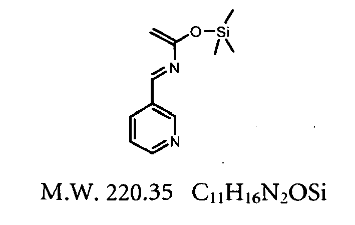

In a manner similar to the method described in example 1b, pyridine-3-carbaldehyde (1.1 g, 10 mmol) (Aldrich) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(3-pyridinyl)-3-trimethylsilyoxy-2-aza-1,3-butadiene as a yellow gum and used for the next step without further purification.

### Example 18b

### Preparation of intermediate racemic (2'R, 3R, 4'S)-6-chloro-4'-(3-chlorophenyl)-2'-(3-pyridinyl) spiro[3H-indole-3,3'-piperidine]-2,6'(1H)-dione

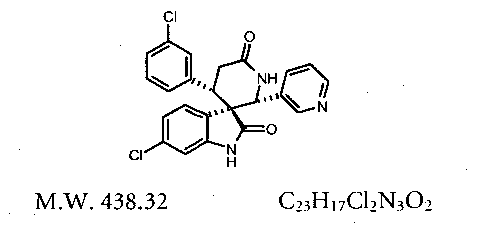

In a manner similar to the method described in example 15c, E/Z-6-chloro-3-(3-chlorobenzylidene)-2-oxo-2,3-dihydro-indole-1-carboxylic acid tert-butyl ester prepared in example 15a (0.3 g, 0.77 mmol) was reacted with 1-(3-pyridinyl)-3-trimethylsilyoxy-2-aza-1,3-butadiene (2.1 g, 9.5 mmol) prepared in example 18a in toluene and then trifluoroacetic acid (10 mL) in dichloromethane to give racemic (2'R, 3R, 4'S)-6-chloro-4'-(3-chlorophenyl)-2'-(3-pyridinyl) spiro[3H-indole-3,3'-piperidine]-2,6'(1H)-dione (0.21 g, 62%).
HRMS(ES⁺) *m*/*z* Calcd for C₂₃H₁₇Cl₂N₃O₂ + H [(M+H)⁺]: 438.0771. Found: 438.0771.

### Example 18c

### Preparation of racemic (2'R, 3R, 4'S)-6-chloro-4'-(3-chlorophenyl)-2'-(3-pyridinyl) spiro[3H-indole-3,3'-piperidine]-2(1H)-one

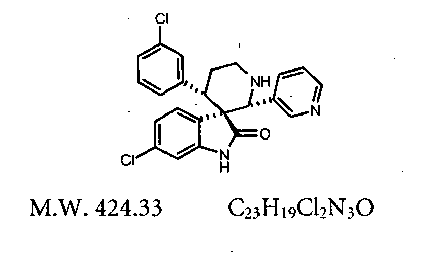

In a manner similar to the method described in example 1d, racemic (2'R, 3R, 4'S)-6-chloro-4'-(3-chlorophenyl)-2'-(3-pyridinyl) spiro[3H-indole-3,3'-piperidine]-2,6'(1H)-dione (180.3 mg, 0.41 mmol) prepared in example 18b was reacted with NaBH₄ (126 mg, 3.3 mmol) in methanol (2 mL) to give racemic (2'R, 3R, 4'S)-6-chloro-4'-(3-chlorophenyl)-2'-(3-pyridinyl) spiro[3H-indole-3,3'-piperidine]-2(1H)-one as a white solid (Yield 50.1 mg, 29.4 %).
HRMS(ES⁺) *m*/*z* Calcd for C₂₃H₁₉Cl₂N₃O + H [(M+H)⁺]: 424.0978. Found: 424.0977.

### Example 19a

### Preparation of intermediate 1-(3,4-difluorophenyl)-3-trimethylsilyoxy-2-aza-1,3-butadiene

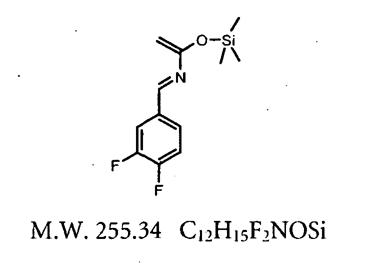

In a manner similar to the method described in example 1b, 3,4-difluoro-benzaldehyde (1.4 g, 10 mmol) (Aldrich) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(3,4-difluorophenyl)-3-trimethylsilyoxy-2-aza-1,3-butadiene as an orange gum and used for the next step without further purification.

### Example 19b

### Preparation of intermediate racemic (2'R, 3R, 4'S)-6-chloro-4'-(3-chlorophenyl)-2'-(3, 4-difluorophenyl) spiro[3H-indole-3,3'-piperidine]-2,6'(1H)-dione

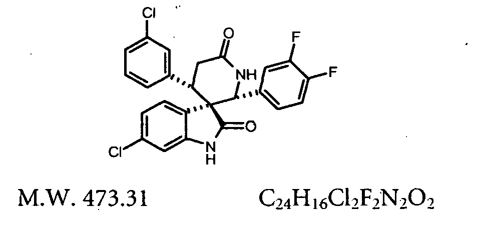

In a manner similar to the method described in example 15b, E/Z-6-chloro-3-(3-chlorobenzylidene)-2-oxo-2,3-dihydro-indole-1-carboxylic acid *tert*-butyl ester prepared in example 15a (0.3 g, 0.77 mmol) was reacted with 1-(3,4-difluorophenyl)-3-trimethylsilyoxy-2-aza-1,3-butadiene (2.1 g, 8.2 mmol) prepared in example 62a in toluene and then trifluoroacetic acid (10 mL) in dichloromethane to give racemic (2'R, 3R, 4'S)-6-chloro-4'-(3-chlorophenyl)-2'-(3, 4-difluorophenyl) spiro[3H-indole-3,3'-piperidine]-2,6'(1H)-dione (0.4 g, 100%).
HRMS(ES⁺) *m*/*z* Calcd for C₂₄H₁₆Cl₂F₂N₂O₂ + H [(M+H)⁺]: 473.0630. Found: 473.0631.

### Example 19c

### Preparation of racemic (2'R, 3R, 4'S)-6-chloro-4'-(3-chlorophenyl)-2'-(3, 4-difluorophenyl) spiro[3H-indole-3,3'-piperidine]-2(1H)-one

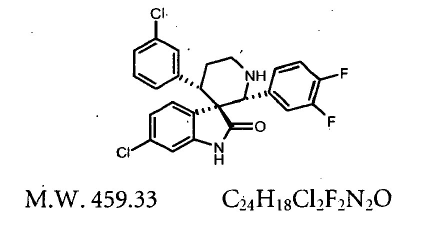

In a manner similar to the method described in example 1d, racemic (2'R, 3R, 4'S)-6-chloro-4'-(3-chlorophenyl)-2'-(3,4-difluorophenyl) spiro[3H-indole-3,3'-piperidine]-2,6'(1H)-dione (158 mg, 0.33 mmol) prepared in example 19b was reacted with NaBH4 (126 mg, 3.3 mmol) in methanol (2 mL) to give racemic (2'R, 3R, 4'S)-6-chloro-4'-(3-chlorophenyl)-2'-(3,4-difluorophenyl) spiro[3H-indole-3,3'-piperidine]-2(1H)-one as a white solid (Yield 48.6 mg, 32.1%).
HRMS(ES⁺) *m*/*z* Calcd for C₂₄H₁₈Cl₂F₂N₃O + H [(M+H)⁺]: 459.0837. Found: 459.0835.

### Example 20a

### Preparation of intermediate racemic (2'R, 3R, 4'S)-6-chloro-4'-(3-chlorophenyl)-2'-(3,4-difluorophenyl)- 6'-thioxo spiro[3H-indole-3,3'-piperidine]-2(1H)-one

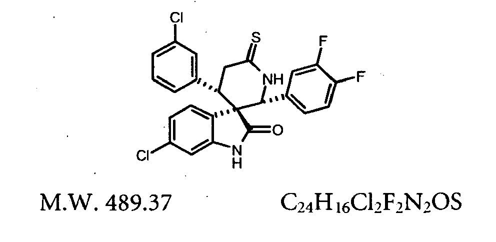

In a manner similar to the method described in example 2d, racemic (2'R, 3R, 4'S)-6-chloro-4'-(3-chlorophenyl)-2'-(3, 4-difluorophenyl) spiro[3H-indole-3,3'-piperidine]-2,6'(1H)-dione (0.3 g, 0.63 mmol) prepared in example 19b was reacted with 2,4-bis-(4-methoxyphenyl)-1,3-dithia-2,4-diphosphetane 2,4-disulfide (0.32 g, 0.77 mmol) in toluene to give racemic (2'R, 3R, 4'S)-6-chloro-4'-(3-chlorophenyl)-2'-(3,4-difluorophenyl)-6'-thioxo spiro[3H-indole-3,3'-piperidine]-2(1H)-one (Yield 0.29 g, 94%).
HRMS(ES⁺) *m*/*z* Calcd for C₂₄H1₆Cl₂F₂N₂OS +H [(M+H)⁺]: 489.0401. Found: 489.0402.

### Example 20b

### Preparation of intermediate racemic (2'R, 3R, 4'S)-[6-chloro-4'-(3-chlorophenyl)-2'-(3, 4-difluorophenyl)-2,3-dihydro-2-oxospiro[3H-indole-3,3'-piperidin]-6-ylene] hydrazine carboxylic acid ethyl ester

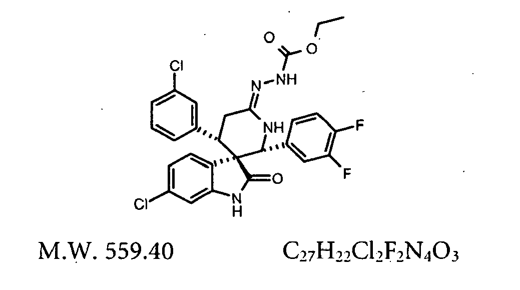

To a solution of racemic (2'R, 3R, 4'S)-6-chloro-4'-(3-chlorophenyl)-2'-(3,4-difluorophenyl)-6'-thioxo spiro[3H-indole-3,3'-piperidine]-2(1H)-one (0.24 g, 0.49 mmol) prepard in example 20a in tetrahydrofuran (20 mL) was added ethyl carbazate (0.1 g, 0.99 mmol) (Aldrich) and mercuric acetate (0.24 g, 0.76 mmol). After the reaction mixture was stirred at room temperature for 2 h, the reaction mixture was filtered through a short pad of celite. The filtrate was concentrated and the residue was purified by chromatography (EtOAc) to give racemic (2'R, 3R, 4'S)- [6-chloro-4'-(3-chlorophenyl)-2'-(3, 4-difluorophenyl)-2,3-dihydro-2-oxospiro[3H-indole-3,3'-piperidin]-6-ylene] hydrazine carboxylic acid ethyl ester as a white solid (Yield 0.21 g, 77.8 %).

### Example 20c

### Preparation of racemic (5'R, 3R, 7'S)-6-chloro-7'-(3-chlorophenyl)-5'-(3,4-difluorophenyl)-5',6',7',8'-tetrahydro-3'-hydroxyspiro[3H-indole-3,6'-(1,2,4-triazolo[4,3-a]pyridine)]-2(1H)- one

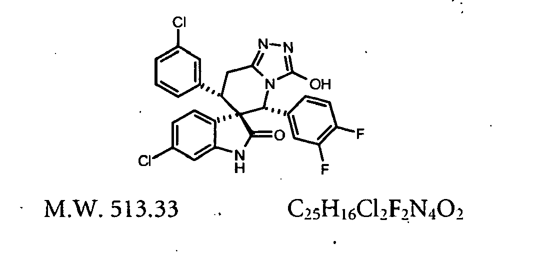

The solution of racemic ((2'R, 3R, 4'S)-[6-chloro-4'-(3-chlorophenyl)-2'-(3,4-difluorophenyl)-2,3-dihydro-2-oxospiro[3H-indole-3,3'-piperidin]-6-ylene] hydrazine carboxylic acid ethyl ester prepared in example 20a (0.17g, 0.30 mmol) in toluene (20 mL) was heated in a sealed tube at 150 °C for 3 h. After the solution was cooled to room temperature, the solvent was removed and the residue was triturated with EtOAc/Hexane to give racemic (5'R, 3R, 7'S)-6-chloro-7'-(3-chlorophenyl)-5'-(3, 4-difluorophenyl)-5',6',7',8'-tetrahydro-3'-hydroxyspiro[3H-indole-3,6'-(1,2,4-triazolo[4,3-a]pyridine)]-2(1H)-one as a white solid (Yield: 0.11 g, 70.5%)
HRMS(ES⁺) *m*/*z* Calcd for C₂₅H₁₆Cl₂F₂N₄O₂ + H [(M+H)⁺]: 513.0691 Found: 513.0689.

### Example 21a

### Preparation of intermediate racemic (2'R, 3R, 4'S)-6-chloro-4'-(3-chlorophenyl)-2'-(5-' fluoro-2-methylphenyl)-6'-thioxo spiro[3H-indole-3,3'-piperidine]-2(1H)-one

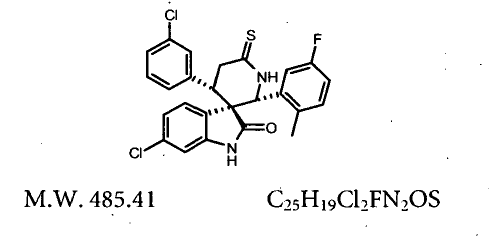

In a manner similar to the method described in example 2d, racemic (2'R, 3R, 4'S)-6-chloro-4'-(3-chlorophenyl)-2'-(5-fluoro-2-methylphenyl) spiro[3H-indole-3,3'-piperidine]-2,6'(1H)-dione (0.1 g, 0.21 mmol) prepared in example 14b was reacted with 2,4-bis-(4-methoxyphenyl)-1,3-dithia-2,4-diphosphetane 2,4-disulfide (0.2 g, 0.49 mmol) in toluene to give racemic (2'R, 3R, 4'S)-6-chloro-4'-(3-chlorophenyl)-2'-(5-fluoro-2-methylphenyl)-6'-thioxo spiro[3H-indole-3,3'-piperidine]-2(1H)-one as a white solid (Yield 0.095 g, 92%).
HRMS (ES⁺) *m*/*z* Calcd for C₂₅H₁₉Cl₂FN₂OS +H [(M+H)⁺]: 485.0652. Found: 485.0648.

### Example 21b

### Preparation of intermediate racemic (2'R, 3R, 4'S)-[6-chloro-4'-(3-chlorophenyl)-2'-(5-fluoro-2-methylphenyl)-2,3-dihydro-2-oxospiro [3H-indole-3,3'-piperidin]-6-ylene] hydrazine carboxylic acid ethyl ester

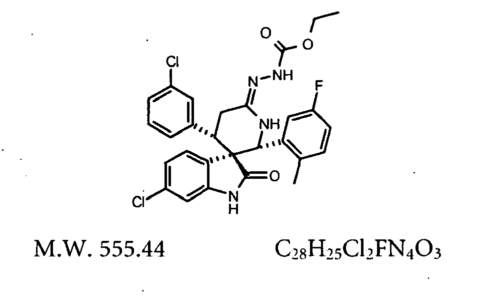

In a manner similar to the method described in example 20b, (2'R, 3R, 4'S)-6-chloro-4'-(3-chlorophenyl)-2'-(5-fluoro-2-methylphenyl)-6'-thioxo spiro[3H-indole-3,3'-piperidine]-2(1H)-one (0.17 g, 0.35 mmol) prepard in example 55 was reacted with ethyl carbazate (0.15 g, 1.49 mmol), mercuric acetate (0.26 g, 0.82 mmol) and triethylamine (0.17 g, 1.69 mmol) in tetrahydrofuran (20 mL) to give racemic (2'R, 3R, 4'S)-[6-chloro-4'-(3-chlorophenyl)-2'-(5-fluoro-2-methylphenyl)-2,3-dihydro-2-oxospiro [3H-indole-3,3'-piperidin]-6-ylene] hydrazine carboxylic acid ethyl ester as a white solid (Yield 0.14 g, 73.7%).

### Example 21c

### Preparation of racemic (5'R, 3R, 7'S)-6-chloro-7'-(3-chlorophenyl)-5'-(5-fluoro-2-methylphenyl)-5',6',7',8'-tetrahydro-3'-hydroxyspiro[3H-indole-3,6'-(1,2,4-triazolo [4,3-a] pyridine)]-2(1H)-one

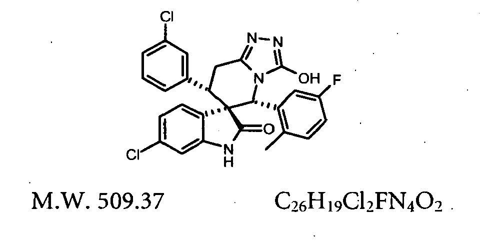

The solution of racemic ((2'R, 3R, 4'S)-[6-chloro-4'-(3-chlorophenyl)-2'-(5-fluoro-2-methylphenyl)-2,3-dihydro-2-oxospiro[3H-indole-3,3'-piperidin]-6-ylene] hydrazine carboxylic acid ethyl ester prepared in example 21b (0.11 g, 1.98 mmol) in toluene (20 mL) was heated in a sealed tube at 165 °C for 4 h. After the solution was cooled to room temperature, the solvent was removed and the residue was triturated with EtOAc/Hexane to give racemic (5'R, 3R, 7'S)-6-chloro-7'-(3-chlorophenyl)-5'-(5-fluoro-2-methylphenyl)-5',6',7',8'-tetrahydro-3'-hydroxyspiro[3H-indole-3,6'-(1,2,4-triazolo[4,3-a]pyridine)]-2(1H)-one as a white solid (Yield: 0.058 g, 58%)
HRMS(ES⁺) *m*/*z* Calcd for C₂₆H₁₉Cl₂FN₄O₂ + H [(M+H)⁺]: 509.0942 Found: 509.0943.

### Example 22a

### Preparation of intermediate 2,3-difluoro-6-methyl-benzaldehyde

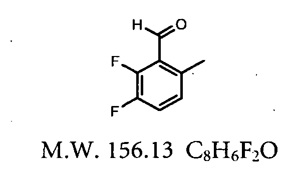

To a solution of 1,2-difluoro-4-methyl-benzene (5.0 g, 39 mmol) in tetrahydrofuran (200 mL) at -78 °C was added lithium diisopropyl amine (24 mL, 1.8 M in THF, 43 mmol) dropwise during a period of 15 min. The mixture was stirred at -78 °C for another 20 min. Then N,N-dimethyl-formamide (3.6 mL, 47 mmol) was added in one portion.

The mixture was stirred at -78 °C for 10 min, then quenched with acetic acid (9.4 g, 1.56 mmol) and followed by the addition of water (37.6 mL). The mixture was partitioned between ethyl acetate and water. The organic layer was separated, concentrated. The residue was purified by chromatography (EtOAc:hexanes =1:1) to give 2,3-difluoro-6-methyl-benzaldehyde as colorless oil (Yield: 3.5 g, 57.5%).

### Example 22b

### Preparation of intermediate 1-(2, 3-difluoro-6-methylphenyl)-3-trimethylsilyoxy-2-aza-1,3-butadiene

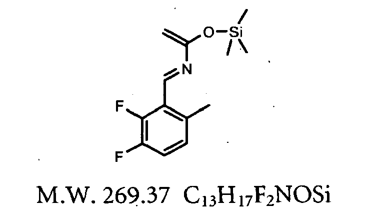

In a manner similar to the method described in example 1b, 2,3-difluoro-6-methyl-benzaldehyde (1.56 g, 10 mmol) prepared in example 78a was used as the starting material in place of 3-chloro-benzaldehyde to react with 1,1,1,3,3,3-hexamethyldisilazane (1.6 g, 10 mmol), n-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(2, 3-difluoro-6-methylphenyl)-3-trimethylsilyoxy-2-aza-1,3-butadiene as an orange gum and used for the next step without-further purification.

### Example 22c

### Preparation of intermediate racemic (2'R, 3R, 4'S)-6-chloro-4'-(3-chlorophenyl)-2'-(2, 3-difluoro-6-methylphenyl) spiro[3H-indole-3,3'-piperidine]-2,6'(1H)-dione

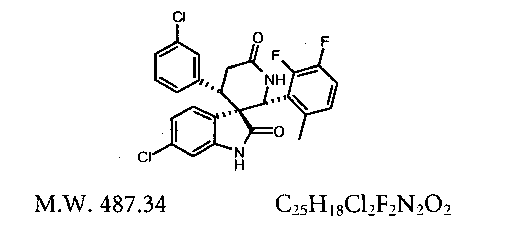

In a manner similar to the method described in example 15c, E/Z-6-chloro-3-(3-chlorobenzylidene)-2-oxo-2,3-dihydro-indole-1-carboxylic acid tert-butyl ester prepared in example 15a (0.4 g, 1.03 mmol) was reacted with 1-(2, 3-difluoro-6-methylphenyl)-3-trimethylsilyoxy-2-aza-1,3-butadiene (2.7 g, 10.0 mmol) prepared in example 22b in toluene and then trifluoroacetic acid (10 mL) in dichloromethane to give racemic (2'R, 3R, 4'S)-6-chloro-4'-(3-chlorophenyl)-2'-(2, 3-difluoro-6-methylphenyl) spiro[3H-indole-3,3'-piperidine]-2,6'(1H)-dione (Yield: 0.41 g, 83.7%). HRMS(ES⁺) *m*/*z* Calcd for C₂₅H₁₈Cl₂F₂N₂O₂ + H [(M+H)⁺]: 487.0786 Found: 487.0780.

### Example 22d

### Preparation of racemic (2'R, 3R, 4'S)-6-chloro-4'-(3-chlorophenyl)-2'-(2, 3-difluoro-6-methylphenyl) spiro[3H-indole-3,3'-piperidine]-2(1H)-one

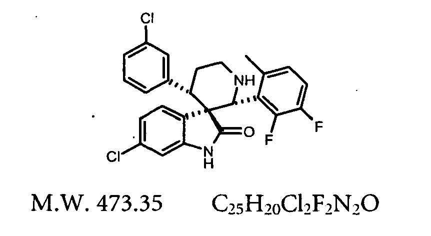

In a manner similar to the method described in example 1d, racemic (2'R, 3R, 4'S)-6-chloro-4'-(3-chlorophenyl)-2'-(2,3-difluoro-6-methylphenyl) spiro[3H-indole-3,3'-piperidine]-2,6'(1H)-dione (48.7 mg, 0.1 mmol) prepared in example 22c was reacted with NaBH₄ (37.8 mg, 1.00 mmol) in methanol (2 mL) to give racemic (2'R, 3R, 4'S)-6-chloro-4'-(3-chlorophenyl)-2'-(2, 3-difluoro-6-methylphenyl) spiro[3H-indole-3,3'-piperidine]-2(1H)-one as a white solid (Yield 25.3 mg, 53.5 %).
HRMS(ES⁺) *m*/*z* Calcd for C₂₅H₂₀Cl₂F₂N₂O + H [(M+H)⁺]: 473.0994. Found: 424.0992.

### Example 23a

### Preparation of intermediate E/Z-6-chloro-3-(3-chloro-benzylidene)-5-fluoro-1,3-dihydro-indol-2-one

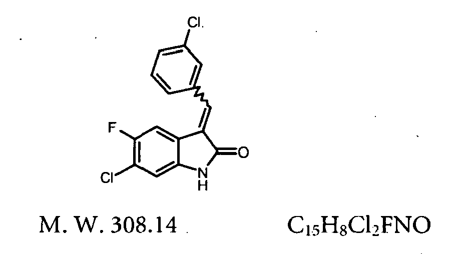

In a manner similar to the method described in example 1a, 6-chloro-5-fluoro-1,3-dihydro-indol-2-one (0.25 g, 1.35 mmol) was reacted with 3-chloro-benzaldehyde (0.34 g, 2.44 mmol) and pyrrolidine (0.19 g, 2.68 mmol) in methanol to give a mixture of E-and Z-6-chloro-3-(3-chloro-benzylidene)-5-fluoro-1,3-dihydro-indol-2-one as a yellow solid.

6-chloro-5-fluoro-1,3-dihydro-indol-2-one was prepared according to literature procedure without modification: EP 153 818.

### Example 23b

### Preparation of intermediate E/Z-6-chloro-3-(3-chloro-benzylidene)-5-fluoro-2-oxo-2,3-dihydro-indole-1-carboxylic acid tert-butyl ester

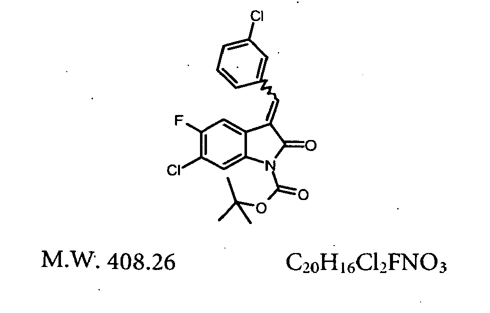

In a manner similar to the method described in example 15a, E/Z-6-chloro-3-(3-chlorobenzylidene)-5-fluoro-1,3-dihydro-indol-2-one (0.45 g, 1.46 mmol) was reacted with di-*tert*-butyl-dicarbonate (0.4 g, 1.83 mmol) (Aldrich), triethyl amine (0.5 g, 4.95 mmol) and 4-dimethylaminopyridine (5 mg) in dichloromethane (30 mL) to give E/Z-6-chloro-3-(3-chloro-benzylidene)-5-fluoro-2-oxo-2,3-dihydro-indole-1-carboxylic acid tert-butyl ester as a yellow solid (Yield: 0.6 g, 100% ).

### Example 23c

### Preparation of intermediate racemic (2'R, 3R, 4'S)-6-chloro-4'-(3-chlorophenyl)-5-fluoro-2'-(5-fluoro-2-methylphenyl) spiro[3H-indole-3,3'-piperidine]-2,6'(1H)-dione

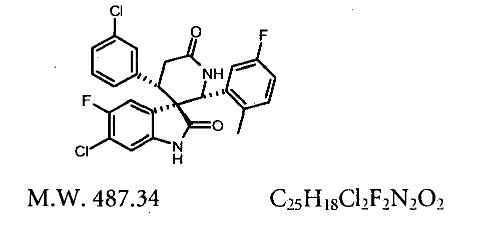

In a manner similar to the method described in example 15b, E/Z-6-chloro-3-(3-chlorobenzylidene)-5-fluoro-2-oxo-2,3-dihydro-indole-1-carboxylic acid tert-butyl ester prepared in example 23b (0.4 g, 0.98 mmol) was reacted with 1-(5-fluoro-2-methylphenyl)-3-trimethylsilyoxy-2-aza-1,3-butadiene (2.1 g, 8.37 mmol) prepared in example 14a in toluene and then trifluoroacetic acid (10 mL) in dichloromethane to give racemic (2'R, 3R, 4'S)-6-chloro-4'-(3-chlorophenyl)-5-fluoro-2' -(5-fluoro-2-methylphenyl) spiro[3H-indole-3,3'-piperidine]-2,6'(1H)-dione (Yield: 0.35 g, 72.9% ). HRMS(ES⁺) *m*/*z* Calcd for C₂₅H₁₈Cl₂F₂N₂O₂ + H [(M+H)⁺]: 487.0786. Found: 487.0779.

### Example 23d

### Preparation of racemic (2'R, 3R, 4'S)-6-chloro-4'-(3-chlorophenyl)-5-fluoro-2'-(5-fluoro-2-methylphenyl) spiro[3H-indole-3,3'-piperidine]-2(1H)-one

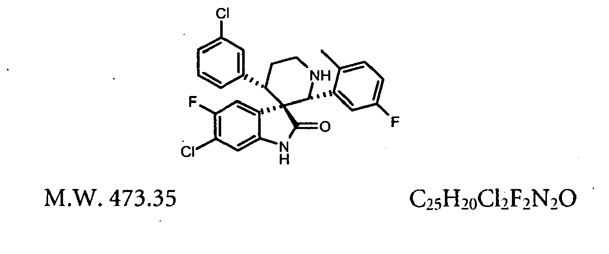

In a manner similar to the method described in example 1d, racemic (2'R, 3R, 4'S)-6-chloro-4'-(3-chlorophenyl)-5-fluoro-2'-(5-fluoro-2-methylphenyl) spiro[3H-indole-3,3'-piperidine]-2,6'(1H)-dione (48.3 mg, 0.1 mmol) prepared in example 23c was reacted with excess of NaBH₄ in methanol (2 mL) to give racemic (2'R, 3R, 4'S)-6-chloro-4'-(3-chlorophenyl)-5-fluoro-2'-(5-fluoro-2-methylphenyl) spiro[3H-indole-3,3'-piperidine]-2(1H)-one as a white solid (Yield 18.8 mg, 39.9 %).
HRMS(ES⁺) *m*/*z* Calcd for C₂₅H₂₀Cl₂F₂N₂O + H [(M+H)⁺]: 473.0994. Found: 473.0992.

### Example 24a

### Preparation of intermediate 1-(1-ethyl-propenyl)-3-trimethylsilyoxy-2-aza-1,3-butadiene

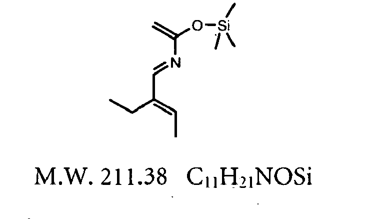

In a manner similar to the method described in example 1b, 2-ethyl-but-2-enal (1.54 g, 10 mmol) (TCI-US) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(1-ethyl-p.ropenyl)-3-trimethylsilyoxy-2-aza-1,3-butadiene and used for the next step without further purification.

### Example 24b

### Preparation of intermediate racemic (2'R, 3R, 4'S)-6-chloro-4'-(3-chlorophenyl)-2'-( 1-ethyl-propenyl) spiro[3H-indole-3,3'-piperidine]-2,6'(1H)-dione

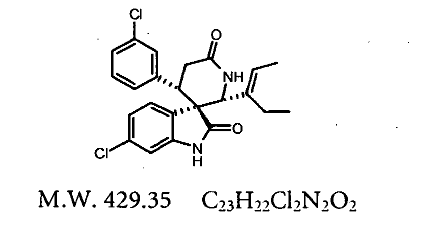

In a manner similar to the method described in example 15c, E/Z-6-chloro-3-(3-chlorobenzylidene)-2-oxo-2,3-dihydro-indole-1-carboxylic acid *tert*-butyl ester prepared in example 15a (0.4 g, 1.02 mmol) was reacted with 1-(1-ethyl-propenyl)-3-trimethylsilyoxy-2-aza-1,3-butadiene (2.1 g, 9.93 mmol) prepared in example 24a in toluene and then trifluoroacetic acid (20 mL) in dichloromethane to give racemic (2'R, 3R, 4'S)-6-chloro-4'-(3-chlorophenyl)-2'-(1-ethyl-propenyl) spiro[3H-indole-3,3'-piperidine]-2,6'(1H)-dione as a white solid (0.24 g, 54.5% ).
HRMS(ES⁺) *m*/*z* Calcd for C₂₃H₂₂Cl₂N2O₂ + H [(M+H)⁺]: 429.1131. Found: 429.1129.

### Example 24c

### Preparation of intermediate racemic (2'R, 3R, 4'S)-6-chloro-4'-(3-chlorophenyl)-2'-(1-ethyl-propyl) spiro[3H-indole-3,3'-piperidine]-2,6'(1H)-dione

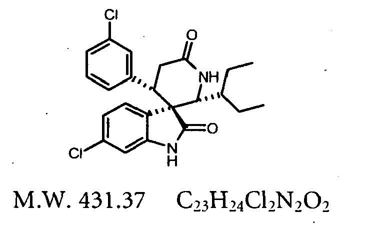

To a solution of racemic (2'R, 3R, 4'S)-6-chloro-4'-(3-chlorophenyl)-2'-(1-ethylpropenyl) spiro[3H-indole-3,3'-piperidine]-2,6'(1H)-dione (0.3 g, 0.70 mmol) prepared in example 24b in ethyl acetate (30 mL) was added platinum oxide (0.35 g, 1.54 mmol). The resulting suspension was vigorously shaken under hydrogen (50 psi) for 6 h. The mixture was filtered through a short pad of celite. The filtrate was concentrated. The residue was purified by chromatography (EtOAc:CH2Cl₂=1:1) to give racemic (2'R, 3R, 4'S)-6-chloro-4'-(3-chlorophenyl)-2'-(1-ethyl-propyl) spiro[3H-indole-3,3'-piperidine]-2,6'(1H)-dione as a white solid (Yield 0.11 g, 37.7%).
HRMS(ES⁺) *m*/*z* Calcd for C₂₃H₂₄Cl₂N₂O₂ +H [(M+H)⁺]: 431.1288 Found: 431.1285.

### Example 24d

### Preparation of racemic (2'R, 3R, 4'S)-6-chloro-4'-(3-chlorophenyl)-2'-(1-ethyl-propyl) spiro[3H-indole-3,3'-piperidine]-2(1H)-one

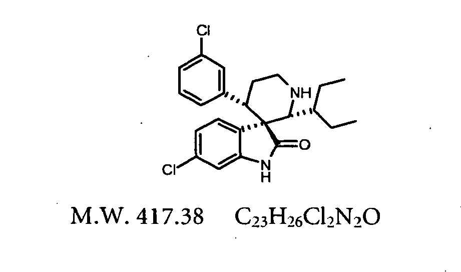

In a manner similar to the method described in example 1d, racemic (2'R, 3R, 4'S)-6-chloro-4'-(3-chlorophenyl)-2'-(1-ethyl-propyl) spiro[3H-indole-3,3'-piperidine]-2,6'(1H)-dione (73.6 mg, 0.17 mmol) prepared in example 24d was reacted with NaBH₄ (64.3 mg, 1.7 mmol) in methanol (3 mL) to give racemic (2'R, 3R, 4'S)-6-chloro-4'-(3-chlorophenyl)-2'-(1-ethyl-propyl) spiro[3H-indole-3,3'-piperidine]-2(1H)-one (Yield 10.5 mg, 14.7 %).
HRMS(ES⁺) *m*/*z* Calcd for C₂₃H₂₆Cl₂N₂O + H [(M+H)⁺]: 417.1495. Found: 417.1494.

### Example 25a

### Preparation of intermediate 1-(2,5-dimethyl-phenyl)-3-trimethylsilyoxy-2-aza-1,3-butadiene

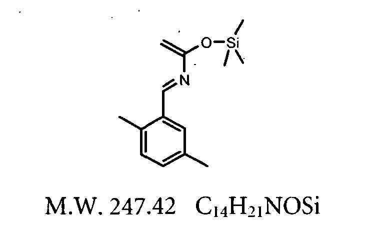

In a manner similar to the method described in example 1b, 2,5-dimethyl-benzaldehyde (1.34 g, 10 mmol) (Aldrich) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride(1.0 g, 13 mmol) to give 1-(2, 5-dimethyl-phenyl)-3-trimethylsilyoxy-2-aza-1,3-butadiene as yellow gum and used for the next step without further purification.

### Example 25b

### Preparation of intermediate racemic (2'R, 3R, 4'S)-6-chloro-4'-(3-chlorophenyl)-2'-(2, 5-dimethyl-phenyl) spiro[3H-indole-3,3'-piperidine]-2,6'(1H)-dione

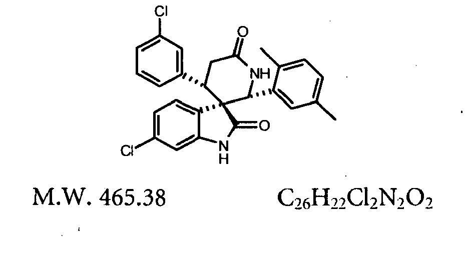

In a manner similar to the method described in example 15c, E/Z-6-chloro-3-(3-chlorobenzylidene)-2-oxo-2,3-dihydro-indole-1-carboxylic acid *tert*-butyl ester prepared in example 15a (0.4 g, 1.02 mmol) was reacted with 1-(2, 5-dimethyl-phenyl)-3-trimethylsilyoxy-2-aza-1,3-butadiene (2.3 g, 9.31 mmol) prepared in example 25a in toluene and then trifluoroacetic acid (20 mL) in dichloromethane to give racemic (2'R, 3R, 4'S)-6-chloro-4'-(3-chlorophenyl)-2'-(2, 5-dimethyl-phenyl) spiro[3H-indole-3,3'-piperidine]-2,6'(1H)-dione as a white solid.
HRMS(ES⁺) *m*/*z* Calcd for C₂₆H₂₂Cl₂N₂O₂ + H [(M+H)⁺]: 465.1131 Found: 465.1128.

### Example 25c

### Preparation of racemic (2'R, 3R, 4'S)-6-chloro-4'-(3-chloro-phenyl)-2'-(2,5-dimethylphenyl) spiro[3H-indole-3,3'-piperidine]-2(1H)-one

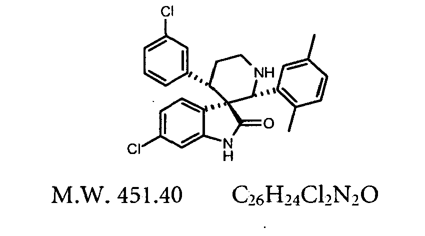

In a manner similar to the method described in example 1d, racemic (2'R, 3R, 4'S)-6-chloro-4'-(3-chlorophenyl)-2'-(2,5-dimethyl-phenyl) spiro[3H-indole-3,3'-piperidine]-2,6'(1H)-dione (42.1 mg, 0.09 mmol) prepared in example 25b was reacted with excess of NaBH₄ in methanol (3 mL) to give racemic (2'R, 3R, 4'S)-6-chloro-4'-(3-chloro-phenyl)-2'-(2,5-dimethyl-phenyl) spiro[3H-indole-3,3'-piperidine]-2(1H)-one as a white solid (Yield 13.7 mg, 33.8%).
HRMS(ES⁺) *m*/*z* Calcd for C₂₆H₂₄Cl₂N₂O + H [(M+H)⁺]: 451.1339. Found: 451.1338.

### Example 26a

### Preparation of intermediate 1-(2,5-dimethyl-2H-pyrazole-3-yl)-3-trimethylsilyoxy-2-aza-1,3-butadiene

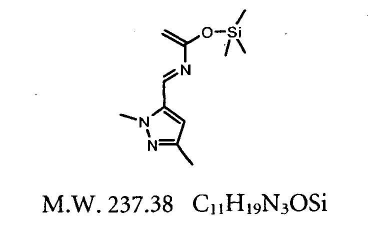

In a manner similar to the method described in example 1b, 2,5-dimethyl-2H-pyrazole-3-carbaldehyde (1.24 g, 10 mmol) (ASDI-INTER) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(2,5-dimethyl-2H-pyrazole-3-yl)-3-trimethylsilyoxy-2-aza-1,3-butadiene as yellow gum and used for the next step without further purification.

### Example 26b

### Preparation of intermediate racemic (2'R, 3R, 4'S)-6-chloro-4'-(3-chlorophenyl)-2'-(2,5-dimethyl-2H-pyrazole-3-yl) spiro[3H-indole-3,3'-piperidine]-2,6'(1H)-dione

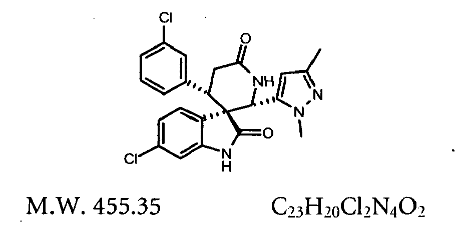

In a manner similar to the method described in example 15c, E/Z-6-chloro-3-(3-chlorobenzylidene)-2-oxo-2,3-dihydro-indole-1-carboxylic acid tert-butyl ester prepared in example 15a (0.4 g, 1.02 mmol) was reacted with 1-(2,5-dimethyl-2H-pyrazole-3-yl)-3-trimethylsilyoxy-2-aza-1,3-butadiene (2.5 g, 10.5 mmol) prepared in example 26a in toluene and then trifluoroacetic acid (15 mL) in dichloromethane to give racemic (2'R, 3R, 4'S)-6-chloro-4'-(3-chlorophenyl)-2'-(2,5-dimethyl-2H-pyrazole-3-yl) spiro[3H-indole-3,3'-piperidine]-2,6'(1H)-dione as a white solid (Yield 0.14 g, 30.4%).
HRMS(ES⁺) *m*/*z* Calcd for C₂₃H₂₀Cl₂N₄O₂ + H [(M+H)⁺]: 455.1036 Found: 455.1035.

### Example 26c

### Preparation of racemic (2'R, 3R, 4'S)-6-chloro-4'-(3-chlorophenyl)-2'-(2,5-dimethyl-2H-pyrazole-3-yl) spiro[3H-indole-3,3'-piperidine]-2(1H)-one

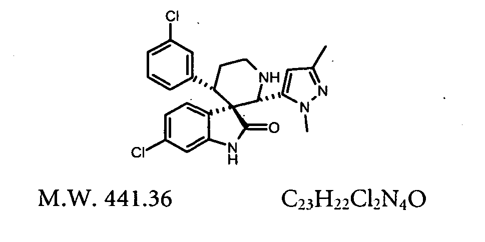

In a manner similar to the method described in example 1d, racemic (2'R, 3R, 4'S)-6-chloro-4'-(3-chlorophenyl)-2'-(2,5-dimethyl-2H-pyrazole-3-yl) spiro[3H-indole-3,3'-piperidine]-2,6'(1H)-dione (81.6 mg, 0.18 mmol) prepared in example 26b was reacted with excess of NaBH₄ in methanol (3 mL) to give racemic (2'R, 3R, 4'S)-6-chloro-4'-(3-chlorophenyl)-2'-(2,5-dimethyl-2H-pyrazole-3-yl) spiro[3H-indole-3,3'-piperidine]-2(1H)-one as a white amorphous (Yield 42.9 mg, 54.3 %).
HRMS(ES⁺) *m*/*z* Calcd for C₂₃H₂₂Cl₂N₄O + H [(M+H)⁺]: 441.1244. Found: 441.1244.

### Example 27

### Preparation of racemic (2'R, 3R, 4'S)-6-chloro-4'-(3-chlorophenyl)-1'-(morpholin-4-carbonyl)-2'-(1-naphthalenyl) spiro[3H-indole-3,3'-piperidine]-2(1H)-one

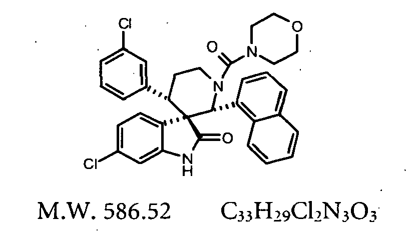

To a solution of racemic (2'R, 3R, 4'S)-6-chloro-4'-(3-chlorophenyl)-2'-(1-naphthalenyl) spiro[3H-indole-3,3'-piperidine]-2(1H)-one (30 mg, 0.062 mmol) prepared in example 17c in dichloromethane (2 mL) was added a solution of morpholine-4-carbonyl chloride (13.9 mg, 0.093 mmol) in dichloromethane and the excess of triethylamine. The reaction mixture was stirred at room temperature for 2 h and then heated to reflux for 2 h. The reaction mixture was cooled down and diluted with ethyl acetate. The organic layer was washed with water, brine and then concentrated. The residue was purified by chromatography (EtOAc:Hexane = 1:1) to give racemic (2'R, 3R, 4'S)-6-chloro-4'-(3-chlorophenyl)-1'-(morpholin-4-carbonyl)-2'-(1-naphthalenyl) spiro[3H-indole-3,3'-piperidine]-2(1H)-one, which was recrystallized from EtOAC-Hexane to afford the desired product as a white solid (Yield 20.5 mg, 56.9%).
MS(ES⁺) *m*/*z* Calcd for C₃₃H₂₉ClaN₃O₃ + H [(M+H)⁺]: 586.1659. Found: 586.1657.

### Example 28a

### Preparation of intermediate E/Z-6-chloro-3-(4-chloro-benzylidene)-1,3-dihydro-indol-2-one

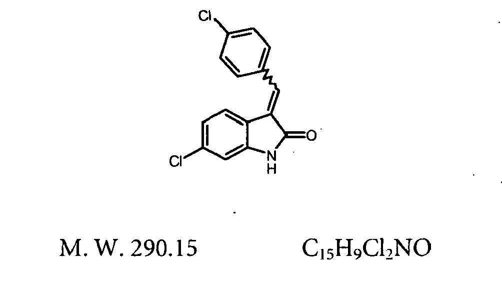

In a manner similar to the method described in example 1a, 6-chlorooxindole (2 g, 11.4 mmol) was reacted with 4-chloro-benzaldehyde (1.91 g, 13.6 mmol)(1.53 g, 13.6 mmol) (Aldrich) and piperidine (1.34 mL, 13.6 mmol) in methanol to give a mixture of E-and Z-6-chloro-3-(4-chloro-benzylidene)-1,3-dihydro-indol-2-one as a yellow solid (Yield: 3.3 g, 100%).

### Example 28b

### Preparation of intermediate E/Z 6-chloro-3-(4-chloro-benzylidene)-2-oxo-2,3-dihydro-indole-1-carboxylic acid ethyl ester

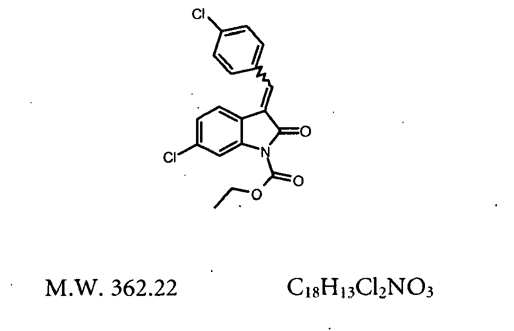

In a manner similar to the method described in example 4b, E/Z -6-chloro-3-(4-chlorobenzylidene)-1,3-dihydro-indol-2-one (3.3 g, 11.3 mmol) was reacted with ethyl chloroformate (1.62 mL, 17.0 mmol) and triethylamine (3.16 mL, 22.6 mmol) in dichloromethane to give E/Z-6-chloro-3-(4-chloro-benzylidene)-2-oxo-2,3-dihydro-indole-1-carboxylic acid ethyl ester as a yellow solid (Yield 3.0 g, 73%).

### Example 28c

### Preparation of intermediate racemic (2'S, 3S, 4'R)-6-chloro-4'-(4-chlorophenyl)-2'-(3-fluorophenyl)-2,3-dihydro-2,6'-dioxo spiro [indole-3,3'-piperidine]-1-carboxylic acid ethyl ester

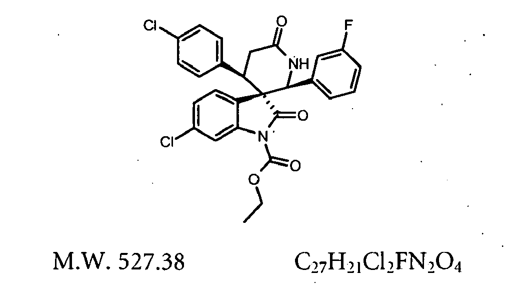

In a manner similar to the method described in example 4d, E/Z-6-chloro-3-(4-chlorobenzylidene)-2-oxo-2,3-dihydro-indole-1-carboxylic acid ethyl ester (0.4 g, 1.1 mmol) prepared in example 28b was reacted with 1-(3-fluorophenyl)-3-trimethylsilyoxy-2-aza-1,3-butadiene (2.6 g, 10.95 mmol) prepared in example 5a, in toluene to give racemic (2'S, 3S, 4'R)-6-chloro-4'-(4-chlorophenyl)-2'-(3-fluorophenyl)-2,3-dihydro-2,6'-dioxo spiro[indole-3,3'-piperidine]-1-carboxylic acid ethyl ester (Yield 0.45 g, 77.6%).

### Example 28d

### Preparation of intermediate racemic (2'S, 3S, 4'R)-6-chloro-4'-(4-chlorophenyl)-2'-(3-fluorophenyl) spiro[3H-indole-3,3'-piperidine]-2,6'(1H)-dione

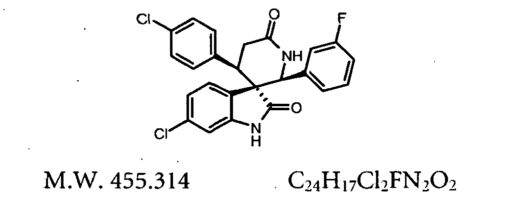

In a manner similar to the method described in example 4e, racemic (2'S, 3S, 4'R)-6-chloro-4'-(4-chlorophenyl)-2'-(3-fluorophenyl)-2,3-dihydro-2,6'-dioxo spiro[indole-3,3'-piperidine]-1-carboxylic acid ethyl ester (0.45 g, 0.85 mmol) prepared in example 28c was reacted with NaOH (69.6 mg, 1.71 mmol) in methanol to give racemic (2'S, 3S, 4'R)-6-chloro-4'-(4-chloro-phenyl)-2'-(3-fluoro-phenyl) spiro[3H-indole-3,3'-piperidine]-2,6'(1H)-dione as a white amorphous solid (Yield 0.15 g, 38.5%). HRMS(ES⁺) *m*/*z* Calcd for C₂₄H₁₇Cl₂FN₂O₂ + H [(M+H)⁺]: 455.0724 Found: 455.0724.

### Example 28e

### Preparation of racemic (2'S, 3S, 4'R)-6-chloro-4'-(4-chloro-phenyl)-2'-(3-fluoro-phenyl) spiro[3H-indole-3,3'-piperidine]-2(1H)-one

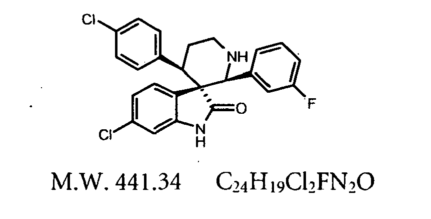

In a manner similar to the method described in example 6c, racemic (2'S, 3S, 4'R)-6-chloro-4'-(4-chloro-phenyl)-2'-(3-fluoro-phenyl) spiro[3H-indole-3,3'-piperidine]-2,6'(1H)-dione (150 mg, 0.33 mmol) prepared in example 28d was reacted with excess of NaBH₄ in methanol (3 mL) to give racemic (2'S, 3S, 4'R)-6-chloro-4'-(4-chloro-phenyl)-2'-(3-fluoro-phenyl) spiro[3H-indole-3,3'-piperidine]-2(1H)-one as a white solid (Yield 75.9 mg, 52.2 %).
HRMS(ES⁺) *m*/*z* Calcd for C₂₄H₁₉Cl₂FN₂O + H [(M+H)⁺]: 441.0931. Found: 441.0926.

### Example 29

### Preparation of racemic (2'S, 3S, 4'R)-1'-tert-butylaminocarbonyl-6-chloro-4'-(4-chlorophenyl)-2'-(3-fluoro-phenyl) spiro[3H-indole-3,3'-piperidine]-2(1H)-one

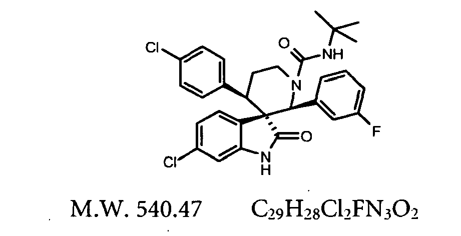

To a solution of racemic (2'S, 3S, 4'R)-6-chloro-4'-(4-chloro-phenyl)-2'-(3-fluoro-phenyl) spiro[3H-indole-3,3'-piperidine]-2(1H)-one (30 mg, 0.068 mmol) prepared in example 28e in dichloromethane (10 mL) was added 2-isocyanato-2-methyl-propane (0.2 mL) (Aldrich). After the reaction mixture was stirred at room temperature overnight, the solvent was removed to give racemic (2'S, 3S, 4'R)-1'-tert-butylaminocarbonyl-6-chloro-4'-(4-chloro-phenyl)-2'-(3-fluoro-phenyl) spiro[3H-indole-3,3'-piperidine]-2(1H)-one as a white powder (Yield 37.1 mg, 100 %). HRMS(ES⁺) *m*/*z* Calcd for C₂₉H₂₈Cl₂FN₃O₂ + H [(M+H)⁺]: 540.1616. Found: 540.1606.

### Example 30

### Preparation of racemic (2'S, 3S, 4'R)-6-chloro-4'-(4-chloro-phenyl)-1'-(3-cyano-phenylaminocarbonyl)-2'-(3-fluoro-phenyl) spiro[3H-indole-3,3'-piperidine]-2(1H)-one

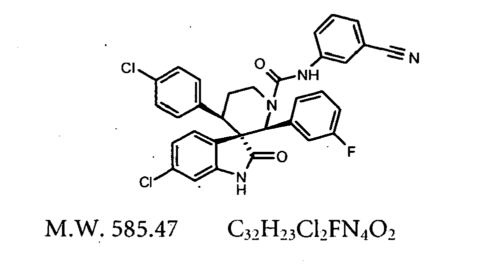

To a solution of racemic (2'S, 3S, 4'R)-6-chloro-4'-(4-chloro-phenyl)-2'-(3-fluoro-phenyl) spiro[3H-indole-3,3'-piperidine]-2(1H)-one (30 mg, 0.068 mmol) prepared in example 28e in dichloromethane (10 mL) was added 3-isocyanato-benzonitrile (0.2 mL) (Aldrich). The reaction mixture was stirred at room temperature overnight. The reaction mixture was diluted with dichloromethane and washed with water. The organic layer was separated and concentrated. The residue was suspended in dichloromethane (3 mL) and filtered to give a white powder. The filtrate was then purified with chromatography to give racemic (2'S, 3S, 4'R)-6-chloro-4'-(4-chloro-phenyl)-1'-(3-cyano-phenylaminocarbonyl)-2'-(3-fluoro-phenyl) spiro[3H-indole-3,3'-piperidine]-2(1H)-one as a white powder (Yield 11.9 mg, 29.9 %).
HRMS(ES⁺) *m*/*z* Calcd for C₃₂H₂₃Cl₂hFN₄O₂ + H [(M+H)⁺]: 585.1255. Found: 585.1246.

### Example 31

### Preparation of racemic (2'R, 3R, 4'S)-6-chloro-4'-(3-chloro-phenyl)-1'-(3-cyano-phenylaminocarbonyl)-2'-(3-fluoro-phenyl) spiro[3H-indole-3,3'-piperidine]-2(1H)-one

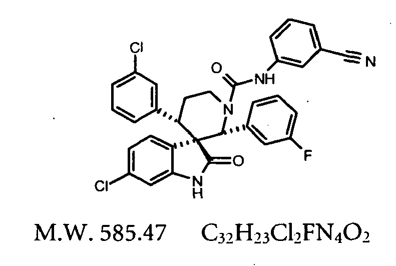

To a solution of racemic (2'R, 3R, 4'S)-6-chloro-4'-(3-chloro-phenyl)-2'-(3-fluorophenyl) spiro[3H-indole-3,3'-piperidine]-2(1H)-one (30 mg, 0.068 mmol) prepared in example 5d in dichloromethane (10 mL) was added 3-isocyanato-benzonitrile (12.7 mg, 0.088 mmol) (Aldrich) and ethyl-diisopropyl amine (0.1 mL). The reaction mixture was stirred at room temperature overnight. The reaction mixture was diluted with dichloromethane and washed with water. The organic layer was separated and concentrated. The residue was suspended in dichloromethane (3 mL) and filtered to give a white powder. The filtrate was then purified with chromatography to give racemic (2'R, 3R, 4'S)-6-chloro-4'-(3-chloro-phenyl)-1'-(3-cyano-phenylaminocarbonyl)-2'-(3-fluoro-phenyl) spiro[3H-indole-3,3'-piperidine]-2(1H)-one as a white powder (Yield 16.8 mg, 42.2 %).
HRMS(ES⁺) *m*/*z* Calcd for C₃₂H₂₃Cl₂FN₄O₂ + H [(M+H)⁺]: 585.1255. Found: 585.1255.

### Example 32

### Preparation of racemic (2'R, 3R, 4'S)-1'-tert-butylaminocarbonyl-6-chloro-4'-(3-chlorophenyl)-2'-(3-fluoro-phenyl) spiro[3H-indole-3,3'-piperidine]-2(1H)-one

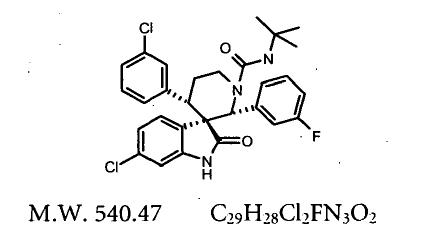

In a manner similar to the method described in example 29, racemic (2'R, 3R, 4'S)-6-chloro-4'-(3-chloro-phenyl)-2'-(3-fluoro-phenyl) spiro[3H-indole-3,3'-piperidine]-2,6'(1H)-dione (30 mg, 0.068 mmol) prepared in example 5d was reacted with 2-isocyanato-2-methyl-propane (8.7 mg, 0.088 mmol) (Aldrich) in dichloromethane to give racemic (2'R, 3R, 4'S)-1'-tert-butylaminocarbonyl-6-chloro-4'-(3-chloro-phenyl)-2'-(3-fluoro-phenyl) spiro[3H-indole-3,3'-piperidine]-2(1H)-one as a white solid (Yield 37.1 mg, 100 %).
HRMS(ES⁺) *m*/*z* Calcd for C₂₉H₂₈Cl₂FN₃O₂ + H [(M+H)⁺]: 540.1616. Found: 540.1608.

### Example 33

### Preparation of racemic (2'R, 3R, 4'S)-1'-benzoyl-6-chloro-4'-(3-chloro-phenyl)-2'-(3-fluoro-phenyl) spiro[3H-indo)e-3,3'-pipendine]-2(H)-once

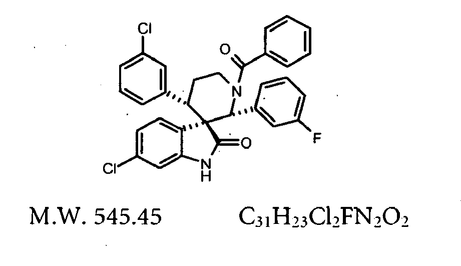

To a solution of racemic (2'R, 3R, 4'S)-6-chloro-4'-(3-chloro-phenyl)-2'-(3-fluoro-phenyl) spiro[3H-indole-3,3'-piperidine]-2(1H)-one (30 mg, 0.068 mmol) prepared in example 5d and the excess of ethyl-diisopropyl amine in dichloromethane (2.5mL) was added 1 drop of benzoyl chloride (Aldrich). The reaction mixture was stirred at room temperature for overnight. Then two more drops of benzoyl chloride were added and the reaction mixture was stirred at room temperature for 2 h. The solvent was removed and the residue was purified with chromatography (EtOAC:Hexane = 1:1) to give racemic (2'R, 3R, 4'S)-1'-benzoyl-6-chloro-4'-(3-chloro-phenyl)-2'-(3-fluoro-phenyl) spiro[3H-indole-3,3'-piperidine]-2(H)-one as a white amorphous (Yield 26.2 mg, 70.6 %).
HRMS(ES⁺) *m*/*z* Calcd for C₃₁H₂₃Cl₂FN₂O₂ + H [(M+H)⁺]: 545.1194. Found: 545.1182.

### Example 34

### Preparation of racemic (2'R, 3R, 4'S)-1'-acetyl-6-chloro-4'-(3-chloro-phenyl)-2'-(3-fluoro-phenyl) spiro[3H-indole-3,3'-piperidine]-2(1H)-one

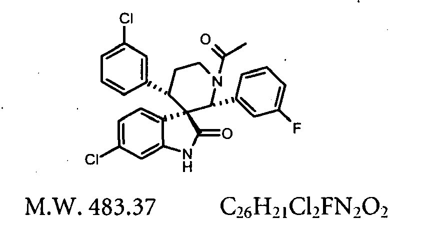

To a solution of racemic (2'R, 3R, 4'S)-6-chloro-4'-(3-chloro-phenyl)-2'-(3-fluoro-phenyl) spiro[3H-indole-3,3'-piperidine]-2(1H)-one (30 mg, 0.068 mmol) prepared in example 5d and the excess of ethyl-diisopropyl-amine in dichloromethane (2.5 mL) was added 1 drop of acetyl chloride. The reaction mixture was stirred at room temperature for overnight. Then two more drops of acetyl chloride were added and the reaction mixture was stirred at room temperature for 2 h. The solvent was removed and the residue was purified with chromatography (EtOAC:Hexane = 1:1) to give racemic (2'R, 3R, 4'S)-1'-acetyl-6-chloro-4'-(3-chloro-phenyl)-2'-(3-fluoro-phenyl) spiro[3H-indole-3,3'-piperidine]-2(1H)-one as a white amorphous (Yield 8.2 mg, 25.0 %). HRMS(ES⁺) *m*/*z* Calcd for C₂₆H₂₁Cl₂FN₂O₂ + H [(M+H)⁺]: 483.1037. Found: 483.1041.

### Example 35

### Preparation of racemic (2'R, 3R, 4'S)-1'-[4-(amonocarbonylmethyl)-piperazine-1-carbonyl]-6-chloro-4'-(3-chloro-phenyl)-2'-(3-fluoro-phenyl) spiro[3H-indole-3,3'-piperidine]-2(1H)-one

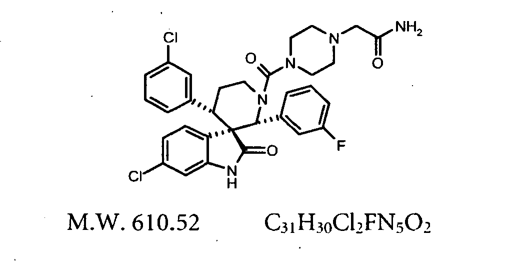

To a solution of racemic (2'R, 3R, 4'S)-6-chloro-4'-(3-chloro-phenyl)-2'-(3-fluoro-phenyl) spiro[3H-indole-3,3'-piperidine]-2(1H)-one (30 mg, 0.068 mmol) prepared in example 5d and ethyl-diisopropyl-amine (0.05 mL) in dichloromethane (2.5 mL) at 0 °C was added 10 drops phosgene (20% in toluene). The reaction mixture was stirred at 0 °C for 3 h. The reaction mixture was then warmed up to room temperature and 2-piperazine-1-yl-acetamide (27.9 mg, 0.10 mmol) (Matrix Scientific) was added. After stirring at room temperature for 2 h, the reaction mixture was concentrated. The residue was purified with reversed phase column to give racemic (2'R, 3R, 4'S)-1'-[4-(amonocarbonylmethyl)-piperazine-1-carbonyl]-6-chloro-4'-(3-chloro-phenyl)-2'-(3-fluoro-phenyl) spiro[3H-indole-3,3'-piperidine]-2(1H)-one as a white trifluoroacetic acid salt (Yield 45.8 mg, 93.1 %).
HRMS(ES⁺) *m*/*z* Calcd for C₃₁H₃₀Cl₂FN₅O₂ + H [(M+H)⁺]: 610.1783. Found: 610.1787.

### Example 36

### Preparation of racemic (2'R, 3R, 4'S)-6-chloro-4'-(3-chloro-phenyl)-2'-(3-fluoro-phenyl)-1'-[4-(3-methanesulfonyl-propyl)-piperazine-1-carbonyl] spiro[3H-indole-3,3'-piperidine]-2(1H)-one

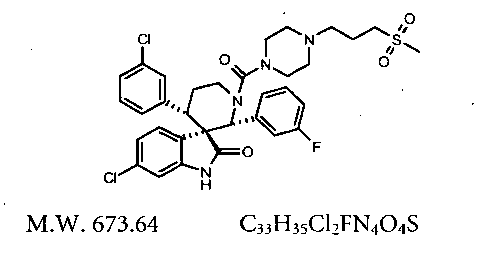

In a manner similar to the method described in example 35, racemic (2'R, 3R, 4'S)-6-chloro-4'-(3-chloro-phenyl)-2'-(3-fluoro-phenyl) spiro[3H-indole-3,3'-piperidine]-2(1H)-one (30 mg, 0.068 mmol) prepared in example 5d was reacted with 10 drops of phosgene (20% in toluene), ethyl-diisopropyl-amine (0.05 mL) and 1-(3-methanesulfonyl-propyl)-piperazine (27.9 mg, 0.1 mmol) in dichloromethane to give racemic (2'R, 3R, 4'S)-6-chloro-4'-(3-chloro-phenyl)-2'-(3-fluoro-phenyl)-1'-[4-(3-methanesulfonyl-propyl)-piperazine-1-carbonyl] spiro[3H-indole-3,3'-piperidine]-2(1H)-one as a white amorphous trifluoroacetic acid salt (Yield: 22.3 mg, 41.8%).
HRMS(ES⁺) *m*/*z* Calcd for C₃₃H₃₅Cl₂FN₄O₄S + H [(M+H)⁺]: 673.1813 Found: 673.1813.

### Example 37

### Preparation of racemic (2'R, 3R, 4'S)-6-chloro-4'-(3-chloro-phenyl)-2'-(3-fluoro-phenyl)-1'-[4-(2-oxo-2-pyrrolidin-1-yl-ethyl)-piperazine-1-carbonyl] spiro[3H-indole-3,3'-piperidine]-2(1H)-one

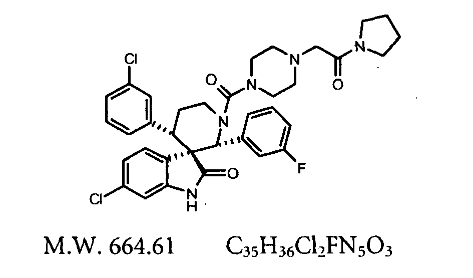

In a manner similar to the method described in example 35, racemic (2'R, 3R, 4'S)-6-chloro-4'-(3-chloro-phenyl)-2'-(3-fluoro-phenyl) spiro[3H-indole-3,3'-piperidine]-2(1H)-one (30 mg, 0.068 mmol) prepared in example 5d was reacted with 10 drops of phosgene (20% in toluene), ethyl-diisopropyl-amine (0.05 mL) and 2-piperazin-1-yl-1-pyrrolidin-1-yl-ethanone (19.7 mg, 0.1 mmol) (Aldrich) in dichloromethane to give racemic (2'R, 3R, 4'S)-6-chloro-4'-(3-chloro-phenyl)-2'-(3-fluoro-phenyl)-1'-[4-(2-oxo-2-pyrrolidin-1-yl-ethyl)-piperazine-1-carbonyl] spiro[3H-indole-3,3'-piperidine]-2(1H)-one as a white amorphous trifluoroacetic acid salt (Yield: 21.8 mg, 39.4%). HRMS(ES⁺) *m*/*z* Calcd for C₃₅H₃₆Cl₂FN₅O₃ + H [(M+H)⁺]: 664.2252 Found: 664.2249.

### Example 38

### Preparation of racemic (2'R, 3R, 4'S)-1'-[4-(2-acetylamino-ethyl)-piperazine-1-carbonyl]-6-chloro-4'-(3-chloro-phenyl)-2'-(3-fluoro-phenyl) spiro[3H-indole-3,3'-piperidine]-2(1H)-one

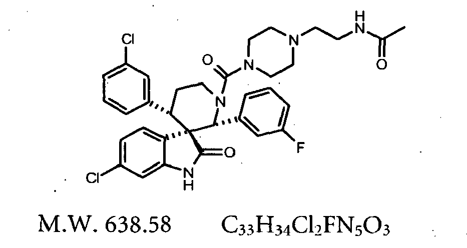

In a manner similar to the method described in example 35, racemic (2'R, 3R, 4'S)-6-chloro-4'-(3-chloro-phenyl)-2'-(3-fluoro-phenyl) spiro[3H-indole-3,3'-piperidine]-2(1H)-one (30 mg, 0.068 mmol) prepared in example 5d was reacted with 10 drops of phosgene (20% in toluene), ethyl-diisopropyl-amine (0.05 mL) and N-(2-piperazin-1-yl-ethyl)-acetamide (24.9 mg, 0.1 mmol) in dichloromethane to give racemic (2'R, 3R, 4'S)-1'-[4-(2-acetylamino-ethyl)-piperazine-1-carbonyl]-6-chloro-4'-(3-chloro-phenyl)-2'-(3-fluoro-phenyl) spiro[3H-indole-3,3'-piperidine]-2(1H)-one as a white amorphous trifluoroacetic acid salt (Yield: 23.3 mg, 46.1%).
HRMS(ES⁺) *m*/*z* Calcd for C₃₃H₃₄Cl₂FN₅O₃ + H [(M+H)⁺]: 638.2096 Found: 638.2092.

### Example 39

### Preparation of racemic (2'R, 3R, 4'S)-6-chloro-4'-(3-chloro-phenyl)-2'-(3-fluoro-phenyl)-1'-[4-(2-hydroxy-ethyl)-piperazine-1-carbonyl] spiro[3H-indole-3,3'-piperidine]-2(1H)-one

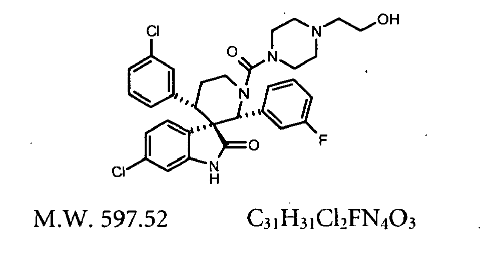

In a manner similar to the method described in example 35, racemic (2'R, 3R, 4'S)-6-chloro-4'-(3-chloro-phenyl)-2'-(3-fluoro-phenyl) spiro[3H-indole-3,3'-piperidine]-2(1H)-one (30 mg, 0.068 mmol) prepared in example 5d was reacted with 10 drops of phosgene (20% in toluene), ethyl-diisopropyl-amine (0.05 mL) and 2-piperazin-1-yl-ethanol (13.0 mg, 0.1 mmol) in dichloromethane to give racemic (2'R, 3R, 4'S)-6-chloro-4'-(3-chloro-phenyl)-2'-(3-fluoro-phenyl)-1'-[4-(2-hydroxy-ethyl)-piperazine-1-carbonyl] spiro[3H-indole-3,3'-piperidine]-2(1H)-one as a white amorphous trifluoroacetic acid salt (Yield: 25.2 mg, 52.2%).
HRMS(ES⁺) *m*/*z* Calcd for C₃₁H₃₁Cl₂FN₄O₃ + H [(M+H)⁺]: 597.1830 Found: 597.1827.

### Example 40

### Preparation of racemic (2'R, 3R, 4'S)-6-chloro-4'-(3-chloro-phenyl)-1'-cyclohexylaminocarbonyl-2'-(3-fluoro-phenyl) spiro[3H-indole-3,3'-piperidine]-2(1H)-one

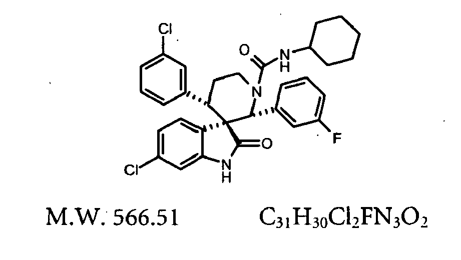

To a solution of racemic (2'R, 3R, 4'S)-6-chloro-4'-(3-chloro-phenyl)-2'-(3-fluoro-phenyl) spiro[3H-indole-3,3'-piperidine]-2(1H)-one (30 mg, 0.068 mmol) prepared in example 5d in dichloromethane (3 mL) was added isocyanato-cycloehexane (11.0 mg, 0.088 mmol) (Aldrich). The reaction mixture was stirred at room temperature for 3 h. The solvent was removed and the residue was crystallized from ethyl acetate/hexane to give racemic (2'R, 3R, 4'S)-6-chloro-4'-(3-chloro-phenyl)-1'-cyclohexylaminocarbonyl-2'-(3-fluoro-phenyl) spiro[3H-indole-3,3'-piperidine]-2(1H)-one as a white amorphous solid (Yield 22.7 mg, 57.7 %).
HRMS(ES⁺) *m*/*z* Calcd for C₃₁H₃₀Cl₂FN₃O₂ + H [(M+H)⁺]: 566.1112. Found: 566.1773. '

### Example 41

### Preparation of racemic (2'R, 3R, 4'S)-1'-benzylaminocarbonyl-6-chloro-4'-(3-chlorophenyl)-2'-(3-fluoro-phenyl) spiro[3H-indole-3,3'-piperidine]-2(1H)-one

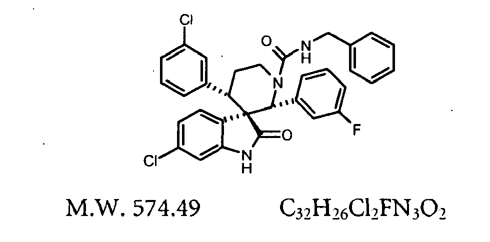

In a manner similar to the method described in example 40, racemic (2'R, 3R, 4'S)-6-chloro-4'-(3-chloro-phenyl)-2'-(3-fluoro-phenyl) spiro[3H-indole-3,3'-piperidine]-2(1H)-one (25 mg, 0.057 mmol) prepared in example 5d was reacted with isocyanatomethyl-benzene (11.7 mg, 0.088 mmol) in dichloromethane to give racemic (2'R, 3R, 4'S)-1'-benzylaminocarbonyl-6-chloro-4'-(3-chloro-phenyl)-2'-(3-fluoro-phenyl) spiro[3H-indole-3,3'-piperidine]-2(1H)-one (Yield: 11.5 mg, 35.3%).
HRMS(ES⁺) *m*/*z* Calcd for C₃₂H₂₆Cl₂FN₃O₂ + H [(M+H)⁺]: 574.1459 Found: 574.1452.

### Example 42a

### Preparation of intermediate 1,2-difluoro-4-isopropoxy-benzene

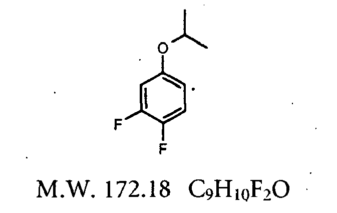

To a solution of 3,4-difluoro-phenol (5 g, 38.4 mmol) in acetone (50 mL) was added potassium carbonate (54 g, 38.4 mmol) and 2-iodo-propane. The reaction mixture was heated at refluxing for 24 h. The crude was cooled down and filtered through a short pad of celite. The filtrate was concentrated and the residue was purified by chromatography (EtOAc:Hexanes=1:9) to give 1,2-difluoro-4-isopropoxy-benzene as colorless oil (Yield 6.12 g, 92.3%).

### Example 42b

### Preparation of intermediate 2,3-difluoro-6-isopropoxy-benzaldehyde

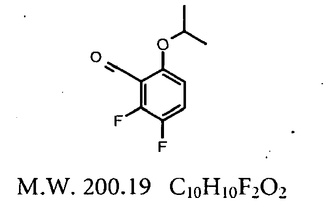

In a manner similar to the method described in example 22a, 1,2-difluoro-4-isopropoxybenzene (5.77 g, 33.5 mmol) prepared in example 42a was reacted with lithium diisopropyl amine (20.5 mL, 1:8 M in THF, 36.9 mmol), N,N-dimethyl-formamide (3.11 mL, 40.2 mmol) and quenched with acetic acid (8.0 g, 134 mmol) in tetrahydrofuran to give 2,3-difluoro-6-isopropoxy-benzaldehyde as a white crystal (Yield: 6.02 g, 89.9%).

### Example 42c

### Preparation of intermediate 1-(2,3-difluoro-6-isopropoxy-phenyl)-3-trimethylsilyoxy-2-aza-1,3-butadiene

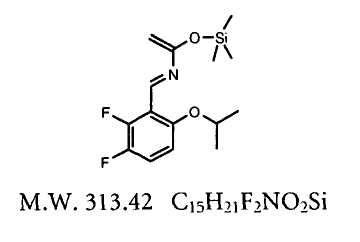

In a manner similar to the method described in example 1b, 2,3-difluoro-6-isopropoxybenzaldehyde (2.0 g, 10 mmol) prepared in example 42b was used as the starting material in place of 3-chloro-benzaldehyde to react with 1,1,1,3,3,3-hexamethyldisilazane (1.6 g, 10 mmol), n-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(2,3-difluoro-6-isopropoxy-phenyl)-3-trimethylsilyoxy-2-aza-1,3-butadiene as yellow oil and used for the next step without further purification.

### Example 42d

### Preparation of intermediate racemic (2'R, 3R, 4'S)-6-chloro-4'-(3-chlorophenyl)-2'-(2,3-difluoro-6-isopropoxy-phenyl) spiro[3H-indole-3,3'-piperidine]-2,6'(1H)-dione

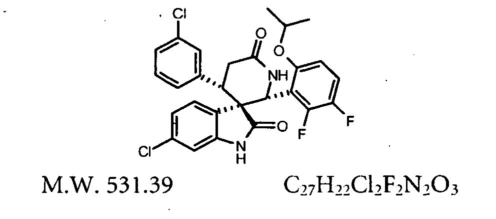

In a manner similar to the method described in example 15c, E/Z-6-chloro-3-(3-chlorobenzylidene)-2-oxo-2,3-dihydro-indole-1-carboxylic acid tert-butyl ester prepared in example 15a (0.4 g, 1.02 mmol) was reacted with 1-(2,3-difluoro-6-isopropoxy-phenyl)-3-trimethylsilyoxy-2-aza-1,3-butadiene (2.5 g, 7.98 mmol) prepared in example 42c in toluene and then trifluoroacetic acid (10 mL) in dichloromethane to give racemic (2'R, 3R, 4'S)-6-chloro-4'-(3-chlorophenyl)-2'-(2,3-difluoro-6-isopropoxy-phenyl) spiro[3H-indole-3,3'-piperidine]-2,6'(1H)-dione as a white solid (0.41 g, 75.9%).
HRMS(ES⁺) *m*/*z* Calcd for C₂₇H₂₂Cl₂F₂N₂O₃+H [(M+H)⁺]: 531.1049. Found: 531.1049.

### Example 42e

### Preparation of intermediate racemic (2'R, 3R, 4'S)-6-chloro-4'-(3-chlorophenyl)-2'-(2,3-difluoro-6-isopropoxy-phenyl)-6'-thioxo spiro[3H-indole-3,3'-piperidine]-2(1H)-one

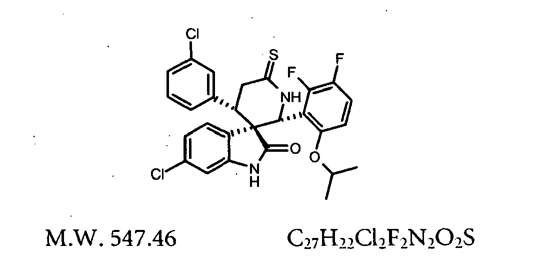

In a manner similar to the method described in example 37, racemic (2'R, 3R, 4'S)-6-chloro-4'-(3-chlorophenyl)-2'-(2,3-difluoro-6-isopropoxy-phenyl) spiro[3H-indole-3,3'-piperidine]-2,6'(1H)-dione (0.45 g, 0.85 mmol) prepared in example 42d was reacted with 2,4-bis-(4-methoxyphenyl)-1,3-dithia-2,4-diphosphetane 2,4-disulfide (0.6 g, 1.8 mmol) in toluene to give racemic (2'R, 3R, 4'S)-6-chloro-4'-(3-chlorophenyl)-2'-(2,3-difluoro-6-isopropoxy-phenyl)-6'-thioxo spiro[3H-indole-3,3'-piperidine]-2(1H)-one (Yield 0.36 g, 78.3%).
HRMS(ES⁺) *m*/*z* Calcd for C₂₇H₂₂Cl₂F₂N₂O₂S +H [(M+H)⁺]: 547.0820. Found: 547.0821.

### Example 42f

### Preparation of intermediate racemic (2'R, 3R, 4'S)-[6-chloro-4'-(3-chlorophenyl)-2'-(2,3-difluoro-6-isopropoxy-phenyl)-2,3-dihydro-2-oxospiro[3H-indole-3,3'-piperidin]-6-ylene] hydrazine carboxylic acid ethyl ester

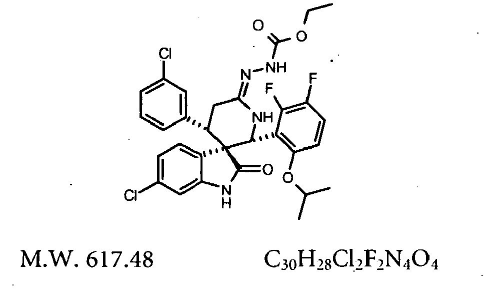

In a manner similar to the method described in example 20b, racemic (2'R, 3R, 4'S)-6-chloro-4'-(3-chlorophenyl)-2'-(2,3-difluoro-6-isopropoxy-phenyl)-6'-thioxo spiro[3H-indole-3,3'-piperidine]-2(1H)-one (0.30 g, 0.55 mmol) prepared in example 42d was reacted with ethyl carbazate (0.3 g, 2.97 mmol), mercuric acetate (0.30 g, 0.95 mmol) and triethylamine (0.1 g, 0.99 mmol) in tetrahydrofuran (40 mL) to give racemic (2'R, 3R, 4'S)- [6-chloro-4'-(3-chlorophenyl)-2'-(2,3-difluoro-6-isopropoxy-phenyl)-2,3-dihydro-2-oxospiro[3H-indole-3,3'-piperidin]-6-ylene] hydrazine carboxylic acid ethyl ester as a white solid (Yield 0.25 g; 73.5 %).
HRMS(ES⁺) *m*/*z* Calcd for C₃₀H₂₈Cl₂F₂N₄O₄ + H [(M+H)⁺]: 617.1529. Found: 617.1523.

### Example 42g

### Preparation of racemic (5'R, 3R, 7'S)-6-chloro-7'-(3-chlorophenyl)-5'-(2,3-difluoro-6-isopropoxy-phenyl)-5',6',7',8'-tetrahydro-3'-hydroxyspiro[3H-indole-3,6'-(1,2,4-triazolo[4,3-a]pyridine)]-2(1H)-one

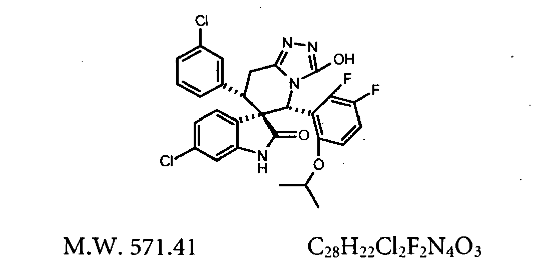

In a manner similar to the method described in example 20c, racemic (2'R, 3R, 4'S)-[6-chloro-4'-(3-chlorophenyl)-2'-(2,3-difluoro-6-isopropoxy-phenyl)-2,3-dihydro-2-oxospiro[3H-indole-3,3'-piperidin]-6-ylene] hydrazine carboxylic acid ethyl ester (0.21 g, 0.34 mmol) prepared in example 42f was heated in toluene (20 mL) to give racemic (5'R, 3R, 7'S)-6-chloro-7'-(3-chlorophenyl)-5'-(2,3-difluoro-6-isopropoxy-phenyl)-5',6',7',8'-tetrahydro-3'-hydroxyspiro[3H-indole-3,6'-(1,2,4-triazolo[4,3-a]pyridine)]-2(1H)-one as a white solid (Yield 0.13 g, 68.4 %).
HRMS(ES⁺) *m*/*z* Calcd for C₂₈H₂₂Cl₂F₂N₄O₃ + H [(M+H)⁺]: 571.1110. Found: 571.1107.

### Example 42h

### Preparation of chiral (5'R, 3R, 7'S)-6-chloro-7'-(3-chlorophenyl)-5'-(2,3-difluoro-6-isopropoxy-phenyl)-5',6',7',8'-tetrahydro-3'-hydroxyspiro[3H-indole-3,6'-(1,2,4-triazolo[4,3-a]pyridine)]-2(1H)-one

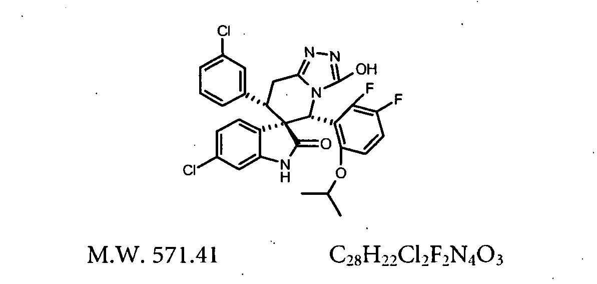

Separation of the two enantiomers from racemic racemic (5'R, 3R, 7'S)-6-chloro-7'-(3-chlorophenyl)-5'-(2,3-difluoro-6-isopropoxy-phenyl)-5',6',7',8'-tetrahydro-3'-hydroxyspiro[3H-indole-3,6'-(1,2,4-triazolo[4,3-a]pyridine)]-2(1H)-one (30 mg) prepared in example 42g was conducted by chiral SFC to provide chiral (5'R, 3R, 7'S)-6-chloro-7'-(3-chlorophenyl)-5'-(2,3-difluoro-6-isopropoxy-phenyl)-5',6',7',8'-tetrahydro-3'-hydroxyspiro[3H-indole-3,6'-(1,2,4-triazolo[4,3-a]pyridine)]-2(1H)-one as a white solid (12 mg, 40%) (RO5167429-000) and chiral (5'S, 3S, 7'R)-6-chloro-7'-(3-chlorophenyl)-5'-(2,3-difluoro-6-isopropoxy-phenyl)-5',6',7',8'-tetrahydro-3'-hydroxyspiro[3H-indole-3,6'-(1,2,4-triazolo[4,3-a]pyridine)]-2(1H)-one as a white solid (5 mg, 17%) (RO5167428-000).

### Example 43a

### Preparation of intermediate E/Z-6-chloro-3-(3-chloro-benzylidene)-1-(2-trimethylsilanyl-ethoxymethyl)-1,3-dihydro-indole-2-one

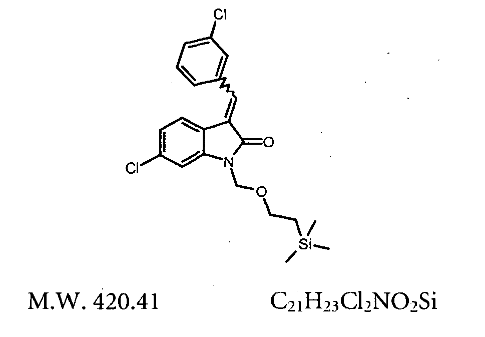

To a solution of E/Z-6-chloro-3-(3-chloro-benzylidene)-1,3-dihydro-indol-2-one prepared in example 4a (2.3 g, 7.9 mmol) in N,N-dimethyl-formamide (20 mL) at 0 °C was added NaH (60% in mineral oil) (0.32 g, 7.9 mmol) (Aldrich), followed by the dropwise addition of 2-(trimethylsilyl)ethoxymethyl chloride (1.32 g, 7.9 mmol) in tetrahydrofuran (20 mL). The reaction mixture was stirred at 0 °C for 0.5 h, then poured into ice-water. The crude was extracted with ethyl acetate twice. The combined organic layer was dried over Na₂SO₄. The solvent was removed and the residue was purified by chromatography (EtOAc:hexanes=1:5) to give E/Z-6-chloro-3-(3-chloro-benzylidene)-1-(2-trimethylsilanyl-ethoxymethyl)-1,3-dihydro-indole-2-one as yellow oil (Yield 3.0 g, 90%).

### Example 43b

### Preparation of intermediate racemic (2'R, 3R, 4'S)-6-chloro-4'-(3-chloro-phenyl)-2'-(3-fluoro-phenyl)-2,3-dihydro-2,6'-dioxo spiro[indole-3,3'-piperidine]-1-methoxyethyl trimethylsilane

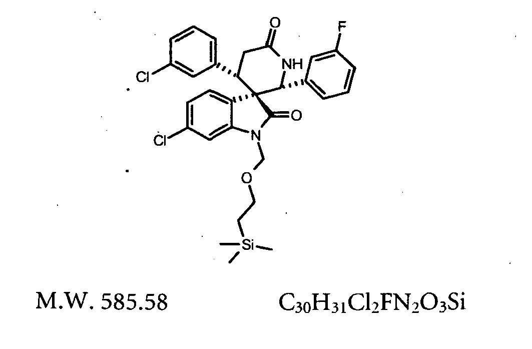

In a manner similar to the method described in example 4d, E/Z-6-chloro-3-(3-chlorobenzylidene)-1₋(2-trimethylsilanyl-ethoxymethyl)-1,3-dihydro-indole-2-one (1.0 g, 2.38 mmol) prepared in example 43a was reacted with 1-(3-fluoro-phenyl)-3-trimethylsilyoxy-2-aza-1,3-butadiene prepared in example 15a in toluene (60 mL) to give racemic (2'R, 3R, 4'S)-6-chloro-4'-(3-chloro-phenyl)-2'-(3-fluoro-phenyl)-2,3-dihydro-2,6'-dioxo spiro[indole-3,3'-piperidine]-1-methoxyethyl trimethylsilane as an oil (Yield 1.06 g, 75%).

### Example 43c

### Preparation of intermediate racemic (2'R, 3R, 4'S)-6-chloro-4'-(3-chloro-phenyl)-2'-(3-fluoro-phenyl) spiro[3H-indole-3,3'-piperidine]-1-methoxyethyl trimethylsilane

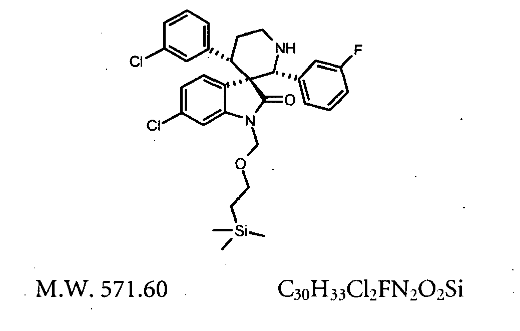

In a manner similar to the method described in example 1d, racemic (2'R, 3R, 4'S)-6-chloro-4'-(3-chloro-phenyl)-2'-(3-fluoro-phenyl)-2,3-dihydro-2,6'-dioxo spiro[indole-3,3'-piperidine]-1-methoxyethyl trimethylsilane (0.50 g, 0.85 mmol) prepared in example 43b was reacted with the excess of NaBH₄ in methanol (40 mL) to give racemic (2'R, 3R, 4'S)-6-chloro-4'-(3-chloro-phenyl)-2'-(3-fluoro-phenyl) spiro[3H-indole-3,3'-piperidine]-1-methoxyethyl trimethylsilane (Yield 78.0 mg, 16.0 %).

### Example 43d

### Preparation of intermediate racemic (2'R, 3R, 4'S)-1'-[(tert-butoxycarbonyl) methyl]-6-chloro-4'-(3-chlorophenyl)-2'-(3-fluoro-phenyl) spiro[indole-3,3'-piperidine]-1-methoxyethyl trimethylsilane

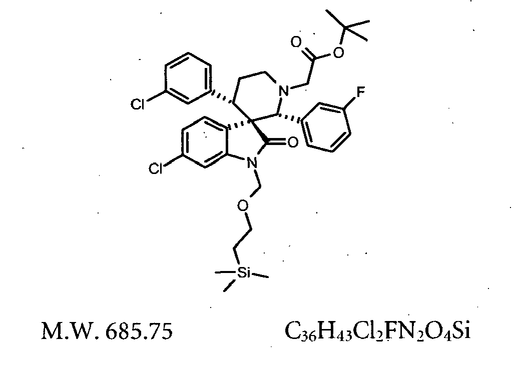

To a solution of racemic (2'R, 3R, 4'S)-6-chloro-4'-(3-chloro-phenyl)-2'-(3-fluoro-phenyl) spiro[3H-indole-3,3'-piperidine]-1-methoxyethyl trimethylsilane (72 mg, 0.126 mmol) prepared in example 43c in N,N-dimethyl-formamide (10 mL) at room temperature was added bromo-acetic acid tert-butyl ester (0.08 g, 0.41 mmol) and cesium carbonate (0.3 g, 0.92 mmol). The reaction mixture was stirred under nitrogen for 2 h, then was poured into saturated aqueous NH₄Cl solution. The mixture was extracted with ethyl acetate. The organic layers were combined, dried over Na₂SO₄ and concentrated. The residue was purified by chromatography to give racemic (2'R, 3R, 4'S)-1'-[(tert-butoxycarbonyl) methyl]-6-chloro-4'-(3-chlorophenyl)-2'-(3-fluoro-phenyl) spiro[indole-3,3'-piperidine]-1-methoxyethyl trimethylsilane (Yield 64.0 mg, 74.1%).

### Example 43e

### Preparation of racemic (2'R, 3R, 4'S)-1'-hydroxycarbonylmethyl-6-chloro-4'-(3-chlorophenyl)-2'-(3-fluoro-phenyl) spiro[3H-indole-3,3'-piperidine]-2(1H)-one

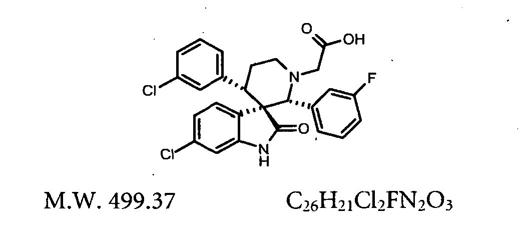

To a solution of racemic (2'R, 3R, 4'S)-1'-[(tert-butoxycarbonyl) methyl]-6-chloro-4'-(3-chlorophenyl)-2'-(3-fluoro-phenyl) spiro[indole-3,3'-piperidine]-1-methoxyethyl trimethylsilane (50 mg, 0.073 mmol) prepared in example 43d in dichloromethane (0.5 mL) was added trifluoroacetic acid (0.5 mL). The reaction mixture was stirred at room temperature for 2 h. The reaction mixture was diluted with ethyl acetate and washed with saturated sodium bicarbonate, water, brine and concentrated. The residue was dissolved in methanol (0.5 mL) and treated with N,N'-diisopropylethylamine (0.5 mL) and refluxed for 2 h. The reaction mixture was diluted with dichloromethane and washed with 4N of NaOH. The aqueous layer was separated and acidified with 6 N of HCl solution, then extracted with ethyl acetate. The organic layer was combined, dried and concentrated to give racemic (2'R, 3R, 4'S)-1'-carboxymethyl-6-chloro-4'-(3-chlorophenyl)-2'-(3-fluoro-phenyl) spiro[3H-indole-3,3'-piperidine]-2(1H)-one (Yield 22.5 mg, 61.8%).
HRMS(ES⁺) *m*/*z* Calcd for C₂₆H₂₁Cl₂FN₂O₃ +H [(M+H)⁺]: 499.0986 Found: 499.0982

### Example 44a

### Preparation of intermediate 4-Methyl-2-methylene-pentan-1-ol

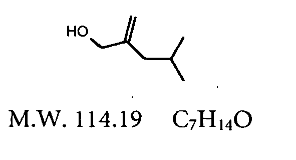

To a solution of propargyl alcohol (14 g, 0.25 mol) (Aldrich) and CuI (40 g, 0.25 mol) (Aldrich) in ether at 0 °C was added isopropylmagnesium chloride (2 M, 375 mL, 0.75 mol) solution in tetrahydrofuran dropwise. The reaction mixture was stirred at room temperature for 48 h. The reaction mixture was quenched with aqueous saturated NH₄Cl solution, extracted with ether twice. The organic layers were combined, washed with water, brine, dried over MgSO₄, filtered and concentrated. The residue was purified with chromatography (EtOAc:Hexane = 1:8) to give 4-methyl-2-methylene-pentan-1-ol as a colorless oil (Yield, 27 g, 95%)

Similar transformation has been reported by Duboudin, J. G.; Jousseaume, B. in J. Organometallic Chem. (1979), 168(1), 1-11 and J. Organometallic Chem. (1975), 91(1), C1-C3.

### Example 44b

### Preparation of intermediate 4-Methyl-2-methylene-pentanal

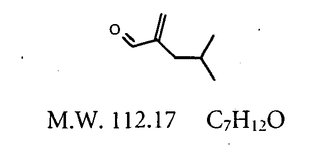

To a solution of oxalyl chloride (32.5 g, 255 mmol) (Aldrich) in dichloromethane (200 mL) at -78 °C was added a solution of dimethyl sulfoxide (36. mL, 510 mmol) in dichloromethane (40 mL) dropwise. After 5 min, the solution of 4-methyl-2-methylene-pentan-1-ol (26.5 g, 230 mmol) prepared in example 44a in dichloromethane (10 mL) was added dropwise. The reaction mixture was stirred at -78 °C for 15 min. Triethylamine (110 mL, 950 mmol) was added and the reaction mixture was slowly warmed to room temperature and stirred at room temperature for 45 min. Water was added, and organic layer was separated. The aqueous layer was extracted with ether. The organic layers were combined, washed with 10% of HCl, saturated NaHCO₃, brine, dried over MgSO₄, and concentrated to give crude 4-methyl-2-methylene-pentanal as a yellow oil (Yield 21 g, 77%).

### Example 44c

### Preparation of intermediate 1-(3-Methyl-1-methylene-butyl)-3-trimethylsilyoxy-2-aza-1,3-butadiene

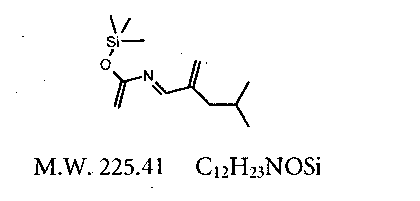

In a manner similar to the method described in example 1b, 4-methyl-2-methylene-pentanal prepared in example 44b (11 g, 100 mmol) was used as the starting material in place of 3-chloro-benzaldehyde to react with 1,1,1,3,3,3-hexamethyldisilazane (16 g, 100 mmol), n-butyllithium (2.5 M, 40 mL, 100 mmol), trimethylsilyl chloride (11 g, 100 mmol), triethylamine (13.6 g, 14 mmol) and acetyl chloride (10.2 g, 14 mmol) to give 1-(3-methyl-1-methylene-butyl)-3-trimethylsilyoxy-2-aza-1,3-butadiene and used for the next step without further purification.

### Example 44d

### Preparation of intermediate racemic (2'R, 3R, 4'S)-6-chloro-4'-(3-chlorophenyl)-2'-(3-methyl-1-methylene-butyl) spiro[3H-indole-3,3'-piperidine]-2,6'(1H)-dione

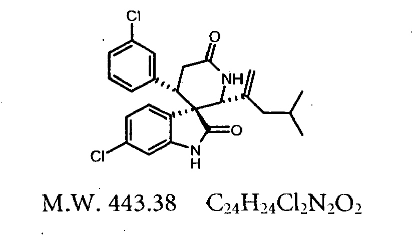

In a manner similar to the method described in example 15b, E/Z-6-chloro-3-(3-chlorobenzylidene)-2-oxo-2,3-dihydro-indole-1-carboxylic acid tert-butyl ester prepared in example 15a (2 g, 5 mmol) was reacted with 1-(3-methyl-1-methylene-butyl)-3-trimethylsilyoxy-2-aza-1,3-butadiene (21 g, 93 mmol) prepared in example 44c in toluene and then trifluoroacetic acid (20 mL) in dichloromethane to give racemic (2'R, 3R, 4'S)-6-chloro-4'-(3-chlorophenyl)-2'-(3-methyl-1-methylene-butyl) spiro[3H-indole-3,3'-piperidine]-2,6'(1H)-dione as a white solid (1.3 g, 59%).
HRMS(ES⁺) *m*/*z* Calcd for C₂₄H₂₄Cl₂N₂O₂ + H [(M+H)⁺]: 443.1288. Found: 443.1285

### Example 44e

### Preparation of intermediate racemic (2'R, 3R, 4'S)-6-chloro-4'-(3-chlorophenyl)-2'-(1-methylene-butyl)-6'-thioxo spiro[3H-indole-3,3'-piperidirie]-2(1H)-one

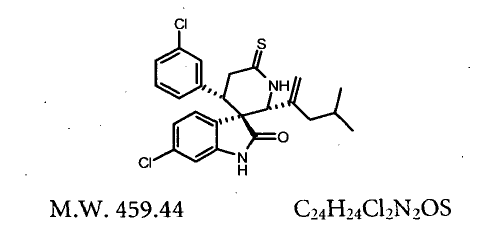

In a manner similar to the method described in example 2c, racemic (2'R, 3R, 4'S)-6-chloro-4'-(3-chlorophenyl)-2'-(1-methylene-butyl) spiro[3H-indole-3,3'-piperidine]-2,6'(1H)-dione (1.4 g, 3.1 mmol) prepared in example 44d was reacted with 2,4-bis-(4-methoxyphenyl)-1,3-dithia-2,4-diphosphetane 2,4-disulfide (1.7 g, 4.25 mmol) in toluene to give racemic (2'R, 3R, 4'S)-6-chloro-4'-(3-chlorophenyl)-2'-(1-methylene-butyl)-6'-thioxo spiro[3H-indole-3,3'-piperidine]-2(1H)-one as a off white solid (Yield 1.2 g, 84%).
HRMS(ES⁺) *m*/*z* Calcd for C₂₄H₂₄Cl₂N₂OS +H [(M+H)⁺]: 459.1059. Found: 459.1055.

### Example 44f

### Preparation of intermediate racemic (2'R, 3R, 4'S)-[6-chloro-4'-(3-chlorophenyl)-2'-(1-methylene-butyl)-2,3-dihydro-2-oxospiro[3H-indole-3,3'-piperidin]-6-ylene] hydrazine carboxylic acid tert-butyl ester

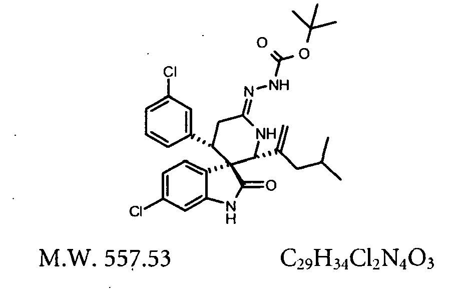

In a manner similar to the method described in example 20b, racemic (2'R, 3R, 4'S)-6-chloro-4'-(3-chlorophenyl)-2'-(1-methylene-butyl)-6'-thioxo spiro[3H-indole-3,3'-piperidine]-2(1H)-one (0.7 g, 1.5 mmol) prepared in example 44e was reacted with tert-butyl carbazate (1.4 g, 11 mmol), mercuric acetate (0.5 g, 1.6 mmol) in tetrahydrofuran (50 mL) to give racemic (2'R, 3R, 4'S)-[6-chloro-4'-(3-chlorophenyl)-2'-(1-methylene-butyl)-2,3-dihydro-2-oxospiro[3H-indole-3,3'-piperidin]-6-ylene] hydrazine carboxylic acid tert-butyl ester as a off white solid (Yield 0.8 g, 96%).

### Example 44g

### Preparation of intermediate racemic (2'R, 3R, 4'S)-6-chloro-4'-(3-chlorophenyl)6'-hydrazono-2'-(1-methylene-butyl)-spiro[3H-indole-3,3'-piperidine]-2(1H)-one

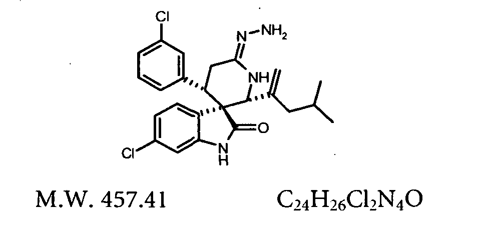

Trifluoroacetic acid (10 mL) was added to a solution of racemic (2'R, 3R, 4'S)-[6-chloro-4'-(3-chlorophenyl)-2'-(-methylene-butyl)-2,3-dihydro-2-oxospiro[3H-indole-3,3'-piperidin]-6-ylene] hydrazine carboxylic acid tert-butyl ester (0.6 g, 1.1 mmol) prepared in example 44f in dichloromethane (20 mL). The mixture was stirred at room temperature for 1 h. The solvent was evaporated. To this residue was added saturated NaHCO₃ solution, and extracted with ethyl acetate. The organic layers were combined, washed with water and brine, dried over MgSO₄ and *in vacuo* overnight to give crude racemic (2'R, 3R, 4'S)-6-chloro-4'-(3-chlorophenyl)-6'-hydrazono-2'-(1-methylene-butyl)-spiro[3H-indole-3,3'-piperidine]-2(1H)-one as a yellow gum (Yield: 0.5 g, 100%).

### Example 44h

### Preparation of racemic (5'R, 3R, 7'S)-6-chloro-7'-(3-chlorophenyl)-5'-(1-methylene-butyl)-5',6',7',8'-tetrahydro-spiro[3H-indole-3,6'-(1,2,4-triazolo[4,3-a] pyridine)]-2(1H)-one

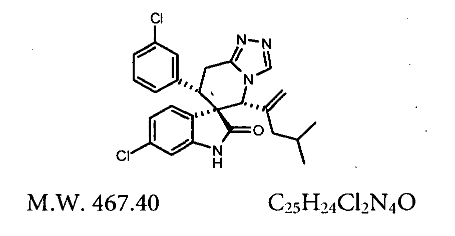

Racemic (2'R, 3R, 4'S)-6-chloro-4'-(3-chlorophenyl)-6'-hydrazono-2'-(1-methylene-butyl)-spiro[3H-indole-3,3'-piperidine]-2(1H)-one (0.2 g, 0.43 mmol) prepared in example 44g was refluxing in formic acid (20 mL, 85%, Aldrich) for 1.5 h. The reaction mixture was cooled to room temperature, concentrated. To the residue was added saturated NaHCO₃ solution, and extracted with ethyl acetate-methanol (9;1) twice. The organic layers were combined, washed with water and brine, dried over MgSO₄ and purified by column chromatography (EtOAc: MeOH=10;1) to give the desired racemic (5'R, 3R, 7'S)-6-chloro-7'-(3-chlorophenyl)-5'-(1-methylene-butyl)-5',6',7',8'-tetrahydro-spiro[3H-indole-3,6'-(1,2,4-triazolo[4,3-a]pyridine)]-2(1H)-one as a white solid (Yield 0.15 g, 75%).
HRMS(ES⁺) *m*/*z* Calcd for C₂₅H₂₄Cl₂N₄O +H [(M+H)⁺]: 467.1400. Found: 467.1397.

### Example 44i

### Preparation of chiral (5'R, 3R, 7'S)-6-chloro-7'-(3-chlorophenyl)-5'-(1-methylene-butyl)-5',6',7',8'-tetrahydro-spiro[3H-indole-3,6'-(1,2,4-triazolo[4,3-a]pyridine)]-2((1H)-one

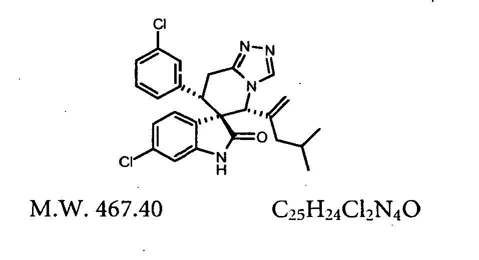

Separation of the two enantiomers from racemic racemic (5'R, 3R, 7'S)-6-chloro-7'-(3-chlorophenyl)-5'-(1-methylene-butyl)-5',6',7',8'-tetrahydro-spiro[3H-indole-3,6'-(1,2,4-triazolo[4,3-a]pyridine)]-2(1H)-one (85 mg) prepared in example 44h was conducted by chiral SFC to provide chiral (5'R, 3R, 7'S)-6-chloro-7'-(3-chlorophenyl)-5'-(1-methylene-butyl)-5',6',7',8'-tetrahydro-spiro[3H-indole-3,6'-(1,2,4-triazolo[4,3-a]pyridine)]-2(1H)-one as a white solid (31 mg, 37%) (RO5170646-000) and chiral (5'S, 3S, 7'R)-6-chloro-7'-(3-chlorophenyl)-5'-(1-methylene-butyl)-5',6',7',8'-tetrahydrospiro[3H-indole-3,6'-(1,2,4-triazolo[4,3-a]pyridine)]-2(1H)-one as a white solid (30 mg, 35%).

### Example 45

### Preparation of racemic (5'R, 3R, 7'S)-6-chloro-7'-(3-chlorophenyl)-3'-methyl-5'-(1-methylene-butyl)-5',6',7',8'-tetrahydro-spiro[3H-indole-3,6'-(1,2,4-triazolo[4,3-a]pyridine)]-2(1H)-one

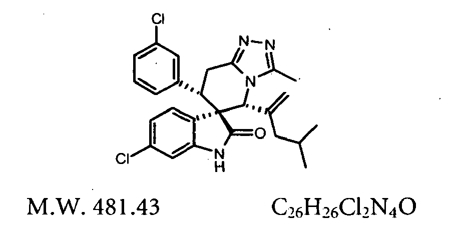

In a manner similar to the method described in example 44i, racemic (2'R, 3R, 4'S)-6-chloro-4'-(3-chlorophenyl)-6'-hydrazono-2'-(1-methylene-butyl)-spiro[3H-indole-3,3'-piperidine]-2(1H)-one (0.16 g, 0.35 mmol) prepared in example 44h was refuxing in glacial acetic acid (10 mL) for 3 h to give racemic (5'R, 3R, 7'S)-6-chloro-7'-(3-chlorophenyl)-3'-methyl-5'-(1-methylene-butyl)-5',6',7',8'-tetrahydro-spiro[3H-indole-3,6'-(1,2,4-triazolo[4,3-a]pyridine)]-2(1H)-one as a white solid (Yield 29 mg, 17%). HRMS(ES⁺) *m*/*z* Calcd for C₂₆H₂₆Cl₂N₄O + H [(M+H)⁺]: 481.1557. Found: 481.1554.

### Example 46

### Preparation of racemic (2'R, 3'R,4'S)-6-chloro-4'-(3-chlorophenyl)-2'-(5-fluoro-2-methyl-phenyl)-6'-(1,4-piperazinyl)-spiro [3H-indole-3,3'-2',3',4',5'-tetrahydro-pyridine]-2-one

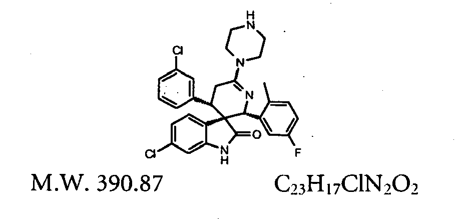

To a stirred solution of racemic (2'R, 3R, 4'S)-6-chloro-4'-(3-chlorophenyl)-2'-(5-fluoro-2-methylphenyl)-6'-thioxo spiro[3H-indole-3,3'-piperidine]-2(1H)-one (48.1 mg, 0.1 mmol) prepared in example 21a in THF (3 mL), HgCl₂ (Aldrich, 100 mg) and piperazine (Aldrich, 25 mg, 0.30 mmol) were added and the mixture was heated at reflux overnight. The mixture was cooled to room temperature and filtered. The filtrate was concentrated and the residue was chromatographied ( 30 min. 5% 2M ammonia in methanol/EtOAc) on an ISCO machine to give an off-white solid (Yield, 24 mg, 45%).
MS *m*/*z* (M+H)⁺: 537

### Example 47

### Preparation of racemic (2'R, 3'R,4'S)-6-chloro-4'-(3-chlorophenyl)-2'-(5-fluoro-2-methyl-phenyl)-6'- [4- [1,1-dimethylethoxy-carbonyl] amino] ethylamino-spiro [3H-indole-3,3'-2',3',4',5'-tetrahydro-pyridine]-2-one

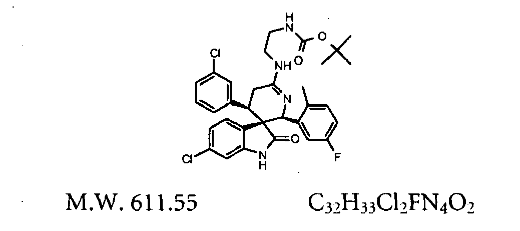

To a stirred solution of racemic (2'R, 3R, 4'S)-6-chloro-4'-(3-chlorophenyl)-2'-(5-fluoro-2-methylphenyl)-6'-thioxo spiro[3H-indole-3,3'-piperidine]-2(1H)-one (96.2 mg, 0.2 mmol) prepared in example 21a in THF (6 mL), HgCl₂ (Aldrich, 82 mg, 0.3 mmol) and (2-amino-ethyl)-carbamic acid tert-butyl ester (Aldrich, 48 mg, 0.30 mmol) were added and the mixture was heated at reflux overnight. The mixture was cooled to room temperature and filtered. The filtrate was concentrated and the residue was chromatographied ( 30 min. 5% methanol/EtOAc) on an ISCO machine to give an off-white solid. 52 mg, 50 %.
MS *m*/*z* (M+H)⁺: 611

### Example 48

### Preparation of racemic (2'R, 3'R,4'S)-6-chloro-4'-(3-chlorophenyl)-2'-(5-fluoro-2-methyl-phenyl)-6'-[4-amino] ethylamino-spiro [3H-indole-3,3'-2',3',4',5'-tetrahydro-pyridine]-2-one, trifluoroacetic acid

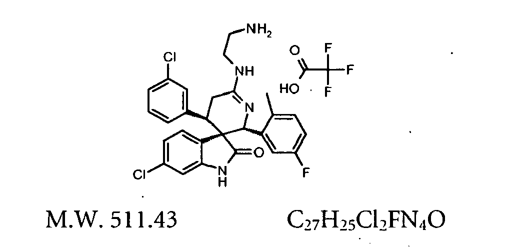

The racemic (2'R, 3'R,4'S)-6-chloro-4'-(3-chlorophenyl)-2'-(5-fluoro-2-methyl-phenyl)-6'- [4-[1,1-dimethylethoxy-carbonyl]amino]ethylamino-spiro[3H-indole-3,3'-2',3',4',5'-tetrahydro-pyridine]-2-one (40 mg, 0.074 mmol) prepared in example 47 was dissolved in 1.5 ml of 30% TFA/CH₂Cl₂ and the mixture was stirred at room temperature for 30 minutes. The solvent was removed under reduced pressure and the residue was lyophilized to give an off-white solid. 44 mg.
MS *m*/*z* (M+H)⁺: 511

### Example 49

### Preparation of racemic (2'R, 3'R,4'S)-6-chloro-4'-(3-chlorophenyl)-2'-(5-fluoro-2-methyl-phenyl)-6'-(4-methyl-1-piperazinyl)-spiro[3H-indole-3,3'-2',3',4',5'-tetrahydro-pyridine]-2-one

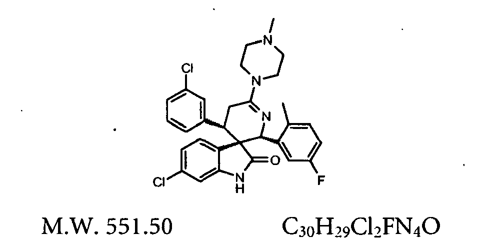

To a stirred solution of racemic (2'R, 3R, 4'S)-6-chloro-4'-(3-chlorophenyl)-2'-(5-fluoro-2-methylphenyl)-6'-thioxo spiro[3H-indole-3,3'-piperidine]-2(1H)-one (48.4 mg, 0.1 mmol) prepared in example 21a in THF (3 mL), HgCl₂ (Aldrich, 82 mg, 0.3 mmol) and N-methyl-piperazine (Aldrich, 29.4 mg, 0.30 mmol) were added and the mixture was heated to reflux overnight. The mixture was cooled to room temperature and filtered. The filtrate was concentrated and the residue was chromatographied ( 30 min. 5% 2M ammonia in methanol/EtOAc) on an ISCO machine to give an off-white solid. 22 mg, 40%.
MS *m*/*z* (M+H)⁺: 551

### Example 50a

### Preparation of intermediate 1,1-Dioxo-tetrahydro-thiopyran-4-one

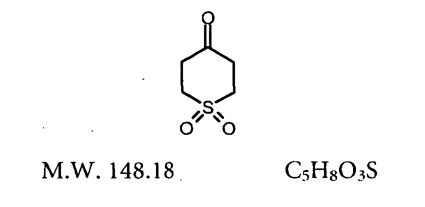

To a stirred solution of tetrahydrothiopyran-4-one (Aldrich, 5.30 g, 43.1 mmol) in 50 mL of EtOAc was added dropwise 32% peracetic acid (24 g, 110 mmol) at a rate to avoid reflux. After the addition, the mixture was cooled to room temperature and the solid filtered to give a white solid, 5.69 g, 89%.

### Example 50b

### Preparation of intermediate 4-(1,1-Dioxo-hexahydro-thiopyran-4-yl)-piperazine-1-carboxylic acid tert-butyl ester

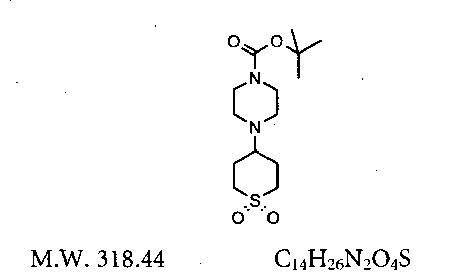

Tetrahydro-4H-thiopyran-4-one (6g, 40.5 mmol) was dissolved in 1.2-dichloroethane (250 ml) with some help of heating. When the temperature goes back to room temperature, 1-Boc-piperazine (Aldrich, 7.62g, 41 mmol), sodium triacetoxyborohydride (Aldrich, 17.01, 56.7 mmol) were added followed by glacial acetic acid (2.4 g, 41 mmol). The reaction mixture was stirred at room temperature overnight. The reaction was quenched with water and the mixture was partitioned. The organic layer was separated and the aqueous layer was extracted with 1.2-dichloroethane (3x20mL). The extracts were combined to the organic portion and the solution was dried over Na₂SO₄, filtered and concentrated to give a white solid. The white solid was purified on an ISCO machine, eluting with 2-7% MeOH/EtOAc in a period of 30 minutes to give a white solid. Yield, 9.3 g, 69%.
MS m/z (M+H)⁺: 319

### Example 50c

### Preparation of intermediate 1-(1,1-Dioxo-hexahydro-thiopyran-4-yl)-piperazine

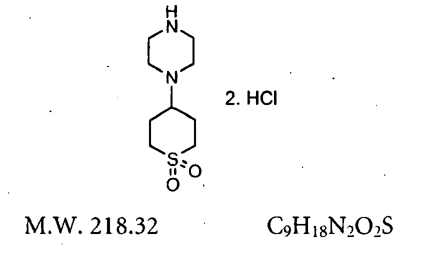

To a stirred solution of 4-(1,1-dioxo-hexahydro-thiopyran-4-yl)-piperazine-1-carboxylic acid tert-butyl ester (8.02 g, 25 mmol) in methanol at 45 °C (150 mL) was added 4 N HCl in 1.4-dioxane (100 mmol, 25 mL). The mixture was stirred at 45 °C for 7 h until TLC (5% methanol in EtOAc) indicates complete reaction. The solvent was removed under reduced pressure to give a white solid. 7.24 g, 99.5%.
MS *m*/*z* (M+H)⁺: 218

### Example 50d

### Preparation of racemic (2'R, 3'R,4'S)-6-chloro-4'-(3-chlorophenyl)-2'-(5-fluoro-2-methyl-phenyl)-6'-[[tetrahydro-1,1-dioxido-2H-thiopyran-4-yl]piperazinyl-spiro[3H-indole-3,3'-2',3',4',5'-tetrahydro-pyridine]-2-one

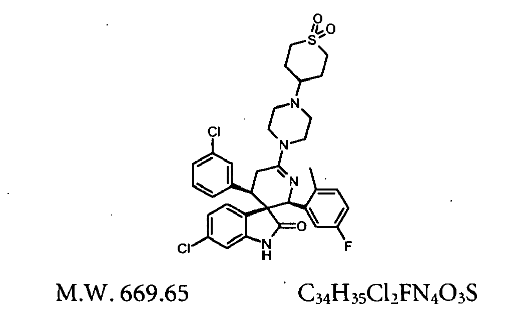

To a stirred solution of racemic (2'R, 3R, 4'S)-6-chloro-4'-(3-chlorophenyl)-2'-(5-fluoro-2-methylphenyl)-6'-thioxo spiro[3H-indole-3,3'-piperidine]-2(1H)-one (48.4 mg, 0.1 mmol) prepared in example 21a in THF (4 mL), HgCl₂ (Aldrich, 105 mg, 0.38 mmol) and 1-(1,1-dioxo-hexahydro-thiopyran-4-yl)-piperazine (Example 243c, 44 mg, 0.15 mmol) and triethylamine (45 mg, 0.45 mmol) were added and the mixture was heated to reflux overnight. The mixture was cooled to room temperature and filtered. The filtrate was concentrated and the residue was chromatographied ( 30 min. 5% methanol/EtOAc) on an ISCO machine to give an off-white solid. 21 mg; 40%.
MS *m*/*z* (M+H)⁺: 669

### Example 51

### Preparation of racemic (2'R, 3'R,4'S)-6-chloro-4'-(3-chlorophenyl)-2'-(5-fluoro-2-methyl-phenyl)-6'-[4-(3-methylsulphonyl)-propyl]piperazinyl-spiro[3H-indole-3,3'-2',3',4',5'-tetrahydro-pyridine]-2-one

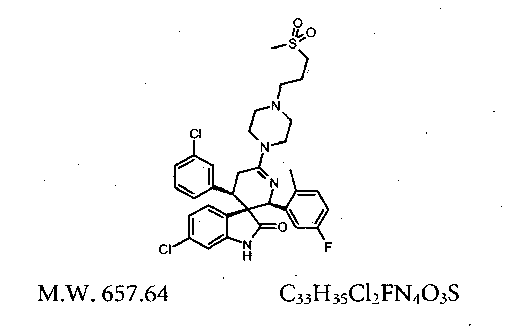

To a stirred solution of racemic (2'R, 3R, 4'S)-6-chloro-4'-(3-chlorophenyl)-2'-(5-fluoro-2-methylphenyl)-6'-thioxo spiro[3H-indole-3,3'-piperidine]-2(1H)-one (48.4 mg, 0.1 mmol) prepared in example 21a in THF (4 mL), HgCl₂ (Aldrich, 91 mg, 0.33 mmol) and 1-(3-methanesulfonyl-propyl)-piperazine (US23289, 36.4 mg, 0.15 mmol) and triethylamine (31 mg, 0.30 mmol) were added and the mixture was heated to reflux overnight. The mixture was cooled to room temperature and filtered. The filtrate was concentrated and the residue was chromatographied (30 min. 5% methanol/EtOAc) on an ISCO machine to give an off-white solid. 14 mg, 21%.
MS *m*/*z* (M+H)⁺: 657

### Example 52

### Preparation of racemic (2'R, 3'R,4'S)-6-chloro-4'-(3-chlorophenyl)-2'-(5-fluoro-2-methyl-phenyl)-6'-[1-methylsulphonyl-4-piperidinyl]amino-spiro[3H-indole-3,3'-2',3',4',5'-tetrahydro-pyridine]-2-one

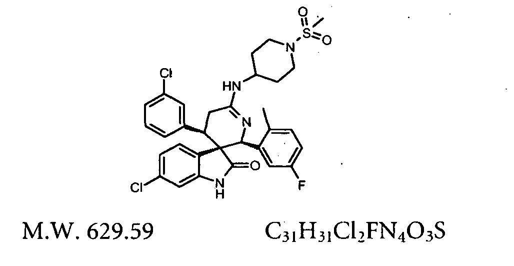

To a stirred solution of racemic (2'R, 3R, 4'S)-6-chloro-4'-(3-chlorophenyl)-2'-(5-fluoro-2-methylphenyl)-6'-thioxo spiro[3H-indole-3,3'-piperidine]-2(1H)-one (48.4 mg, 0.1 mmol) prepared in example 21a in THF (4 mL), HgCl₂ (Aldrich, 35 mg, 0.12 mmol) and 1-methanesulfonyl-piperidin-4-ylamine (WO 2003/097048, 87.6 mg, 0.15 mmol) and triethylamine (41 mg, 0.40 mmol) were added and the mixture was heated to reflux overnight. The mixture was cooled to room temperature and filtered. The filtrate was concentrated and the residue was chromatographied ( 30 min. 5% methanol/EtOAc) on an ISCO machine to give an off-white solid. 8 mg, 13%.
MS *m*/*z* (M+H)⁺: 630

### Example 53

### In Vitro Activity Assay

The ability of the compounds to inhibit the interaction between p53 and MDM2 proteins was measured by an HTRF (homogeneous time-resolved fluorescence) assay in which recombinant GST-tagged MDM2 binds to a peptide that resembles the MDM2-interacting region of p53 (Lane et al.). Binding of GST-MDM2 protein and p53-peptide (biotinylated on its N-terminal end) is registered by the FRET (fluorescence resonance energy transfer) between Europium (Eu)-labeled anti-GST antibody and streptavidin-conjugated Allophycocyanin (APC).

Test is performed in black flat-bottom 384-well plates (Costar) in a total volume of 40 uL containing:90 nM biotinylate peptide, 160 ng/ml GST-MDM2, 20 nM streptavidin-APC (PerkinElmerWallac), 2 nM Eu-labeled anti-GST-antibody (PerkinElmerWallac), 0.2% bovine serum albumin (BSA), 1 mM dithiothreitol (DTT) and 20 mM Tris-borate saline (TBS) buffer as follows: Add 10 uL of GST-MDM2 (640 ng/ml working solution) in reaction buffer to each well. Add 10 uL diluted compounds (1:5 dilution in reaction buffer) to each well, mix by shaking. Add 20 uL biotinylated p53 peptide (180 nM working solution) in reaction buffer to each well and mix on shaker. Incubate at 37°C for 1 h. Add 20 uL streptavidin-APC and Eu-anti-GST antibody mixture (6 nM Eu-anti-GST and 60 nM streptavidin-APC working solution) in TBS buffer with 0.2% BSA, shake at room temperature for 30 minutes and read using a TRF-capable plate reader at 665 and 615 nm (Victor 5, Perkin ElmerWallac). If not specified, the reagents were purchased from Sigma Chemical Co.
IC₅₀'s showing the biological activity of this invention exhibit activities less than about 10µM. Some representative values are:

| Example No. | IC₅₀(µM) 0.2% BSA |
|---|---|
| 8c | 5.2123 |
| 11c | 6.9498 |
| 16c | 2.3103 |
| 44h | 1.0308 |

## Claims

1. A compound selected from the group consisting of 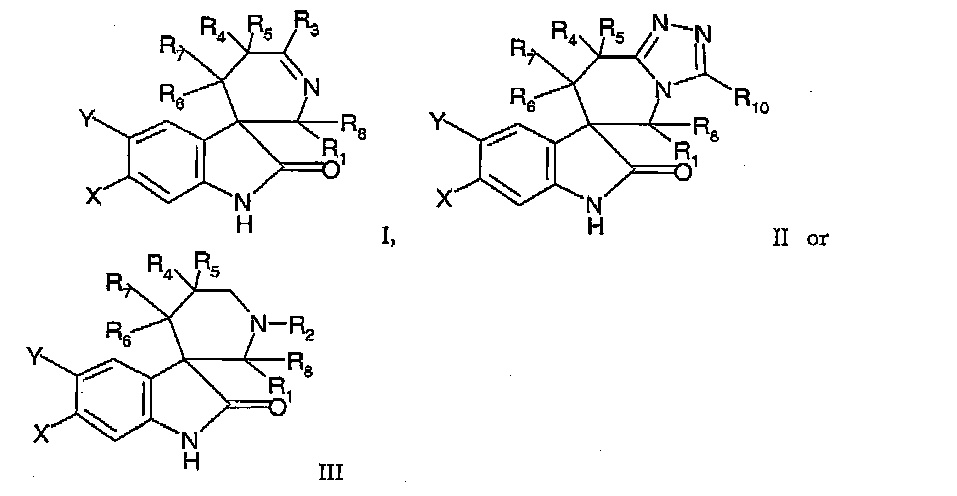 wherein
X is selected from the group consisting of hydrogen, halogen, cyano, nitro, ethynyl, cyclopropyl, methyl, ethyl, isopropyl, methoxy, and vinyl,
Y is hydrogen or fluorine,
R₄, R₅ are selected from the group consisting of hydrogen and C₁-C₆-alkyl, one of R₁ and R₈ is selected from the group consisting of C₁-C₆-alkyl, substituted C₁-C₆-alkyl, C₂-C₆-alkenyl, substituted C₂-C₆-alkenyl, aryl, substituted aryl, heteroaryl, substituted heteroaryl, heterocycle, substituted heterocycle, cycloalkyl, substituted cycloalkyl, cycloalkenyl, and substituted cycloalkenyl and the other is hydrogen,
one of R₆ and R₇ is selected from the group consisting of C₁-C₆-alkyl, substituted C₁-C₆-alkyl, C₂-C₆-alkenyl, substituted C₂-C₆-alkenyl, aryl, substituted aryl, heteroaryl, substituted heteroaryl, heterocycle, substituted heterocycle, cycloalkyl, substituted cycloalkyl, cycloalkenyl, and substituted cycloalkenyl and the other is hydrogen, C₁-C₆-alkyl, or cyano
R₂ is selected from the group consisting of hydrogen, C₁-C₆-alkyl, substituted C₁-C₆-alkyl, C(=O)R₉, C(=O)NHR₉, C(=O)NR₉R₉, and C(=O)OR₉,
R₃ is selected from the group consisting of NHR₉, SR₉, and NR₉R₉,
R₉ is selected from the group consisting of C₁-C₆-alkyl, substituted C₁-C₆-akyl, aryl, substituted aryrl, heteroaryl, substituted heteroaryl, heterocycle, substituted heterocycle, cycloalkyl and substituted cycloalkyl,
R₉ is selected from the group consisting of C₁-C₆-alkyl and substituted C₁-C₆-alkyl and in the case of R₉ and R_{9'} they may independently link to form a cyclic structure selected from heterocycle or substituted heterocycle and
R₁₀ is selected from the group consisting of hydrogen, hydroxyl and C₁-C₆-akyl and the pharmaceutically acceptable salts and esters thereof;
wherein
aryl is a monovalent, monocyclic or bicyclic, aromatic carbocyclic hydrocarbon radical; heteroaryl is an aromatic heterocyclic ring system containing up to two rings; and heterocycle is a substituted or unsubstituted 5 to 8 membered, mono- or bicyclic, aromatic or non-aromatic hydrocarbon, wherein 1 to 3 carbon atoms are replaced by a hetero atom selected from nitrogen,oxygen or sulfur atom.

2. A compound of claim 1 wherein said compound is a compound of the formula 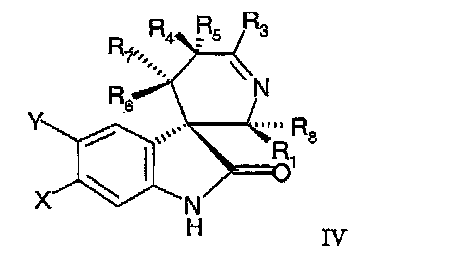 wherein
X is Cl or Br,
Y is hydrogen,
R₁ is hydrogen,
R₄ and R₅ are both hydrogen,
R₆ is hydrogen,
R₇ is a substituted phenyl or substituted heteroaryl selected from group consisting of 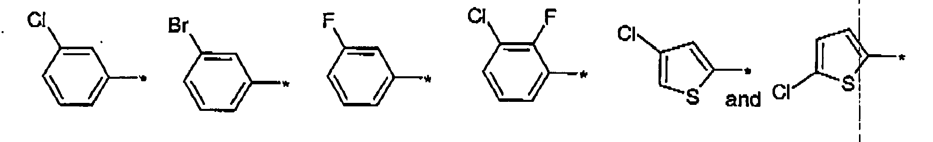 R₈ is selected from the group consisting of C₁-C₆-alkyl, substituted C₁-C₆-alkyl, C₂-C₆-alkenyl, substituted C₂-C₆-alkenyl, aryl, substituted aryl, heteroaryl, substituted heteroaryl, cycloalkyl, substituted cycloalkyl, cycloalkenyl, and substituted cycloalkenyl, R₃ is selected from the group consisting of NHR₉ and NR₉R_{9'},
R₉ is selected from the group consisting of C₁-C₆-alkyl, substituted C₁-C₆-alkyl, aryl, substituted aryl, heteroaryl, substituted heteroaryl, heterocycle, substituted heterocycle, cycloalkyl and substituted cycloalkyl,
R₉ is selected from the group consisting of C₁-C₆-alkyl and substituted C₁-C₆-alkyl and.in the case of R₉ and R_{9'} they may independently link to form a cyclic structure selected from heterocycle or substituted heterocycle
and the pharmaceutically acceptable salts and esters thereof.

3. A compound of claim 1 wherein said compound is a compound of the formula 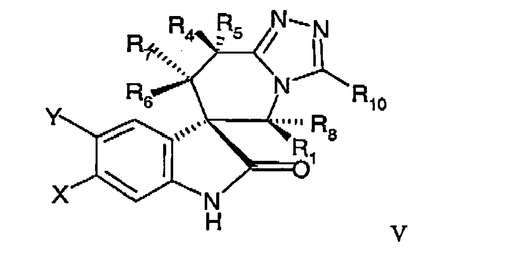 wherein
X is Cl or Br,
Y is hydrogen,
R₁ is hydrogen,
R₄ and R₅ are both hydrogen,
R₆ is hydrogen,
R₇ is a substituted phenyl or substituted heteroaryl selected from group consisting of 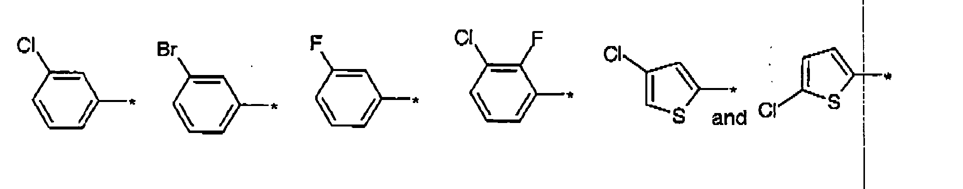
R₈ is selected from the group consisting of C₁-C₆-alkyl, substituted C₁-C₆-alkyl, C₂-C₆-alkenyl, substituted C₂-C₆-alkenyl, aryl, substituted aryl, heteroaryl, substituted heteroaryl, cycloalkyl, substituted cycloalkyl, cycloalkenyl and substituted cycloalkenyl, R₁₀ is selected from the group consisting of hydrogen, hydroxyl, and methyl and the pharmaceutically acceptable salts and esters thereof.

4. A compound of claim 1 wherein said compound is a compound of the formula 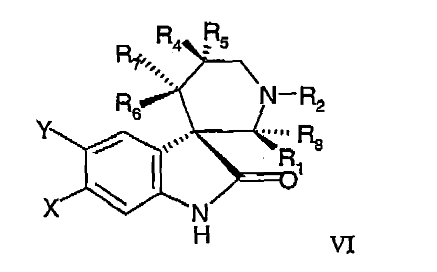 wherein
X is Cl or Br
Y is hydrogen,
R₁ is hydrogen,
R₄ and R₅ are both hydrogen,
R₆ is hydrogen,
R₇ is a substituted phenyl or substituted heteroaryl selected from group consisting of 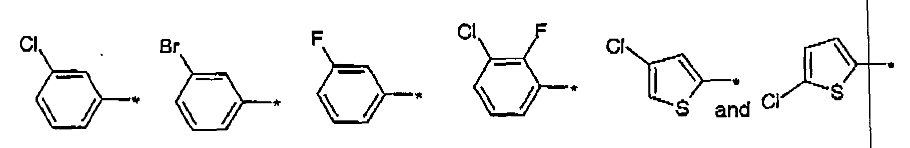
R₈ is selected from the group consisting of C₁-C₆-alkyl, substituted alkyl, C₂-C₆-alkenyl, substituted C₂-C₆-alkenyl, aryl, substituted aryl, heteroaryl, substituted heteroaryl, cycloalkyl, substituted cycloalkyl, cycloalkenyl and substituted cycloalkenyl,
R₂ is selected from the group consisting of hydrogen, C₁-C₆-alkyl, substituted C₁-C₆-alkyl, C(=O)R₉, C(=O)NHR₉, C(=O)NR₉R_{9'} and C(=O)OR₉,
R₉ is selected from the group consisting of C₁-C₆-alkyl, substituted C₁-C₆-alkyl, aryl, substituted aryl, heteroaryl, substituted heteroaryl, heterocycle, cycloalkyl and substituted cycloalkyl,
R₉ is selected from the group consisting of C₁-C₆-alkyl and substituted C₁-C₆-alkyl and in the case of R₉ and R_{9'} they may independently link to form a cyclic structure selected from heterocycle and substituted heterocycle
and the pharmaceutically acceptable salts and esters thereof.

5. A compound of claim 1 wherein said compound is a compound of the formula 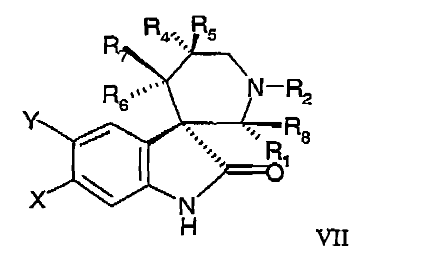 wherein
X is Cl or Br,
Y is hydrogen,
R₁ is hydrogen,
R₄ and R₅ are both hydrogen,
R₆ is hydrogen,
R₈ is a substituted phenyl selected from group consisting of 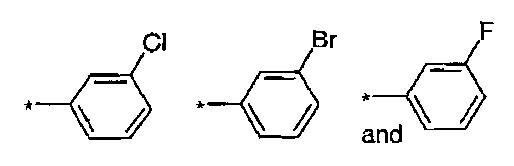 and
R₇ is selected from the group consisting of C₁-C₆-alkyl, substituted C₁-C₆-alkyl, C₂-C₆-alkenyl, substituted C₂-C₆-alkenyl, aryl, substituted aryl, heteroaryl, substituted heteroaryl, heterocycle, substituted heterocycle, cycloalkyl, substituted cycloalkyl, cycloalkenyl and substituted cycloalkenyl.
R₂ is selected from the group consisting of hydrogen, C₁-C₆-alkyl, substituted C₁-C₆-alkyl, C(=O)R₉, C(=O)NHR₉, C(=O)NR₉R_{9'} and C(=O)OR₉,
R₉ is selected from the group consisting of C₁-C₆-alkyl, substituted C₁-C₆-alkyl, aryl, substituted aryl, heteroaryl, substituted heteroaryl, heterocycle, cycloalkyl and substituted cycloalkyl,
R₉· is selected from the group consisting of C₁-C₆-alkyl and substituted C₁-C₆-alkyl and in the case of R₉ and R_{9'} they may independently link to form a cyclic structure selected from heterocycle and substituted heterocycle
and the pharmaceutically acceptable salts and esters thereof.

6. A compound of claim 1 selected from the group consisting of
racemic (2'S, 3S, 4'S)-6-chloro-2'-(3-chlorophenyl)-4'-(2,2-dimethylpropyl) spiro[3H-indole-3,3'-piperidine]-2(1H)-one,
racemic (2'S, 3R)-6-chloro-2'-(3-chlorophenyl)-2',3',4',5'-tetrahydro-4'-isopropyl-6'-(methylthio) spiro[3H-indole-3,3'-pyridin]-2(1H)-one,
racemic (2'S, 3S, 4'S)-6-chloro-2-(3-chlorophenyl)-4'-ethyl spiro[3H-indole-3,3'-piperidine]-2(1H)-one,
racemic (2'R, 3R, 4'S)-6-cloro-4'-(3-chlorophenyl)-2'-(3-methylphenyl) spiro[3H-indoie-3,3'-piperidine]-2(1H)-one,
racemic (2'R, 3R, 4'S)-6-chloro-4'-(3-chlorophenyl)-2'-(3-fluorophenyl) spiro[3H-indole-3,3'-piperidine]-2(1H)-one,
racemic (2'S, 3S, 4'R)-6-chloro-2'-(3-chlorophenyl)-4'-phenyl spiro[3H-indole-3,3'-pipendine]-2(1H)-one,
racemic (2'R, 3R, 4'S, 5'S)-6-chloro-4'-(3-chlorophenyl)-5'-methyl-2'-(2-methylphenyl) spiro [3H-indole-3,3'-piperidine]-2(1H)-one,
racemic (2'R, 3R, 4'S)-6-chloro-4'-(3-chlorophenyl)-2'-(3-methoxyphenyl) spiro[3H-indole-3,3'-piperidine]-2(1H)-one,
racemic (2'R, 3R, 4'S)-6-chloro-4'-(3-chlorophenyl)-2'-(3-cyanophenyl) spiro[3H-indole-3,3'-piperidine]-2(1H)-one and
racemic(2'R,3R,4'S)-6-chloro-4'-(3-chlorophenyl)-2'-(2,3-dimethylphenyl) spiro[3H-indole-3,3'-piperidine]-2(1H)-one.

7. A compound of claim 1 selected from the group consisting of
racemic (2'R, 3R, 4'S)-6-chloro-4'-(3-chlorophenyl)-2'-[2-(trifluoromethyl)-phenyl)] spiro[3H-indole-3,3'-piperidine]-2(1H)-one,
racemic (2'S, 3R)-6-chloro-4,'-(3-chlorophenyl)-2',3',4','-tetrahydro-6'-(methylthio)-2'-[2-(trifluoromethyl)-phenyl] spiro[3H-indole-3,3'-pyridin]-2(1H)-one,
racemic (2'R, 3R, 4'S)-6-chloro-4'-(3-chlorophenyl)-2'-[5-fluoro-2-(trifluoromethyl)-phenyl] spiro[3H-indole-3,3'-piperidine]-2(1H)-one,
racemic (2'R, 3R, 4'S)-6-chloro-4'-(3-chlorophenyl)-2'-(5-fluoro-2-methylphenyl) spio[3H-indole-3,3'-piperidine]-2(1H)-one,
racemic (2'R, 3R, 4'S)-6-chloro-4'-(3-chlorophenyl)-2'-(2,4-difluorophenyl) spiro[3H-indole-3,3'-piperidine]-2(1H)-one,
racemic (2'R, 3R, 4'S)-6-chloro-4'-(3-chlorophenyl)-2'-(5-fluoro-2-methoxyphenyl) spiro[3H-indole-3,3'-piperidine]-2(1H)-one,
racemic (2'R, 3R, 4'S)-6-chloro-4'-(3-chlorophenyl)-2'-(1-naphthalenyl) spiro[3H-indole-3,3'-piperidine]-2(1H)-one,
racemic (2'R, 3R, 4'S)-6-chloro-4'-(3-chlorophenyl)-2'-(3-pyridinyl) spiro[3H-indole-3,3'-piperidine]-2(1H)-one,
racemic (2'R, 3R, 4'S)-6-chloro-4'-(3-chlorophenyl)-2'-(3, 4-difluoro-phenyl) spiro[3H-indole-3,3'-piperidine]-2(1H)-one and
racemic(5'R,3R,7'S)-6-chloro-7'-(3-chlorophenyl)-5'-(3,4-difluorophenyl)-5',6',7',8'-tetrahydro-3'-hydroxyspiro[3H-indole-3,6'-(1,2,4-triazolo[4,3-a]pyridine)]-2(1H)-one.

8. A compound of claim 1 selected from the group consisting of
racemic (5'R, 3R, 7'S)-6-chloro-7'-(3-chlorophenyl)-5'-(5-fluoro-2-methylphenyl)-5',6',7',8'-tetrahydro-3'-hydroxyspiro[3H-indole-3,6'-(1,2,4-triazolo[4,3-a]pyridine)]-2(1H)-one,
racemic (2'R, 3R, 4'S)-6-chloro-4'-(3-chlrophenyl)-2'-(2,3-difluoro-6-methylphen) spiro[3H-indole-3,3'-piperidine]-2(1H)-one,
racemic (2'R, 3R, 4'S)-6-chloro-4'-(3-chlorophenyl)-5-fluoro-2'-(5-fluoro-2-methylphenyl) spiro[3H-indole-3,3'-piperidine]-2(1H)-one,
racemic (2'R, 3R, 4'S)-6-chloro-4'-(3-chlorophenyl)-2'-(1-ethylpropyl) spiro[3H-indole-3,3'-piperidinel-2(1H)-one,
racemic (2'R, 3R, 4'S)-6-chloro-4'-(3-chloro-phenyl)-2'-(2,5-dimethyl-phenyl) spiro[3H-indole-3,3'-piperidine]-2(1H)-one,
racemic (2'R, 3R, 4'S)-6-chloro-4'-(3-chlorophenyl)-2'-(2,5-dimethyl-2H-pyrazole-3-yl) spiro[3H-indole-3,3'-piperidine]-2(1H)-one,
racemic (2'R, 3R, 4'S)-6-chloro-4'-(3-chlorophenyl)-1'-(morpholin-4-carbonyl)-2'-(1-naphthalenyl) spiro[3H-indole-3,3'-piperidine]-2(1H)-one,
racemic (2'S, 3S, 4'R)-6-chloro-4'-(4-chloro-phenyl)-2'-(3-fluoro-phenyl) spiro[3H-indole-3,3'-piperidine]-2(1H)-one,
racemic (2'S, 3S, 4'R)-1'-tert-butylaminocarbonyl-6-chloro-4'-(4-chloro-phenyl)-2'-(3-fluoro-phenyl) spiro[3H-indole-3,3'-piperidine]-2(1H)-one and
racemic (2'S, 3S, 4'R)-6-chloro-4'-(4-chloro-phenyl)-1'-(3-cyano-phenylaminocarbonyl)-2'-(3-fluoro-phenyl) spiro[3H-indole-3,3'-piperidine]-2(1H)-one.

9. A compound of claim 1 selected from the group consisting of
racemic (2'R, 3R, 4'S)-6-chloro-4'-(3-chloro-phenyl)-1'-(3-cyano-phenylaminocarbonyl)-2'-(3-fluoro-phenyl) spiro[3H-indole-3,3'-piperidine]-2(1H)-one,
racemic (2'R, 3R, 4'S)-1'-tert-butylaminocarbonyl-6-chloro-4'-(3-chloro-phenyl)-2-(3-fluoro-phenyl) spiro [3H-indole-3,3'-piperidine]-2(1H) -one,
racemic (2'R, 3R, 4'S)-1'-benzoyl-6-chloro-4'-(3-chloro-phenyl)-2'-(3-fluoro-phenyl) spiro[3H-indole-3,3'-piperidine] -2(1H)-one,
racemic (2'R, 3R, 4'S)-1'-acetyl-6-chloro-4'-(3-chloro-phenyl)-2'-(3-fluoro-phenyl) spiro [3H-indole-3,3'-piperidine]-2(1H)-one,
racemic (2'R, 3R, 4'S)-1'-[4-(amonocarbonylmethyl)-piperazine-1-carbonyl]-6-chloro-4'-(3-chloro-phenyl)-2'-(3-fluoro-phenyl) spiro[3H-indole-3,3'-piperidine]-2(1H)-one,
racemic (2'R, 3R, 4'S)-6-chloro-4'-(3-chloro-phenyl)-2'-(3-fluoro-phenyl)-1'-[4-(3-methanesulfonyl-propyl)-piperazine-1-carbonyl] spiro[3H-indole-3,3'-piperidine]-2(1H)-one,
racemic (2'R, 3R, 4'S)-6-chloro-4'-(3-chloro-phenyl)-2'-(3-fluoro-phenyl)-1'-[4-(2-oxo-2-pyrrolidin-1-yl-ethyl)-piperazine-1-carbonyl]spiro[3H-indole-3,3'-piperidine]-2(1H)-one,
racemic (2'R, 3R, 4'S)-1'-[4-(2-acetylamino-ethyl)-piperazine-1-carbonyl]-6-chloro-4'-(3-chloro-phenyl)-2'-(3-fluoro-phenyl) spiro[3H-indole-3,3'-piperidine]-2(1H)-one and
racemic (2'R, 3R, 4'S)-6-chloro-4'-(3-chloro-phenyl)-2'-(3-fluoro-phenyl)-1'-[4-(2-hydroxy-ethyl)-piperazine-1-carbonyl]spiro[3H-indole-3,3'-piperidine]-2(1H)-one.

10. A compound of claim 1 selected from the group consisting of
racemic (2'R, 3R, 4'S)-6-chloro-4'-(3-chloro-phenyl)-1'-cyclohexylaminocarbonyl-2'-(3-fluoro-phenyl) spiro [3H-indole-3,3'-piperidine] -2(1H)-one,
racemic (2'R, 3R, 4'S)-1'-benzylaminocarbonyl-6-chloro-4'-(3-chloro-phenyl)-2'-(3-fluoro-phenyl) spiro[3H-indole-3,3'-piperidine]-2(1H)-one,
racemic (5'R, 3R, 7'S)-6-chloro-7'-(3-chlorophenyl)-5'-(2,3,-difluoro-6-isopropoxyphenyl)-5',6',7',8'-tetrahydro-3'-hydroxyspiro[3H-imdole-3,6'-(1,2,4-triazolo[4,3-a]pyridine)]-2(1H)-one,
chiral (5'R, 3R, 7'S)-6-chloro-7'-(3-chlorophenyl)-5'-(2,3-difluoro-6-isopropoxyphenyl)-5',6',7',8'-tetrahy*o-3'-hydroxyspiro[3H-indole-3,6'-(1,2,4-triazolo[4,3-a]pyridine)]-2(1H)-one,
racemic (2'R, 3R, 4'S)-1'-hydroxycarbonylmethyl-6-chloro-4'-(3-chloro-phenyl)-2'-(3-fluoro-phenyl)spiro[3H-indole-3,3'-piperidine]-2(1H)-one,
racemic (5'R, 3R, 7's)-6-chloro-7'-(3-chlorophenyl)-5'-(1-methylene-butyl)-5',6',7',8'-tetrahydro-spiro[3H-indole-3,6'-(1,2,4-triazolo[4,3-a]pyridine)]-2(1H)-one,
chiral (5'R, 3R, 7'S)-6-chloro-7-(3-chlorophenyl)-5'-(1-methylene-butyl)-5',6',7',8'-tetrahydro-spiro [3H-indole-3,6'-(1,2,4-triazolo[4,3-a]pyridine)]-2(1H)-one,
racemic (5'R, 3R, 7'S)-6-chloro-7'-(3-chlorophenyl)-3'-methyl-5'-(1-methylene-butyl)-5',6',7,8'-tetrahydro-spiro[3H-indole-3,6'-(1,2,4-triazolo[4,3-a]pyridine)]-2(1H)-one,
racemic (2'R, 3'R,4'S)-6-chloro-4'-(3-chlorophenyl)-2'-(5-fluoro-2-methyl-phenyl)-6'-(1,4-piperazinyl)-spiro[3H-indole-3,3'-2',3',4',5'-tetrahydro-pyridine]-2-one and
racemic (2'R, 3'R,4'S)-6-chlozo-4'-(3-chlorophenzyl)-2'-(5-fluoro-2-methyl-phenyl)-6'-[4-[1,1-dimethylethoxy-carbonyl]amino]ethylamino-spiro[3H-indole-3,3'-2',3',4',5-tetrahydro-pyridine]-2-one.

11. A compound of claim 1 selected from the group consisting of
racemic (2'R, 3'R,4'S)-6-chloro-4'-(3-chlorophenyl)-2'-(5-fluoro-2-methyl-phenyl)-6'-[4-amino]ethylamino-spiro[3H-indole-3,3'-2',3',4',5'-tetrahydro-pyridine]-2-one, trifluoroacetic acid,
racemic (2'R, 3'R,4'S)-6-chloro-4'-(3-chlorophenyl)-2'-(5-fluoro-2-methyl-phenyl)-6'-(4-methyl-1-piperazinyl)-spiro[3H-indole-3,3'-2',3',4',5'-tetrahydro-pyridine]-2-one,
racemic (2'R, 3'R,4'S)-6-chloro-4'-(3-chlorophenyl)-2'-(5-fluoro-2-methyl-phenyl)-6'-[[tetrahydro-1,1-dioxido-2H-thiopyran-4-yl]piperazinyl-spiro[3H-indole-3,3'-2',3',4',5'-tetrahydro-pyridile]-2-one,
racemic (2'R, 3'R,4'S)-6-chloro-4'-(3-chlorophenyl)-2'-(5-fluoro-2-methyl-phenyl)-6'-[4-(3-methylsulphonyl)-propyl]piperazinyl-spiro[3H-indole-3,3'-2',3',4',5'-tetrahydro-pyridine]-2-one and
racemic (2'R, 3'R,4'S)-6-chloro-4'-(3-chlorophenyl)-2'-(5-fluoro-2-methyl-phenyl)-6'-[ 1-methylysulphonyl-4-piperidinyl]amino-spiro[3H-indole-3,3'-2',3',4',5'-tehahydro-pyridine]-2-one.

12. A pharmaceutical formulation comprising a compound of the formula 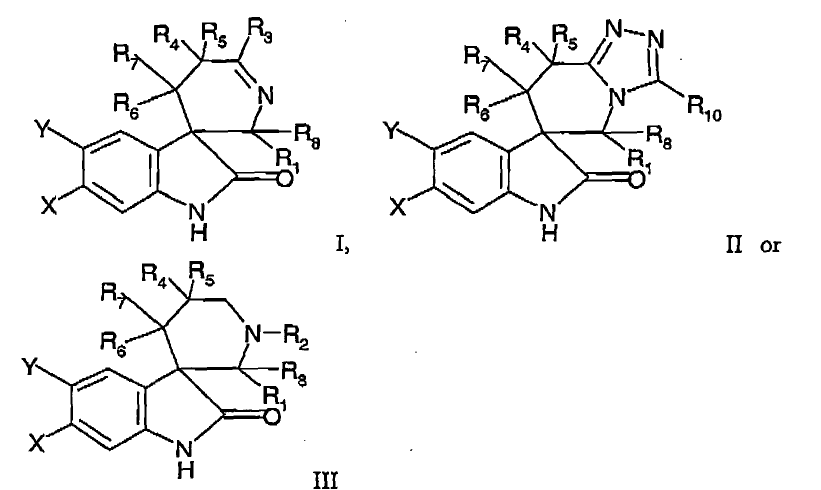 wherein
X is selected from the group consisting of hydrogen, halogen, cyano, nitro, ethynyl, cyclopropyl, methyl, ethyl, isopropyl, methoxy, and vinyl,
Y is hydrogen or fluorine,
R₄, R₅ are selected from the group consisting of hydrogen and C₁-C₆-alkyl one of R₁ and R₈ is selected from the group consisting of C₁-C₆-alkyl,
substituted C₁-C₆-alkyl, C₂-C₆-alkenyl, substituted C₂-C₆-alkenyl, aryl, substituted aryl, heteroaryl, substituted heteroaryl, heterocycle, substituted heterocycle, cycloalkyl,
substituted cycloalkyl, cycloalkenyl, and substituted cycloalkenyl and the other is hydrogen,
one of R₆ and R₇ is selected from the group consisting of C₁-C₆-alkyl, substituted C₁-₆-alkyl, C₂-C₆-akenyl, substituted C₂-C₆-alkenyl, aryl, substituted aryl, heteroaryl,
substituted heteroaryl, heterocycle, substituted heterocycle, cycloalkyl, substituted cycloalkyl, cycloalkenyl, and substituted cycloalkenyl and the other is hydrogen, C₁-C₆-alkyl or cyano
R₂ is selected from the group consisting of hydrogen, C₁-C₆-alkyl, substituted C₁-C₆-alkyl, C(=O) R₉, C(=O)NHR₉, C(=O)NR₉R_{9'} and C(=O)OR₉,
R₃ is selected from the group consisting of NHR₉, SR₉, and NR₉R_{9'},
R₉ is selected from the group consisting of C₁-C₆-alkyl, substituted C₁₋₄-alkyl, aryl,
substituted aryl, heteroaryl, substituted heteroaryl, heterocycle, substituted heterocycle, cycloalkyl and substituted cycloalkyl,
R₉ is selected from the group consisting of C₁-C₆-alkyl and substituted C₁-C₆-alkyl and in the case of R₉ and R_{9'} they may independently link to form a cyclic structure selected from heterocycle or substituted heterocycle,
R₁₀ is selected from the group consisting of hydrogen, hydroxyl and C₁-C₆-alkyl and the pharmaceutically acceptable salts and esters thereof together with a pharmaceutically acceptable carrier or excipient;
wherein
aryl is a monovalent, monocyclic or bicyclic, aromatic carbocyclic hydrocarbon radical; heteroaryl is an aromatic heterocyclic ring system containing up to two rings; and heterocycle is a substituted or unsubstituted 5 to 8 membered, mono- or bicyclic, aromatic or non-aromatic hydrocarbon, wherein 1 to 3 carbon atoms are replaced by a hetero atom selected from nitrogen,oxygen or sulfur atom.

13. The pharmaceutical formulation according to claim 12 for the treatment of cancer, in particular solid tumors, more particularly breast, colon, lung and prostate tumors.

14. The compounds according to claims 1 to 11 for the use as medicaments.

15. The compounds according to claims 1 to 11 for the use as medicaments for the treatment of cancer, in particular solid tumors, more particularly breast, colon, lung and prostate tumors.

16. The use of a compound according to claims 1 to 11 for the manufacture of medicaments for the treatment of cancer, in particular solid tumors, more particularly breast, colon, lung and prostate tumors.

17. A process to produce a compound of the formula 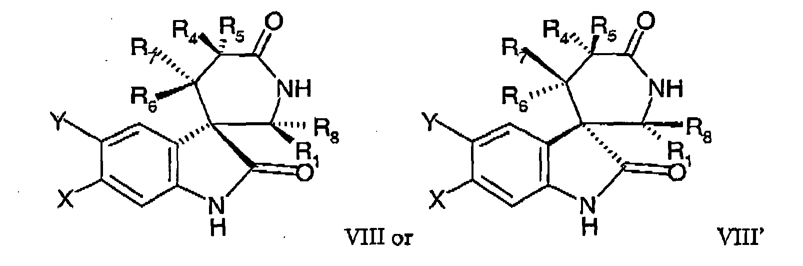 wherein
X is selected from the group consisting of hydrogen, halogen, cyano, nitro, cyclopropyl, methyl, ethyl, and isopropyl;
Y is hydrogen or fluorine;
R₁ is hydrogen;
R₄ and R₅ are hydrogen or C₁-C₆-alkyl;
R₈ is selected from the group consisting of C₁₋₄-alkyl, substituted C₁-C₆-alkyl,C₂-C₆-alkenyl, substituted C₂-C₆-alkenyl, aryl, substituted aryl, heteroaryl, substituted heteroaryl, heterocycle, substituted heterocycle, cycloalkyl, substituted cycloalkyl, cycloalkenyl, and substituted cycloalkenyl;
one of R₆ and R₇ are selected from the group consisting of C₁-C₆-alkyl, substituted C₁-C₆-alkyl, C₂-C₆-alkenyl, substituted C₂-C₆-alkenyl, aryl, substituted aryl, heteroaryl, substituted heteroaryl, heterocycle, substituted heterocycle, cycloalkyl, substituted cycloalkyl, cycloalkenyl, and substituted cycloalkenyl and the other is hydrogen, cyano or C₁-C₆-alkyl
which comprises reacting a compound of the formula 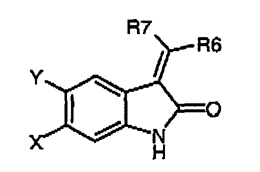 with a compound of the formula 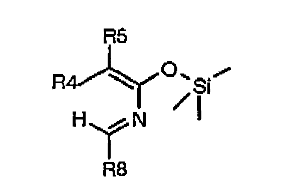
at about 110-160°C and under anhydrous conditions to produce a compound of formula V or V';
wherein
aryl is a monovalent, monocyclic or bicyclic, aromatic carbocyclic hydrocarbon radical heteroaryl is an aromatic heterocyclic ring system containing up to two rings; and heterocycle, is a substituted or unsubstituted 5 to 8 membered, mono- or bicyclic, aromatic or non-aromatic hydrocarbon, wherein 1 to 3 carbon atoms are replaced by a hetero atom selected from nitrogen,oxygen or sulfur atom.

18. A process to produce a compound of the formula 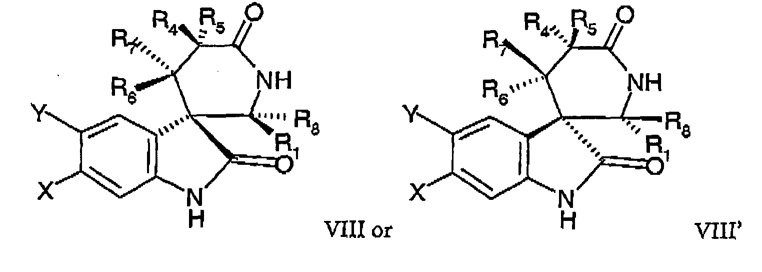 wherein
X is selected from the group consisting of hydrogen, halogen, cyano, nitro, cyclopropyl, methyl, ethyl, and isopropyl;
Y is hydrogen or fluorine;
R₁ is hydrogen;
R₄ and R₅ are hydrogen or C₁-C₆-alkyl;
R₈ is selected from the group consisting of C₁-C₆-alkyl, substituted C₁-C₆-alkyl, C₂-C₆-alkenyl, substituted C₂-C₆-alkenyl, aryl, substituted aryl, heteroaryl, substituted heteroaryl, heterocycle, substituted heterocycle, cycloalkyl, substituted cycloalkyl, cycloalkenyl, and substituted cycloalkenyl;
one of R₆ and R₇ are selected from the group consisting of C₁-C₆-alkyl, substituted C₁-C₆-alkyl, C₂-C₆-alkenyl, substituted C₂-C₆-alkenyl, aryl, substituted aryl, heteroaryl, substituted heteroaryl, heterocycle, substituted heterocycle, cycloalkyl, substituted cycloalkyl, cycloalkenyl, and substituted cycloalkenyl and the other is hydrogen, cyano or C₁-C₆-alkyl
which comprises reacting a compound of the formula 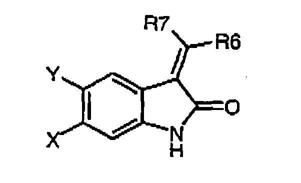 with a suitable protecting group (Pg) to provide a compound of the formula 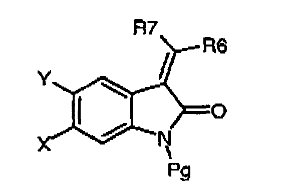 which is thereafter reacted with a compound of the formula 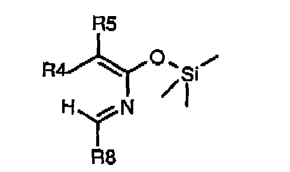
at about 110-160°C under anhydrous conditions and thereafter deprotecting the resultant product to produce a compound of formula V and V;
wherein
aryl is a monovalent, monocyclic or bicyclic, aromatic carbocyclic hydrocarbon radical; heteroaryl is an aromatic heterocyclic ring system containing up to two rings; and
heterocycle is a substituted or unsubstituted 5 to 8 membered, mono- or bicyclic, aromatic or non-aromatic hydrocarbon, wherein 1 to 3 carbon atoms are replaced by a hetero atom selected from nitrogen,oxygen or sulfur atom.

## Patentansprüche

1. Verbindung ausgewählt aus der Gruppe bestehend aus 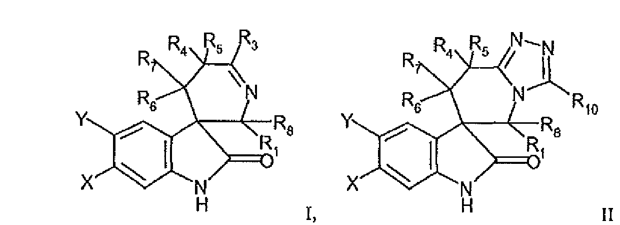 wobei
X ausgewählt ist aus der Gruppe bestehend aus Wasserstoff, Halogen, Cyano, Nitro, Ethinyl, Cyclopropyl, Methyl, Ethyl, Isopropyl, Methoxy und Vinyl,
Y Wasserstoff oder Fluor ist,
R₄, R₅ ausgewählt sind aus der Gruppe bestehend aus Wasserstoff und C₁-C₆-Alkyl, einer von R₁ und R₈ ausgewählt ist aus der Gruppe bestehend aus C₁-C₆-Alkyl, substituiertem C₁-C₆-Alkyl, C₂-C₆-Alkenyl, substituiertem C₂-C₆-Alkenyl, Aryl, substituiertem Aryl, Heteroaryl, substituiertem Heteroaryl, Heterocyclyl, substituiertem Heterocyclyl, Cycloalkyl, substituiertem Cycloalkyl, Cycloalkenyl und substituiertem Cycloalkenyl, und der andere Wasserstoff ist,
einer von R₆ und R₇ ausgewählt ist aus der Gruppe bestehend aus C₁-C₆-Alkyl, substituiertem C₃-C₆-Alkyl, C₂-C₆-Alkenyl, substituiertem C₂-C₆-Alkenyl, Aryl, substituiertem Aryl, Heteroaryl, substituiertem Heteroaryl, Heterocyclyl, substituiertem Heterocyclyl, Cycloalkyl, substituiertem Cycloalkyl, Cycloalkenyl und substituiertem Cycloalkenyl, und der andere Wasserstoff, C₁-C₆-Alkyl oder Cyano ist,
R₂ ausgewählt ist aus der Gruppe bestehend aus Wasserstoff, C₁-C₆-Alkyl, substituiertem C₁-C₆-Alkyl, C(=O)R₉, C(=O)NHR₉, C(=O)NR₉R₉ and C(=O)OR₉,
oder 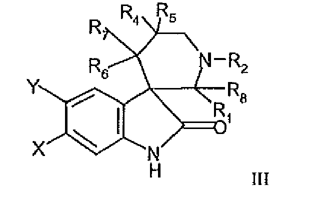 R₃ ausgewählt ist aus der Gruppe bestehend aus NHR₉, SR₉ und NR₉R₉,
R₉ ausgewählt ist aus der Gruppe bestehend aus C₁-C₆-Alkyl, substituiertem C₁-C₆-Alkyl, Aryl, substituiertem Aryl, Heteroaryl, substituiertem Heteroaryl, Heterocyclyl, substituiertem Heterocyclyl, Cycloalkyl und substituiertem Cycloalkyl,
Rg ausgewählt ist aus der Gruppe bestehend aus C₁-C₆-Alkyl und substituiertem C₁-C₆-Alkyl und in dem Fall von R₉ und R₉ können sie sich unabhängig verbinden, um eine zyklische Struktur ausgewählt aus Heterocyclyl oder substituiertem Heterocyclyl zu bilden, und
R₁₀ ausgewählt ist aus der Gruppe bestehend aus Wasserstoff, Hydroxyl und C₁-C₆-Alkyl, und pharmazeutisch akzeptable Salze und Ester davon;
wobei
Aryl ein monovalenter, monozyklischer oder bizyklischer, aromatischer carbozyklischer Kohlenwasserstoff-Rest ist;
Heteroaryl ein aromatisches heterozyklisches Ringsystem enthaltend bis zu zwei Ringe ist; und
Heterocyclyl ein substituierter oder unsubstituierter 5- bis 8-gliedriger, mono- oder bicyclischer, aromatischer oder nicht aromatischer Kohlenwasserstoff ist, wobei 1 bis 3 Kohlenstoffatome durch ein Heteroatom, ausgewählt aus einem Stickstoff-, Sauerstoff- oder Schwefelatom, ersetzt werden.

2. Verbindung nach Anspruch 1, wobei die Verbindung eine Verbindung der Formel 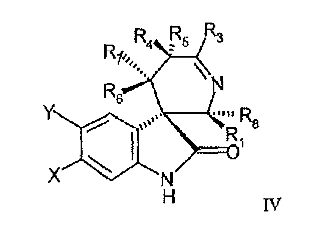 ist, wobei
X Cl oder Br ist,
Y Wasserstoff ist,
R₁ Wasserstoff ist,
sowohl R₄ als auch R₅ Wasserstoff sind,
R₆ Wasserstoff ist,
R₇ ein substituiertes Phenyl oder substituiertes Heteroaryl ist, ausgewählt aus der Gruppe bestehend aus 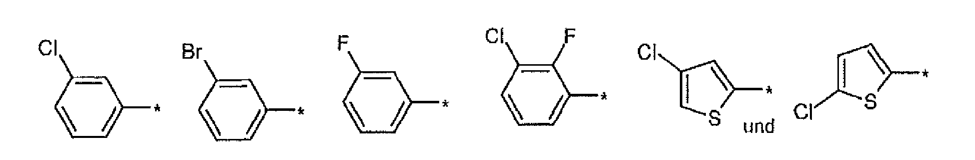 R₈ ausgewählt ist aus der Gruppe bestehend aus C₁-C₆-Alkyl, substituiertem C₁-C₆-Alkyl, C₂-C₆-Alkenyl, substituiertem C₂-C₆-Alkenyl, Aryl, substituiertem Aryl, Heteroaryl, substituiertem Heteroaryl, Cycloalkyl, substituiertem Cycloalkyl, Cycloalkenyl und substituiertem Cycloalkenyl,
R₃ ausgewählt ist aus der Gruppe bestehend aus NHR₉ und NR₉R₉,
R₉ ausgewählt ist aus der Gruppe bestehend aus C₁-C₆-Alkyl, substituiertem C₁-C₆-Alkyl, Aryl, substituiertem Aryl, Heteroaryl, substituiertem Heteroaryl, Heterocyclyl, substituiertem Heterocyclyl, Cycloalkyl und substituiertem Cycloalkyl,
Rg ausgewählt ist aus der Gruppe bestehend aus C₁-C₆-Alkyl und substituiertem C₁-C₆-Alkyl und in dem Fall von R₉ und R₉ können sie sich unabhängig verbinden, um eine zyklische Struktur, ausgewählt aus Heterocyclyl oder substituiertem Heterocyclyl, zu bilden,
und die pharmazeutisch akzeptablen Salze und Ester davon.

3. Verbindung nach Anspruch 1, wobei die Verbindung eine Verbindung der Formel 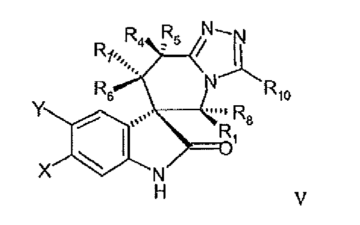 ist, wobei
X Cl oder Br ist,
Y Wasserstoff ist,
R₁ Wasserstoff ist,
sowohl R₄ als auch R₅ Wasserstoff sind, R₆ Wasserstoff ist,
R₇ ein substituiertes Phenyl oder substituiertes Heteroaryl ist, ausgewählt aus der Gruppe bestehend aus 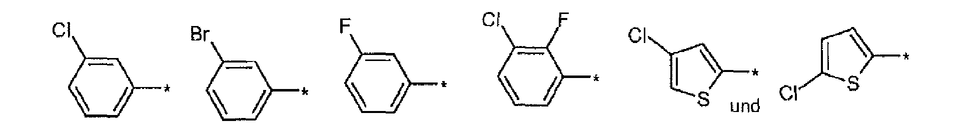 R₈ ausgewählt ist aus der Gruppe bestehend aus C₁-C₆-Alkyl, substituiertem C₁-C₆-Alkyl, C₂-C₆-Alkenyl, substituiertem C₂-C₆-Alkenyl, Aryl, substituiertem Aryl, Heteroaryl, substituiertem Heteroaryl, Cycloalkyl, substituiertem Cycloalkyl, Cycloalkenyl, und substituiertem Cycloalkenyl,
R₁₀ ausgewählt ist aus der Gruppe bestehend aus Wasserstoff, Hydroxyl und Methyl, und die pharmazeutisch akzeptablen Salze und Ester davon.

4. Verbindung nach Anspruch 1, wobei die Verbindung eine Verbindung der Formel 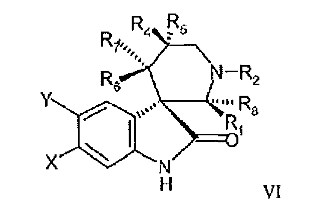 ist, wobei
X Cl oder Br ist,
Y Wasserstoff ist,
R₁ Wasserstoff ist,
sowohl R₄ als auch R₅ Wasserstoff sind,
R₆ Wasserstoff ist,
R₇ ein substituiertes Phenyl oder substituiertes Heteroaryl ist, ausgewählt aus der Gruppe bestehend aus 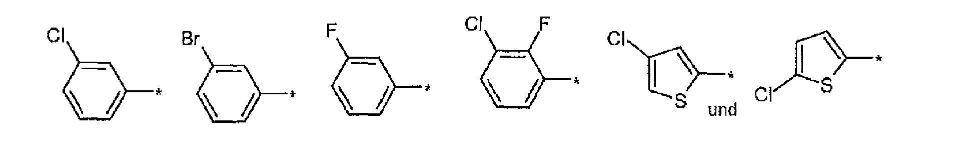 R₈ ausgewählt ist aus der Gruppe bestehend aus C₁-C₆-Alkyl, substituiertem Alkyl, C₂-C₆-Alkenyl, substituiertem C₂-C₆-Alkenyl, Aryl, substituiertem Aryl, Heteroaryl, substituiertem Heteroaryl, Cycloalkyl, substituiertem Cycloalkyl, Cycloalkenyl, und substituiertem Cycloalkenyl,
R₂ ausgewählt ist aus der Gruppe bestehend aus Wasserstoff, C₁-C₆-Alkyl, substituiertem C₁-C₆-Alkyl, C(=O)R₉, C(=O)NHR₉, C(=O)NR₉R₉ und C(=O)OR₉,
R₉ ausgewählt ist aus der Gruppe bestehend aus C₁-C₆-Alkyl, substituiertem C₁-C₆-Alkyl, Aryl, substituiertem Aryl, Heteroaryl, substituiertem Heteroaryl, Heterocyclyl, Cycloalkyl, und substituiertem Cycloalkyl,
R₉ ausgewählt ist aus der Gruppe bestehend aus C₁-C₆-Alkyl und substituiertem C₁-C₆-Alkyl und in dem Fall von R₉ und R₉ können sie sich unabhängig verbinden, um eine zyklische Struktur, ausgewählt aus Heterocyclyl und substituiertem Heterocyclyl zu bilden, und die pharmazeutisch akzeptable Salze und Ester davon.

5. Verbindung nach Anspruch 1, wobei die Verbindung eine Verbindung der Formel 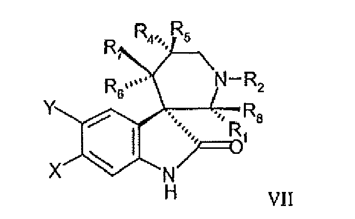 ist, wobei
X Cl oder Br ist,
Y Wasserstoff ist,
R₁ Wasserstoff ist,
sowohl R₄ als auch R₅ Wasserstoff sind,
R₆ Wasserstoff ist,
R₈ ein substituiertes Phenyl ist, ausgewählt aus der Gruppe bestehend aus 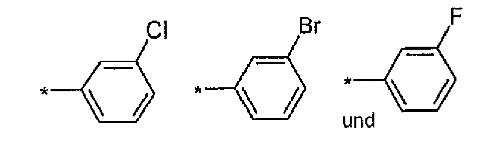 und
R₇ ausgewählt ist aus der Gruppe bestehend aus C₁C₆-Alkyl, substituiertem C₁-C₆-Alkyl, C₂-C₆-Alkenyl, substituiertem C₂-C₆-Alkenyl, Aryl, substituiertem Aryl, Heteroaryl,
substituiertem Heteroaryl, Heterocyclyl, substituiertem Heterocyclyl, Cycloalkyl, substituiertem Cycloalkyl, Cycloalkenyl und substituiertem Cycloalkenyl,
R₂ ausgewählt ist aus der Gruppe bestehend aus Wasserstoff, C₁-C₆-Alkyl, substituiertem C₁-C₆-Alkyl, C(=O)R₉, C(=O)NHR₉, C(=O)NR₉R₉, und C(=O)OR₉,
R₉ ausgewählt ist aus der Gruppe bestehend aus C₁-C₆-Alkyl, substituiertem C₁-C₆-Alkyl, Aryl, substituiertem Aryl, Heteroaryl, substituiertem Heteroaryl, Heterocyclyl, Cycloalkyl, und substituiertem Cycloalkyl,
R₉ ausgewählt ist aus der Gruppe bestehend aus C₁-C₆-Alkyl und substituiertem C₁-C₆-Alkyl und in dem Fall von R₉ und R₉ können sie sich unabhängig verbinden, um eine zyklische Struktur, ausgewählt aus Heterocyclyl und substituiertem Heterocyclyl, zu bilden, und die pharmazeutisch akzeptablen Salze und Ester davon.

6. Verbindung nach Anspruch 1 ausgewählt aus der Gruppe bestehend aus racemischem (2'S,3S,4'S)-6-Chlor-2'-(3-chlorphenyl)-4'-(2,2-dimethylpropyl) spiro[3H-indol-3,3'-piperidin]-2(1H)-on,
racemischem (2'S,3R)-6-Chlor-2'-(3-chlorphenyl)-2',3',4',5'-tetrahydro-4'-isopropyl-6'-(methylthio) spira[3H-indol-3,3'-pyridin]-2(1H)-on,
racemischem (2'S,3S,4'S)-6-Chlor-2'-(3-chlorphenyl)-4'-ethyl spiro[3H-indol-3,3'-piperidin]-2(1H)-on,
racemischem (2'R,3R,4'S)-6-Chlor-4'-(3-chlorphenyl)-2'-(3-methylphenyl) spiro[3H-indol-3,3'-piperidin]-2(1H)-on,
racemischem (2'R,3R,4'S)-6-Chlor-4'-(3-chlorphenyl)-2'-(3-fluorphenyl)spiro[3H-indol-3,3'-piperidin]-2(1H)-on,
racemischem (2'S,3S,4'R)-6-Chlor-2'-(3-chlorphenyl)-4'-phenyl spiro[3H-indol-3,3'-piperidin]-2(1H)-on,
racemischem (2'R,3R,4'S,5'S)-6-Chlor-4'-(3-chlorphenyl)-5'-methyl-2'(2-methylphenyl) spiro[3H-indol-3,3'-piperidin]-2(1H)-on,
racemischem (2'R,3R,4'S)-6-Chlor-4'-(3-chlorphenyl)-2'-(3-methoxyphenyl) spiro[3H-indol-3,3'-piperidin]-2(1H)-on,
racemischem (2'R,3R,4'S)-6-Chlor-4'-(3-chlorphenyl)-2'-(3-cyanophenyl)spiro[3H-indol-3,3'-piperidin]-2(1H)-on und
racemischem (2'R,3R,4'S)-6-Chlor-4'-(3-chlorphenyl)-2'-(2,3-dimethylphenyl) spiro[3H-indol-3,3'-piperidin]-2(1H)-on.

7. Verbindung nach Anspruch 1, ausgewählt aus der Gruppe bestehend aus racemischem (2'R,3R,4'S)-5-Chlor-4'-(3-chlorphenyl)-2'-[2-(trifluormethyl)-phenyl)] spiro[3H-indol-3,3'-piperidin]-2(1H)-on,
racemischem (2'S,3R)-8-Chlor-4'-(3-chlorphenyl)-2',3',4',5'-tetrahydro-6'-(methylthio)-2'-[2-(trifluormethyl)-phenyl] spiro[3H-indol-3,3'-pyridin]-2(1H)-on,
racemischem (2'R,3R,4'S)-6-Chlor-4'-(3-chlorphenyl)-2'-[5-fluor-2-(trifluoromethyl)- phenyl] spiro[3H-indol-3,3'-piperidin]-2(1H)-on,
racemischem (2'R,3R,4'S)-6-Chlor-4'-(3-chlorphenyl)-2'-(5-fluor-2-methylphenyl) spiro[3H-indol-3,3'-piperidin]-2(1H)-on,
racemischem (2'R,3R,4'S)-6-Chlor-4'-(3-chlorphenyl)-2'-(2,4-difluorphenyl) spiro[3H-indol-3,3'-piperidin]-2(1H)-on,
racemischem (2'R,3R,4'S)-6-Chlor-4'-(3-chlorphenyl)-2'-(5-fluor-2-methoxyphenyl) spiro[3H-indol-3,3'-piperidin]-2(1H)-on,
racemischem (2'R,3R,4'S)-6-Chlor-4'-(3-chlorphenyl)-2'-(1-naphthalenyl) spiro[3H-indol-3,3'-piperidin]-2(1H)-on,
racemischem (2'R,3R,4'S)-6-Chlor-4'-(3-chlorphenyl)-2'-(3-pyridinyl) spiro[3H-indol-3,3'-piperidin]-2(1H)-on,
racemischem (2'R,3R,4'S)-6-Chlor-4'-(3-chlorphenyl)-2'-(3,4-difluorphenyl) spiro[3H-indol-3,3'-piperidin]-2(1 H)-on und
racemischem (5'R,3R,7'S)-6-Chlor-7'-(3-chlorphenyl)-5'-(3,4-difluorphenyl)-5',6',7',8'-tetrahydro-3'-hydroxyspiro[3H-indol-3,6'-(1,2,4-triazolo[4,3-a]pyridin)]-2(1H)-on.

8. Verbindung nach Anspruch 1, ausgewählt aus der Gruppe bestehend aus
racemischem (5'R,3R,7'S)-6-Chlor-7'-(3-chlorphenyl)-5'-(5-fluor-2-methylphenyl)-5',6',7',8'-tetrahydro-3'-hydroxyspiro[3H-indol-3,6'-(1,2,4-triazolo[4,3-a]pyridin)]-2(1H)-on,
racemischem (2'R,3R,4'S)-6-Chlor-4'-(3-chlorphenyl)-2'-(2,3-difluor-6-methylphenyl) spiro[3H-indol-3,3'-piperidin]-2(1H)-on,
racemischem (2'R,3R,4'S)-6-Chlor-4'-(3-chlorphenyl)-5-fluor-2'-(5-fluor-2-methylphenyl) spiro[3H-indol-3,3'-piperidin]-2(1H)-on,
racemischem (2'R,3R,4'S)-6-Chlor-4'-(3-chlorphenyl)-2'-(1-ethylpropyl) spiro [3H-indol-3,3'-piperidin]-2(1H)-on,
racemischem (2'R,3R,4'S)-6-Chlor-4'-(3-chlorphenyl)-2'-(2,5-dimethylphenyl) spiro[3H-indol-3,3'-piperidin]-2(1H)-on,
racemischem (2'R,3R,4'S)-6-Chlor-4'-(3-chlorphenyl)-2'-(2,5-dimethyl-2H-pyrazol-3-yl) spiro[3H-indol-3,3'-piperidin]-2(1H)-on,
racemischem (2'R,3R,4'S)-6-Chlor-4'-(3-chlorphenyl)-1'-(morpholin-4-carbonyl)-2'-(1-naphthalenyl) spiro[3H-indol-3,3'-piperidin]-2(1H)-on,
racemischem (2'S,3S,4'R)-6-Chlor-4'-(4-chlorphenyl)-2'-(3-fluorphenyl)spiro[3H-indol-3,3'-piperidin]-2(1H)-on,
racemischem (2S,3S,4'R)-1'-Tert-butylaminocarbonyl-6-chlor-4'-(4-chlorphenyl)-2'-(3-fluorphenyl) spiro[3H-indol-3,3'-piperidin]-2(1H)-on und
racemischem (2'S,3S,4'R)-6-Chlor-4'-(4-chlorphenyl)-1'-(3-cyanophenylaminocarbonyl)-2'-(3-fluorphenyl) spiro[3H-indol-3,3'-piperidin]-2(1H)-on.

9. Verbindung nach Anspruch 1, ausgewählt aus der Gruppe bestehend aus racemischem (2'R,3R,4'S)-6-Chlor-4'-(3-chlorphenyl)-1'-(3-cyanophenylaminocarbonyl)-2'-(3-fluorphenyl) spiro[3H-indol-3,3'-piperidin]-2(1H)-on,
racemischem (2'R,3R,4'S)-1'-Tert-butylaminocarbonyl-6-chloro-4'-(3-chlorphenyl)-2'-(3-fluorphenyl) spiro[3H-indol-3,3'-piperidin]-2(1H)-on,
racemischem (2'R,3R,4'S)-1'-Benzoyl-6-chlor-4'-(3-chlorphenyl)-2'-(3-fluorphenyl) spiro[3H-indol-3,3'-piperidin]-2(1H)-on,
racemischem (2'R,3R,4'S)-1'-Acetyl-6-chlor-4'-(3-chlorphenyl)-2'-(3-fluorphenyl) spiro[3H-indol-3,3'-piperidin]-2(1H)-on,
racemischem (2'R,3R,4'S)-1'-[4-(Aminocarbonylmethyl)-piperazin-1-carbonyl]-6-chlor-4'-(3-chlorphenyl)-2'-(3-fluorphenyl) spiro[3H-indol-3,3'-piperidin]-2(1H)-on,
racemischem (2'R,3R,4'S)-6-Chlor-4'-(3-chlorphenyl)-2'-(3-fluorphenyl)-1'-[4-(3-methansulfonylpropyl)-piperazin-1-carbonyl] spiro[3H-indol-3,3'-piperidin]-2(1H)-on,
racemischem (2'R,3R,4'S)-6-Chlor-4'-(3-chlorphenyl)-2'-(3-fluorphenyl)-1'-[4-(2-oxo-2-pyrrolidin-1-yl-ethyl)-piperazin-1-carbonyl] spiro[3H-indol-3,3'-piperidin]-2(1H)-on,
racemischem (2'R,3R,4'S)-1'-[4-(2-Acetylaminoethyl)-piperazin-1-carbonyl]-6-chlor-4'-(3-chlorphenyl)-2'-(3-fluorphenyl) spiro[3H-indol-3,3'-piperidin]-2(1H)-on und
racemischem (2'R,3R, 4'S)-6-Chlor-4'-(3-chlorphenyl)-2'-(3-fluorphenyl)-1'-[4-(2-hydroxyethyl)-piperazin-1-carbonyl] spiro[3H-indol-3,3'-piperidin]-2(1H)-on.

10. Verbindung nach Anspruch 1, ausgewählt aus der Gruppe bestehend aus racemischem (2'R,3R,4'S)-6-Chlor-4'-(3-chlorphenyl)-1-cyclohexylaminocarbonyl-2'-(3-fluorphenyl) spiro[3H-indol-3,3'-piperidin]-2(1H)-on,
racemischem (2'R,3R,4'S)-1'-benzylaminocarbonyl-6-chlor-4'-(3-chlorphenyl)-2'-(3-fluorphenyl) spiro[3H-indol-3,3'-piperidin]-2(1H)-on,
racemischem (5'R,3R,7'S)-6-Chloro'-(3-chlorphenyl)-5'(2,3-difluor-6-isopropoxy-phenyl)-5',6',7',8'-tetrahydro-3'-hydroxyspiro[3H-indol-3,6'-(1,2,4-triazolo[4,3-a]pyridin)]-2(1H)-on, chiralem (5'R,3R,7'S)-6-Chloro-7'-(3-chlorphenyl)-5'(2,3-difluor-6-isopropoxy-phenyl)-5',6',7',8'-tetrahydro-3'-hydroxyspiro[3H-indol-3,6'-(1,2,4-triazolo[4,3-a]pyridiny]-2(1H)-on,
racemischem (2'R,3R,4'S)-1'-Hydroxycarbonylmethyl-6-chlor-4'-(3-chlorphenyl)-2'-(3-fluorophenyl) spiro[3H-indol-3,3'-piperidin]-2(1H)-on,
racemischem (5'R,3R,7'S)-6-Chlor-7'-(3-chlorphenyl)-5'-(1-methylenbutyl)-5',6',7',8'-tetrahydro-spiro[3H-indol-3,6'-(1,2,4-triazolo[4,3-a]pyridin)]-2(1H)-on,
chiralem (5'R,3R,7'S)-6-Chlor-7'-(3-chlorphenyl)-5'-(1-methylenbutyl)-5',6',7',8'-tetrahydro-spiro[3H-indol-3,6'-(1,2,4-triazolo[4,3-a]pyridin)]-2(1H)-on,
racemischem (5'R,3R,7'S)-6-Chlor-7'-(3-chlorphenyl)-3'-methyl-5'-(1-methylenbutyl)-5',6',7',8'-tetrahydro-spiro[3H-indol-3,6'-(1,2,4-triazolo[4,3-a]pyridin)]-2(1H)-on,
racemischem (2'R,3'R,4'S)-6-Chlor-4'-(3-chlorphenyl)-2'-(5-fluor-2-methylphenyl)-6'-(1,4-piperazinyl)-spiro[3H-indol-3,3'-2',3',4',5'-tetrahydropyridin]-2-on und
racemischem (2'R,3'R,4'S)-6-Chlor-4'-(3-chlorphenyl)-2'-(5-fluor-2-methylphenyl)-6'-[4-[1,1-dimethylethoxycarbonyl]amino]ethylamino-spiro[3H-indol-3,3'-2',3',4',5'-tetrahydropyridin]-2-on.

11. Verbindung nach Anspruch 1, ausgewählt aus der Gruppe bestehend aus
racemischem (2'R,3'R,4'S)-6-Chlor-4'-(3-chlorphenyl)-2'-(5-fluor-2-methylphenyl)-6'-[4-amino]ethylamino-spiro[3H-indol-3,3'-2',3',4',5'-tetrahydro-pyridin]-2-on, Trifluoressigsäure,
racemischem (2'R,3'R,4'S)-6-Chlor-4'-(3-chlorphenyl)-2'-(5-fluoro-2-methyl-phenyl)-6'-(4-methyl-1-piperazinyl)-spiro[3H-indol-3,3'-2',3',4',5'-tetrahydropyridin]-2-on,
racemischem (2'R,3'R,4'S)-6-Chlor-4'-(3-chlorphenyl)-2'-(5-fluor-2-methylphenyl)-6'-[[tetrahydro-1,1-dioxido-2H-thiopyran-4-yl]piperazinyl-spiro[3H-indol-3,3'-2',3',4',5'-tetrahydropyridin]-2-on,
racemischem (2'R,3'R,4'S)-6-Chlor-4'-(3-chlorphenyl)-2'-(5-fluor-2-methylphenyl)-6'-[4-(3-methylsulphonyl)-propyl]piperazinyl-spiro[3H-indol-3,3'-2',3',4',5'-tetrahydropyridin]-2-on und
racemischem (2'R,3'R,4'S)-6-Chlor-4'-(3-chlorphenyl)-2'-(5-fluor-2-methylphenyl)-6'-[1-methylsulphonyl-4-piperidinyl]amino-spiro[3H-indol-3,3'-2',3',4',5'-tetrahydro-pyridin]-2-on.

12. Pharmazeutische Formulierung umfassend eine Verbindung der Formel 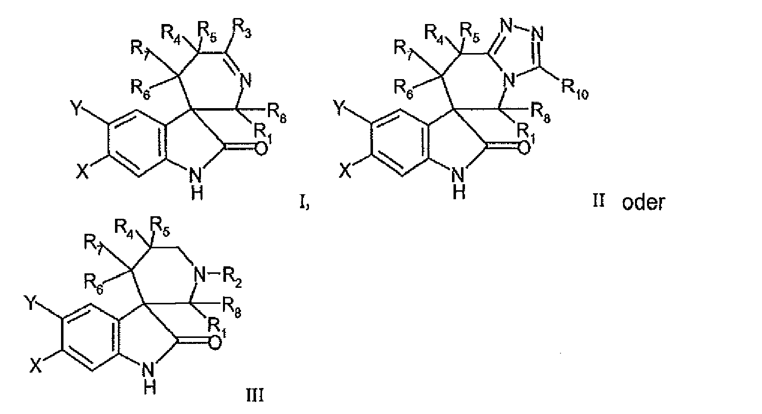 wobei
X ausgewählt ist aus der Gruppe bestehend aus Wasserstoff, Halogen, Cyano, Nitro, Ethinyl, Cyclopropyl, Methyl, Ethyl, Isopropyl, Methoxy und Vinyl,
Y Wasserstoff oder Fluor ist,
R₄, R₅ ausgewählt sind aus der Gruppe bestehend aus Wasserstoff und C₁-C₆-Alkyl, einer von R₁ und R₈ ausgewählt ist aus der Gruppe bestehend aus C₁-C₆-Alkyl, substituiertem C₁-C₆-Alkyl, C₂-C₆-Alkenyl, substituiertem C₂-C₆-Alkenyl, Aryl, substituiertem Aryl, Heteroaryl, substituiertem Heteroaryl, Heterocyclyl, substituiertem Heterocyclyl, Cycloalkyl, substituiertem Cycloalkyl, Cycloalkenyl und substituiertem Cycloalkenyl, und der andere Wasserstoff ist,
einer von R₆ und R₇ ausgewählt ist aus der Gruppe bestehend aus C₁-C₆-Alkyl, substituiertem C₁-C₆-Alkyl, C₂-C₆-Alkenyl, substituiertem C₂-C₆-Alkenyl, Aryl, substituiertem Aryl, Heteroaryl, substituiertem Heteroaryl, Heterocyclyl, substituiertem Heterocyclyl, Cycloalkyl, substituiertem Cycloalkyl, Cycloalkenyl und substituiertem Cycloalkenyl, und der andere Wasserstoff, C₁-C₆-Alkyl oder Cyano ist,
R₂ ausgewählt ist aus der Gruppe bestehend aus Wasserstoff, C₁-C₆-Alkyl, substituiertem C₁-C₆-Alkyl, C(=O)R₉, C(=O)NHR₉, C(=O)NR₉R₉ and C(=O)OR₉,
R₃ ausgewählt ist aus der Gruppe bestehend aus NHR₉, SR₉ und NR₉R₉,
R₉ ausgewählt ist aus der Gruppe bestehend aus C₁-C₆-Alkyl, substituiertem C₁-C₆-Alkyl, Aryl, substituiertem Aryl, Heteroaryl, substituiertem Heteroaryl, Heterocyclyl, substituiertem Heterocyclyl, Cycloalkyl und substituiertem Cycloalkyl,
R₉ ausgewählt ist aus der Gruppe bestehend aus C₁-C₆-Alkyl und substituiertem C₁-C₆-Alkyl und in dem Fall von R₉ und R₉ können sie sich unabhängig verbinden, um eine zyklische Struktur ausgewählt aus Heterocyclyl oder substituiertem Heterocyclyl zu bilden,
R₁₀ ausgewählt ist aus der Gruppe bestehend aus Wasserstoff, Hydroxyl und C₁-C₆-Alkyl, und die pharmazeutisch akzeptablen Salze und Ester davon, zusammen mit einem pharmazeutisch akzeptablen Träger oder Hilfsstoff,
wobei
Aryl ein monovalenter, monozyklischer oder bizyklischer, aromatischer carbozyklischer Kohlenwasserstoff-Rest ist;
Heteroaryl ein aromatisches heterozyklisches Ringsystem enthaltend bis zu zwei Ringe ist; und
Heterocyclyl ein substituierter oder unsubstituierter 5- bis 8-gliedriger, mono- oder bicyclischer, aromatischer oder nicht aromatischer Kohlenwasserstoff ist, wobei 1 bis 3 Kohlenstoffatome durch ein Heteroatom, ausgewählt aus einem Stickstoff-, Sauerstoff- oder Schwefelatom, ersetzt werden.

13. Pharmazeutische Formulierung nach Anspruch 12 zur Behandlung von Krebs, insbesondere festen Tumoren, stärker bevorzugt Brust-, Dickdarm-, Lungen- und Prostatatumoren.

14. Verbindungen nach Anspruch 1 bis 11 zur Verwendung als Medikamente.

15. Verbindungen nach den Ansprüchen 1 bis 11 zur Verwendung als Medikamente zur Behandlung von Krebs, Insbesondere soliden Tumoren, stärker bevorzugt Brust-, Dickdarm-, Lungen- und Prostatatumoren.

16. Verwendung einer Verbindung nach den Ansprüchen 1 bis 11 zur Herstellung eines Medikaments zur Behandlung von Krebs, Insbesondere soliden Tumoren, stärker bevorzugt Brust-, Dickdarm-, Lungen- und Prostatatumoren.

17. Verfahren zur Herstellung einer Verbindung der Formel 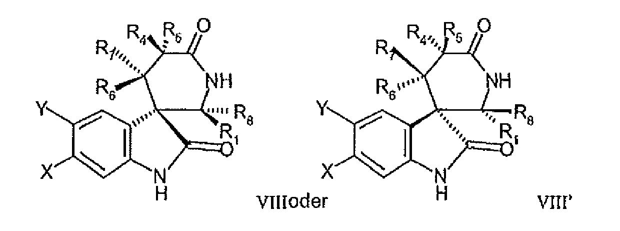 wobei X ausgewählt ist aus der Gruppe bestehend aus Wasserstoff, Halogen, Cyano, Nitro, Cyclopropyl, Methyl, Ethyl und Isopropyl;
Y Wasserstoff oder Fluor ist;
R₁ Wasserstoff ist;
R₄ und R₅ Wasserstoff oder C₁-C₆-Alkyl sind;
R₈ ausgewählt ist aus der Gruppe bestehend aus C₁-C₈-Alkyl, substituiertem C₁-C₆-Alkyl, C₂-C₆-Alkenyl, substituiertem C₂-C₆-Alkenyl, Aryl, substituiertem Aryl, Heteroaryl, substituiertem Heteroaryl, Heterocyclyl, substituiertem Heterocyclyl, Cycloalkyl, substituiertem Cycloalkyl, Cycloalkenyl, und substituiertem Cycloalkenyl;
einer von R₆ und R₇ ausgewählt ist aus der Gruppe bestehend aus C₁-C₆-Alkyl, substituiertem C₁-C₆-Alkyl, C₂-C₆-Alkenyl, substituiertem C₂-C₆-Alkenyl, Aryl, substituiertem Aryl, Heteroaryl, substituiertem Heteroaryl, Heterocyclyl, substituiertem Heterocyclyl, Cycloalkyl, substituiertem Cycloalkyl, Cycloalkenyl und substituiertem Cycloalkenyl, und der andere Wasserstoff, Cyano oder C₁-C₆-Alkyl ist,
welches das Umsetzen einer Verbindung der Formel 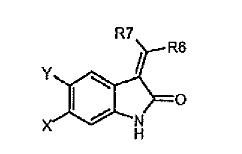 mit einer Verbindung der Formel 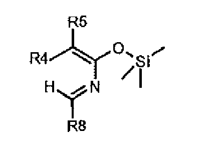 bei etwa 110-160°C und unter wasserfreien Bedingungen umfasst, um eine Verbindung der Formel V oder V' herzustellen;
wobei
Aryl ein monovalenter, monozyklischer oder bizyklischer, aromatischer carbozyklischer Kohlenwasserstoff-Rest ist;
Heteroaryl ein aromatisches heterozyklisches Ringsystem enthaltend bis zu zwei Ringe ist; und
Heterocyclyl ein substituierter oder unsubstituierter 5- bis 8-gliedriger, mono- oder bicyclischer, aromatischer oder nicht aromatischer Kohlenwasserstoff ist, wobei 1 bis 3 Kohlenstoffatome durch ein Heteroatom, ausgewählt aus einem Stickstoff-, Sauerstoff- oder Schwefelatom, ersetzt werden.

18. Verfahren zur Herstellung einer Verbindung der Formel 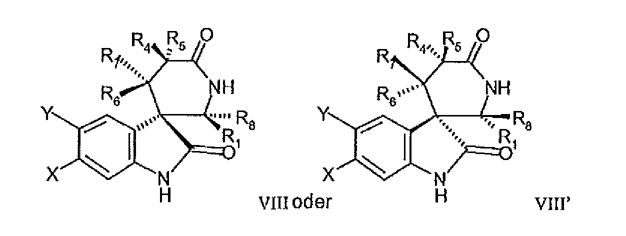 wobei
X ausgewählt ist aus der Gruppe bestehend aus Wasserstoff, Halogen, Cyano, Nitro, Cyclopropyl, Methyl, Ethyl und Isopropyl;
Y Wasserstoff oder Fluor ist;
R₁ Wasserstoff ist;
R₄ und R₅ Wasserstoff oder C₁-C₆-Alkyl sind;
R₈ ausgewählt ist aus der Gruppe bestehend aus C₁-C₆-Alkyl, substituiertem C₁-C₆-Alkyl, C₂-C₆-Alkenyl, substituiertem C₂-C₆-Alkenyl, Aryl, substituiertem Aryl, Heteroaryl, substituiertem Heteroaryl, Heterocyclyl, substituiertem Heterocyclyl, Cycloalkyl, substituiertem Cycloalkyl, Cycloalkenyl, und substituiertem Cycloalkenyl;
einer von R₆ und R₇ ausgewählt ist aus der Gruppe bestehend aus C₁-C₆-Alkyl, substituiertem C₁-C₆-Alkyl, C₂-C₆-Alkenyl, substituiertem C₂-C₈-Alkenyl, Aryl, substituiertem Aryl, Heteroaryl, substituiertem Heteroaryl, Heterocyclyl, substituiertem Heterocyclyl, Cycloalkyl, substituiertem Cycloalkyl, Cycloalkenyl und substituiertem Cycloalkenyl, und der andere Wasserstoff, Cyano oder C₁-C₆-Alkyl ist,
welches das Umsetzen einer Verbindung der Formel 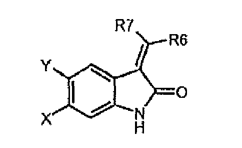 mit einer geeigneten Schutzgruppe (Pg) umfasst, um eine Verbindung der Formel 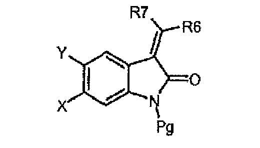 bereitzustellen,
die danach mit einer Verbindung der Formel 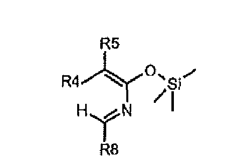 bei etwa 110-160°C unter wasserfreien Bedingungen umgesetzt wird, und anschließendes Entschützen des resultierenden Produkts, um eine Verbindung der Formel V und V' herzustellen;
wobei
Aryl ein monovalenter, monozyklischer oder bizyklischer, aromatischer carbozyklischer Kohlenwasserstoff-Rest ist;
Heteroaryl ein aromatisches heterozyklisches Ringsystem enthaltend bis zu zwei Ringe ist; und
Heterocyclyl ein substituierter oder unsubstituierter 5- bis 8-gliedriger, mono- oder bicyclischer, aromatischer oder nicht aromatischer Kohlenwasserstoff ist, wobei 1 bis 3 Kohlenstoffatome durch ein Heteroatom, ausgewählt aus einem Stickstoff-, Sauerstoff- oder Schwefelatom, ersetzt werden.

## Revendications

1. Composé choisi dans le groupe constitué par: 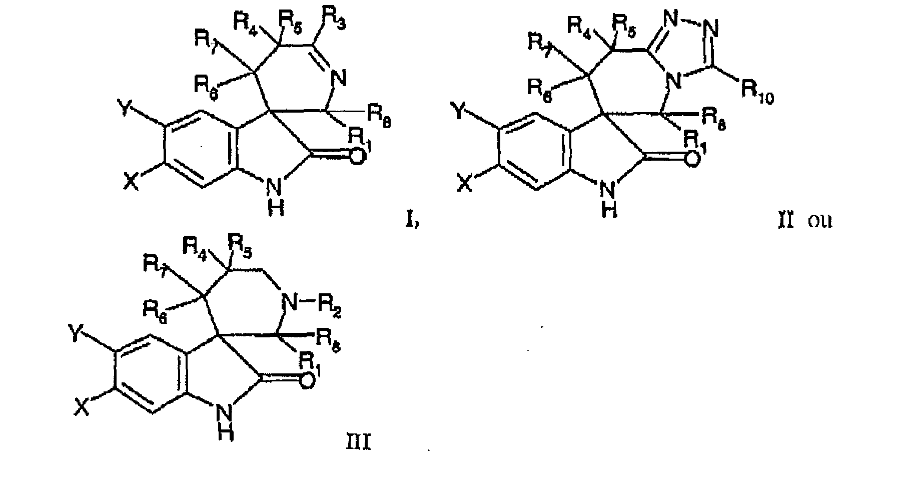 où
X est choisi dans le groupe constitué par l'hydrogène, halogène, cyano, nitro, éthynyle, cyclopropyle, méthyle, éthyle, isopropyle, méthoxy et vinyle,
Y est l'hydrogène ou le fluor,
R₄, R₅ sont choisis dans le groupe constitué par l'hydrogène et alkyle en C₁-C₆, l'un des R₁ et R₈ est choisi dans le groupe constitué par alkyle en C₁-C₆, alkyle en C₁-C₆ substitué, alcényle en C₂-C₆, alcényle en C₂-C₆ substitué, aryle, aryle substitué, hétéroaryle, hétéroaryle substitué, hétérocycle, hétérocycle substitué, cycloalkyle, cycloalkyle substitué, cycloalcényle et cycloalcényle substitué, et l'autre est l'hydrogène,
l'un des R₆ et R₇ est choisi dans le groupe constitué par alkyle en C₁-C₆, alkyle en C₁-C₆ substitué, alcényle en C₂-C₆, alcényle en C₂-C₆ substitué, aryle, aryle substitué, hétéroaryle, hétéroaryle substitué, hétérocycle, hétérocycle substitué, cycloalkyle, cycloalkyle substitué, cycloalcényle, et cycloalcényle substitué et l'autre est l'hydrogène, alkyle en C₁-C₆ ou cyano,
R₂ est choisi dans le groupe constitué par l'hydrogène, alkyle en C₁-C₆, alkyle en C₁-C₆ substitué, C(=O)R₉, C(=O)NHR₉, C(=O)NR₉R₉ et C(=O)OR₉,
R₃ est choisi dans le groupe constitué par NHR₉, SR₉, et NR₉R_{9'},
R₉ est choisi dans le groupe constitué par alkyle en C₁-C₆, alkyle en C₁-C₆ substitué, aryle, aryle substitué, hétéroaryle, hétéroaryle substitué, hétérocycle, hétérocycle substitué, cycloalkyle et cycloalkyle substitué,
R_{9'} est choisi dans le groupe constitué par alkyle en C₁-C₆ et alkyle en C₁-C₆ substitué, et, dans le cas de R₉ et R_{9'}, ils peuvent se lier indépendamment pour former une structure cyclique choisie parmi un hétérocycle et un hétérocycle substitué, et
R₁₀ est choisi dans le groupe constitué par l'hydrogène, hydroxyle et alkyle en C₁-C₆,
et ses sels et esters pharmaceutiquement acceptables;
où
aryle est un radical hydrocarboné carbocyclique aromatique, monocyclique ou bicyclique, monovalent,
hétéroaryle est un système cyclique hétérocyclique aromatique contenant jusqu'à deux cycles; et
hétérocycle est un hydrocarbure aromatique ou non aromatique, mono- ou bicyclique de 5 à 8 chaînons, substitué ou non substitué, dans lequel 1 à 3 atomes de carbone sont remplacés par un hétéroatome choisi parmi les atomes d'azote, d'oxygène ou de soufre.

2. Composé selon la revendication 1, ledit composé étant un composé de formule: 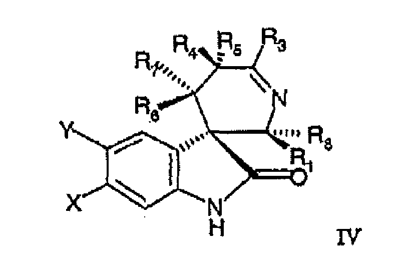 où
X est Cl ou Br,
X xest l'hydrogène,
R₁ est l'hydrogène,
R₄ et R₅ sont tous les deux l'hydrogène,
R₆ est l'hydrogène,
R₇ est un phényle substitué ou un hétéroaiyle substitué choisis dans le groupe constitué par 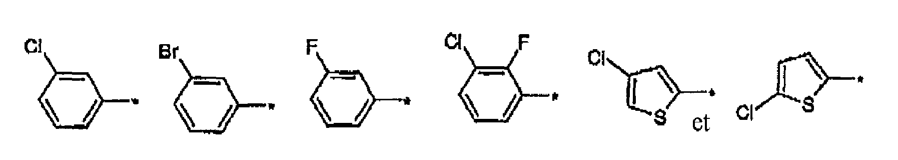
R₈ est choisi dans le groupe constitué par alkyle en C₁-C₆, alkyle en C₁-C₆ substitué, alcényle en C₂-C₆, alcényle en C₂-C₆ substitué, aryle, aryle substitué, hétéroaryle, hétéroaryle substitué, cycloalkyle, cycloalkyle substitué, cycloalcényle, et cycloalcényle substitué,
R₃ est choisi dans le groupe constitué par NHR₉ et NR₉R_{9'},
R₉ est choisi dans le groupe constitué par alkyle en C₁-C₆, alkyle en C₁-C₆ substitué, aryle, aryle substitué, hétéroaryle, hétéroaryle substitué, hétérocycle, hétérocycle substitué, cycloalkyle et cycloalkyle substitué,
R_{9'} est choisi dans le groupe constitué par alkyle en C₁-C₆ et alkyle en C₁-C₆ substitué, et, dans le cas de R₉ et R_{9'}, ils peuvent se lier indépendamment pour former une structure cyclique choisie parmi un hétérocycle et un hétérocycle substitué,
et ses sels et esters pharmaceutiquement acceptables.

3. Composé selon la revendication 1, ledit composé étant un composé de formule: 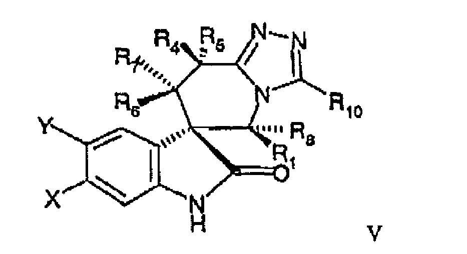 où
X est Cl ou Br,
Y est l'hydrogène,
R₁ est l'hydrogène,
R₄ et R₅ sont tous les deux l'hydrogène,
R₆ est l'hydrogène,
R₇ est un phényle substitué ou un hétéroaryle substitué choisis dans le groupe constitué par 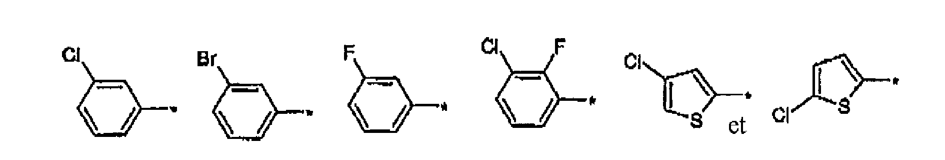
R₈ est choisi dans le groupe constitué par alkyle en C₁-C₆, alkyle en C₁-C₆ substitué, alcényle en C₂-C₆, alcényle en C₂-C₆ substitué, aryle, aryle substitué, hétéroaryle, hétéroaryle substitué, cycloalkyle, cycloalkyle substitué, cycloalcényle, et cycloalcényle substitué,
R₁₀ est choisi dans le groupe constitué par l'hydrogène, hydroxyle et méthyle,
et ses sels et esters pharmaceutiquement acceptables.

4. Composé selon la revendication 1, ledit composé étant un composé de formule: 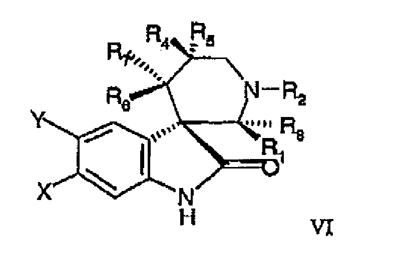 où
X est Cl ou Br,
Y est l'hydrogène,
R₁ est l'hydrogène,
R₄ et R₅ sont tous les deux l'hydrogène,
R₆ est l'hydrogène,
R₇ est un phényle substitué ou un hétéroaryle substitué choisis dans le groupe constitué par 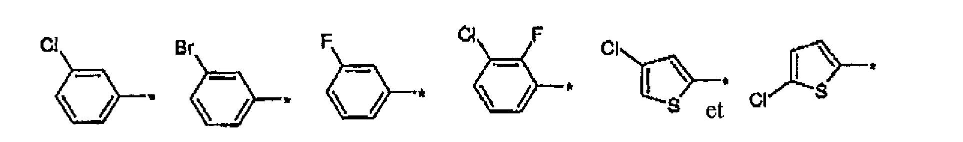
R₈ est choisi dans le groupe constitué par alkyle en C₁-C₆, alkyle en C₁-C₆ substitué, alcényle en C₂-C₆, alcényle en C₂-C₆ substitué, aryle, aryle substitué, hétéroaryle, hétéroaryle substitué, cycloalkyle, cycloalkyle substitué, cycloalcényle et cyeloalcényle substitué,
R₂ est choisi dans le groupe constitué par l'hydrogène, alkyle en C₁-C₆, alkyle en C₁-C₆ substitué, C(=O)R₉, C(=O)NHR₉, C(=O)NR₉R_{9'}, et C(=O)OR₉,
R₉ est choisi dans le groupe constitué par alkyle en C₁-C₆, alkyle en C₁-C₆ substitué, aryle, aryle substitué, hétéroaryle, hétéroaryle substitué, hétérocycle, cycloalkyle et cycloalkyle substitué,
R_{9'} est choisi dans le groupe constitué par alkyle en C₁-C₆ et alkyle en C₁-C₆ substitué, et, dans le cas de R₉ et R_{9'}, ils peuvent se lier indépendamment pour former une structure cyclique choisie parmi un hétérocycle et un hétérocycle substitué,
et ses sels et esters pharmaceutiquement acceptables.

5. Composé selon la revendication 1, ledit composé étant un composé de formule: 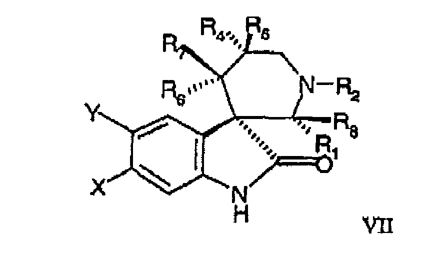 où
X est Cl ou Br,
Y est l'hydrogène,
R₁ est l'hydrogène,
R₄ et R₅ sont tous les deux l'hydrogène,
R₆ est l'hydrogène,
R₈ est un phényle substitué choisi dans le groupe constitué par 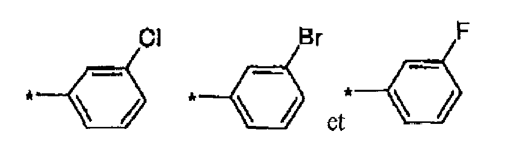 et
R₇ est choisi dans le groupe constitué par alkyle en C₁-C_{6,} alkyle en C₁-C₆ substitué, alcényle en C₂-C₆, alcényle en C₂-C₆ substitué, aryle, aryle substitué, hétéroaryle, hétéroaryle substitué, hétérocycle, hétérocycle substitué, cycloalkyl, cycloalkyle substitué, cycloalcényle et cycloalcényle substitué,
R₂ est choisi dans le groupe constitué par l'hydrogène, alkyle en C₁-C₆, alkyle en C₁-C₆ substitué, C(=O)R₉, C(=O)NHR₉, C(=O)NR₉R_{9'}, et C(=O)OR₉,
R₉ est choisi dans le groupe constitué par alkyle en C₁-C₆, alkyle en C₁-C₆ substitué, aryle, aryle substitué, hétéroaryle, hétéroaryle substitué, hétérocycle, cycloalkyle et cycloalkyle substitué,
R_{9'} est choisi dans le groupe constitué par alkyle en C₁-C₆ et alkyle en C₁-C₆ substitué, et, dans le cas de R₉ et R_{9'}, ils peuvent se lier indépendamment pour former une structure cyclique choisie parmi un hétérocycle et un hétérocycle substitué,
et ses sels et esters pharmaceutiquement acceptables.

6. Composé selon la revendication 1, choisi dans le groupe constitué par:
la (2'S, 3S, 4'S)-6-chloro-2'-(3-chlorophényl)-4'-(2,2-diméthylpropyl)spiro[3H-indole-3,3'-pipéridine]-2(1H)-one racémique,
la (2'S, 3R)-6-chloro-2'-(3-chlorophényl)-2',3',4',5'-tétrahydro-4'-isopropyl-6'-(méthylthio)spiro[3H-indole-3,3'-pyridine]-2(1H)-one racémique,
la (2'S, 3S, 4'S)-6-chloro-2'-(3-chlorophényl)-4'-éthylspiro[3H-indole-3,3'-pipéridine]-2(1H)-one racémique,
la (2'R, 3R, 4'S)-6-chloro-4'-(3-chlorophényl)-2'-(3-métylphényl)spiro[3H-indole-3,3'-pipéridine]-2(1H)-one racémique,
la (2'R, 3R, 4'S)-6-chloro-4'-(3-chlorophényl)-2'-(3-fluorophényl)spiro[3H-indole-3,3'-pipéridine]-2(1H)-one racémique,
la (2'S, 3S, 4'R)-6-chloro-2'-(3-chlorophényl)-4'-phénylspiro[3H-indole-3,3'-pipéridine]-2(1H)-one racémique,
la (2'R, 3R, 4'S, 5'S)-6-chloro-4'-(3-chlorophényl)-5'-méthyl-2'-(2-méthylphényl)-spiro[3H-indole-3,3'-pipéridine]-2(1H)-one racémique,
la (2'R, 3R, 4'S)-6-chloro-4'-(3-chlorophényl)-2'-(3-méthoxyphényl)spiro[3H-indole-3,3'-pipéridine]-2(1H)-one racémique,
la (2'R, 3R, 4'S)-6-chloro-4'-(3-chlorophényl)-2'-(3-cyanophényl)spiro[3H-indole-3,3'-pipéridine]-2(1H)-one racémique, et
la (2'R, 3R, 4'S)-6-chloro-4'-(3-chlorophényl)-2'-(2,3-diméthylphényl)spiro[3H-indole-3,3'-pipéridine]-2(1H)-one racémique.

7. Composé selon la revendication 1, choisi dans le groupe constitué par:
la (2'R, 3R, 4'S)-6-chloro-4'-(3-chlorophényl)-2'-[2-(trifluoroinéthyl)phényl]-spiro[3H-indole-3,3'-pipéridine]-2(1H)-one racémique,
la (2'S, 3R)-6-chloro-4'-(3-chlorophényl)-2',3',4',5'-tétrahydro-6'-(méthylthio)-2'-[2-(trifluorométhyl)phényl]spiro[3H-indole-3,3'-pyridine]-2(1H)-one racémique,
la (2'R, 3R, 4'S)-6-chloro-4'-(3-chlorophényl)-2'-[5-fluoro-2-(trifluorométhyl)-phényl]spiro[3H-indole-3,3'-pipéridine]-2(1H)-one racémique,
la (2'R, 3R, 4'S)-6-chloro-4'-(3-chlorophényl)-2'-(5-fluoro-2-méthylphényl)spiro[3H-indole-3,3'-pipéridine]-2(1H)-one racémique,
la (2'R, 3R, 4'S)-6-chloro-4'-(3-chlorophényl)-2'-(2,4-difluorophényl)spiro[3H-indole-3,3'-pipéridine]-2(1H)-one racémique,
la (2'R, 3R, 4'S)-6-chloro-4'-(3-chlorophényl)-2'-(5-fluoro-2-méthoxyphényl)spiro-[3H-indole-3,3'-pipéridine]-2(1H)-one racémique,
la (2'R, 3R, 4'S)-6-chloro-4'-(3-chlorophényl)-2'-(1-naphtalényl)spino[3H-indole-3,3'-pipéridine]-2(1H)-one racémique,
la (2'R, 3R, 4'S)-6-chloro-4'-(3-chlorophényl)-2'-(3-pyridinyl)spiro[3H-indole-3,3'-pipéridine]-2(1H)-one racémique,
la (2'R, 3R, 4'S)-6-chloro-4'-(3-chlorophényl)-2'-(3,4-difluorophényl)spiro[3H-indole-3,3'-pipéridine]-2(1H)-one racémique, et
la (5'R, 3R, 7'S)-6-chloro-7'-(3-chlorophényl)-5'-(3,4-difluorophényl)-5',6',7',8'-tétrahydro-3'-hydroxyspiro[3H-indole-3,6'-(1,2,4-triazolo[4,3-a]pyridine)]-2(H)-one racémique.

8. Composé selon la revendication 1, choisi dans le groupe constitué par:
la (5'R, 3R, 7'S)-6-chloro-7'-(3-chlorophényl)-5'-(5-fluoro-2-méthylphényl)-5',6',7',8'-tétrahydo-3'-hydroxyspino[3H-indole-3,6'-(1,2,4-triazolo[4,3-a]pyridine)]-2(1H)-one racémique,
la (2'R, 3R, 4'S)-6-chloro-4'-(3-chlorophényl)-2'-(2,3-difluoro-6-méthylphényl)spiro-[3H-indole-3,3'-pipéridine]-2(1H)-one racémique,
la (2'R, 3R, 4'S)-6-chloro-4'-(3-chlorophényl)-5-fluoro-2'-(5-fluoro-2'-méthylphényl)-spiro[3H-indole-3,3'-pipéridine]-2(1H)-one racémique,
la (2'R, 3R, 4'S)-6-chloro-4'-(3-chlorophényl)-2'-(1-éthylpropyl)spiro[3H-indole-3,3'-pipéridine]-2(1H)-one racémique,
la (2'R, 3R, 4'S)-6-chloro-4'-(3-chlorophényl)-2'-(2,5-diméthylphényl)spiro[3H-indole-3,3'-pipéridine]-2(1H)-one racémique,
la (2'R, 3R, 4'S)-6-chloro-4'-(3-chlorophényl)-2'-(2,5-diméthyl-2H-pyrazol-3-yl)-spiro[3H-indole-3,3'-pipéridine]-2(H)-one racémique,
la (2'R, 3R, 4'S)-6-chloro-4'-(3-chlorophényl)-1'-(morpholine-4-carbonyl)-2'-(1-naphtalényl)spiro[3H-indole-3,3'-pipéridine]-2(1H)-one racémique,
la (2'S, 3S, 4'R)-6-chloro-4'-(4-chlorophényl)-2'-(3-fluorophényl)spiro[3H-indole-3,3'-pipéridine]-2(1H)-one racémique,
la (2'S, 3S, 4'R)-1'-tert-butylaminocarbonyl-6-chloro-4'-(4-chlorophényl)-2'-(3-fluorophényl)spiro[3H-indole-3,3'-pipéridine]-2(1H)-one racémique, et
la (2'S, 3S, 4'R)-6-chloro-4'-(4-chlorophényl)-1'-(3-cyanophénylaminocarbonyl)-2'-(3-fluorophényl)spiro[3H-indole-3,3'-pipéridine]-2(1H)-one racémique.

9. Composé selon la revendication 1, choisi dans le groupe constitué par:
la (2'R, 3R, 4'S)-6-chloro-4'-(3-chlorophényl)-1'-(3-cyanophénylaminocarbonyl)-2'-(3-fluorophényl)spiro[3H-indole-3,3'-pipéridine]-2(1H)-one racémique,
la (2'R, 3R, 4'S)-1'-tert-butylaminocarbonyl-6-chloro-4'-(3-chlorophényl)-2'-(3-fluorophényl)spiro[3H-indole-3,3'-pipéridine]-2(1H)-one racémique,
la (2'R, 3R, 4'S)-1'-benzoyl-6-chloro-4'-(3-chlorophényl)-2'-(3-fluorophényl)spiro-[3H-indole-3,3'-pipéridine]-2(1H)-one racémique,
la (2'R, 3R, 4S)-1'-acétyl-6-chloro-4'-(3-chlorophényl)-2-(3-fluorophényl)spiro[3H-indole-3,3'-pipéridine]-2(1H)-one racémique,
la (2'R, 3R, 4'S)-1'-[4-(aminocarbonylmétyl)pipérazine-1-carbonyl]-6-chloro-4'-(3-chlorophényl)-2'-(3-fluorophényl)spiro[3H-indole-3,3'-pipéridine]-2(1H)-one racémique,
la (2'R, 3R, 4'S)-6-chloro-4'-(3-chlorophényl)-2'-(3-fluorophényl)-1'-[4-(3-méthanesulfonylpropyl)pipérazine-1-carbonyl]spiro[3H-indole-3,3'-pipéridine]-2(1H)-one racémique,
la (2'R, 3R, 4'S)-6-chloro-4'-(3-chlorophényl)-2'-(3-fluorophényl)-1'-[4-(2-oxo-2-pyrrolidin-1-yléthyl)pipérazine-1-carbonyl]spiro[3H-indole-3,3'-pipéridine]-2(1H)-one racémique,
la (2'R, 3R, 4'S)-1'-[4-(2-acétylaminoéthyl)pipérazine-1-carbanyl]-6-chloro-4'-(3-chlorophényl)-2'-(3-fluorophényl)spiro[3H-indole-3,3`-pipéridine]-2(1H)-one racémique, et
la (2'R, 3R, 4'S)-6-chloro-4'-(3-chlorophényl)-2'-(3-fluorophényl)-1'-[4-(2-hydroxyéthyl)pipérazine-1-carbonyl]spiro[3H-indole-3,3'-pipéridine]-2(1H)-one racémique.

10. Composé selon la revendication 1, choisi dans le groupe constitué par:
la (2'R, 3R, 4'S)-6-chloro-4'-(3-chlorophényl)-1'-cyclohexylaminocarbonyl-2'-(3-fluorophényl)spiro[3H-indole-3,3'-pipéridine]-2(1H)-one racémique,
la (2'R, 3R, 4'S)-1'-benzylaminocarbonyl-6-chloro-4'-(3-chlorophényl)-2'-(3-fluorophényl)spiro[3H-indole-3,3'-pipéridine]-2(1H)-one racémique,
la (5'R, 3R, 7'S)-6-chloro-7'-(3-chlorophényl)-5'-(2,3-difluoro-6-isopropoxyphényl)-5',6',7',8'-tétrahydro-3'-hydroxyspiro[3H-indole-3,6'-(1,2,4-triazolo[4,3-a]pyridine)]-2(1H)-one racémique,
la (5'R, 3R, 7'S)-6-chloro-7'-(3-chlorophényl)-5'-(2,3-difluoro-6-isopropoxyphényl)-5',6',7',8'-tétrahydro-3'-hydroxyspiro[3H-indole-3,6'-(1,2,4-triazolo[4,3-a]pyridine)]-2(1H)-one chirale,
la (2'R, 3R, 4'S)-1'-hydroxycarbonylméthyl-6-chloro-4'-(3-chlorophényl)-2'-(3-fluorophényl)spiro[3H-indole-3,3'-pipéridine]-2(1H)-one racémique,
la (5'R, 3R, 7'S)-6-chloro-7'-(3-chlorophényl)-5'-(1-méthylénebutyl)-5',6',7',8'-tétrahydrospiro[3H-indole-3,6'-(1,2,4-triazolo[4,3-a]pyridine)]-2(1H)-one racémique,
la (5'R, 3R, 7'S)-6-chloro-7'-(3-chlorophényl)-5'-(1-méthylènebutyl)-5',6',7',8'-tétrahydrospiro[3H-indole-3,6'-(1,2,4-triazolo[4,3-a]pyridine)]-2(1H)-one chirale,
la (5'R, 3R, 7'S)-6-chloro-7'-(3-chlorophényl)-3'-méthyl-5'-(1-méthylénebutyl)-5',6',7',8'-tétrahydrospiro[3H-indole-3,6'-(1,2,4-triazolo[4,3-a]pyridine)]-2(1H)-one racémique,
la (2'R, 3'R, 4'S)-6-chloro-4'-(3-chlorophényl)-2'-(5-fluoro-2-méthylphényl)-6'-(1,4-pipérazinyl)spiro[3H-indole-3,3'-2',3',4',5'-tétrahydropyridine]-2-one racémique, et
la (2'R, 3'R, 4S)-6-chloro-4'-(3-chlorophényl)-2'-(5-fluoro-2-métylphényl)-6'-[4-(1,1-diméthyléthoxycarbonyl)amino]éthylaminospiro[3H-indole-3,3'-2',3',4',5'-tétrahydropyridine]-2-one racémique.

11. Composé selon la revendication 1, choisi dans le groupe constitué par:
la (2'R, 3'R, 4'S)-6-chloro-4'-(3-chlorophényl)-2'-(5-fluoro-2-méthylphényl)-6'-[4-amino]éthylaminospiro[3H-indole-3,3'-2',3',4',5'-tétrahydropyridine]-2-one racémique, sel d'acide trifluoroacétique,
la (2'R, 3'R, 4'S)-6-chloro-4'-(3-chlorophényl)-2'-(5-fluoro-2-méthylphényl)-6'-(4-méthyl-1-pipérazinyl)spiro[3H-indole-3,3'-2',3',4',5'-tétrahydropyridine]-2-one racémique,
la (2'R, 3'R, 4'S)-6-chloro-4'-(3-chlorophényl)-2'-(5-fluoro-2-méthylphényl)-6'-[[tétrahydro-1,1-dioxydo-2H-thiopyran-4-yl]pipérazinyl]spiro[3H-indole-3,3'-2',3',4',5'-tétrahydropyridine]-2-one racémique,
la (2'R, 3'R, 4'S)-6-chloro-4'-(3-chlorophényl)-2'-(5-fluoro-2-méthylphényl)-6'-[4-(3-méthylsulfonyl)propyl]pipérazinylspiro[3H-indole-3,3'-2',3',4',5'-tétrahydropyridine]-2-one racémique, et
la (2'R, 3'R, 4'S)-6-chloro-4'-(3-chlorophényl)-2'-(5-fluoro-2-méthylphényl)-6'-[1-méthylsulfonyl-4-pipéridinyl]spiro[3H-indole-3,3'-2',3',4',5'-tétrahydropyridine]-2-one racémique.

12. Formulation pharmaceutique comprenant un composé de formule 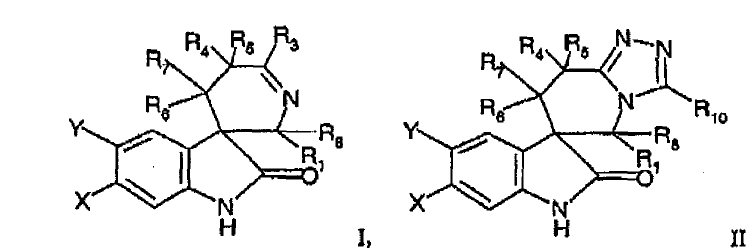 ou 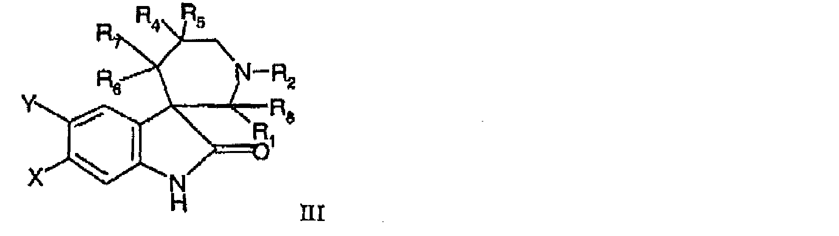 où
X est choisi dans le groupe constitué par l'hydrogène, halogène, cyano, nitro, éthynyle, cyclopropyle, méthyle, éthyle, isopropyle, méthoxy et vinyle,
Y est l'hydrogène ou le fluor,
R₄, R₅ sont choisis dans le groupe constitué par l'hydrogène et alkyle en C₁-C₆,
l'un des R₁ et R₈ est choisi dans le groupe constitué par alkyle en C₁-C₆, alkyle en C₁-C₆ substitué, alcényle en C₂-C₆, alcényle en C₂-C₆ substitué, aryle, aryle substitué, hétéroaryle, hétéroaryle substitué, hétérocycle, hétérocycle substitué, cycloalkyle, cycloalkyle substitué, cycloalcényle et cycloalcényle substitué, et l'autre est l'hydrogène,
l'un des R₆ et R₇ est choisi dans le groupe constitué par alkyle en C₁-C₆, alkyle en C₁-C₆ substitué, alcényle en C₂-C₆, alcényle en C₂-C₆ substitué, aryle, aryle substitué, hétéroaryle, hétéroaryle substitué, hétérocycle, hétérocycle substitué, cycloalkyle, cycloalkyle substitué, cycloalcényle et cycloalcényle substitué, et l'autre est l'hydrogène, alkyle en C₁-C₆ ou cyano,
R₂ est choisi dans le groupe constitué par l'hydrogène, alkyle en C₁-C₆, alkyle en C₁-C₆ substitué, C(=O)R₉, C(=O)NHR₉, C(=O)NR₉R_{9'} et C(=O)OR₉,
R₃ est choisi dans le groupe constitué par NHR₉, SR₉, et NR₉R_{9'},
R₉ est choisi dans le groupe constitué par alkyle en C₁-C₆, alkyle en C₁-C₆ substitué, aryle, aryle substitué, hétéroaryle, hétéroaryle substitué, hétérocycle, hétérocycle substitué, cycloalkyle et cycloalkyle substitué,
R_{9'} est choisi dans le groupe constitué par alkyle en C₁-C₆ et alkyle en C₁-C₆ substitué, et, dans le cas de R₉ et R_{9'}, ils peuvent se lier indépendamment pour former une structure cyclique choisie parmi un hétérocycle et un hétérocycle substitué, et
₁₀ R est choisi dans le groupe constitué par l'hydrogène, hydroxyle et alkyle en C₁-C₆,
et ses sels et esters pharmaceutiquement acceptables, avec un support ou excipient pharmaceutiquement acceptable;
où
aryle est un radical hydrocarboné carbocyclique aromatique, monocyclique ou bicyclique, monovalent,
hétéroaryle est un système cyclique hétérocyclique aromatique contenant jusqu'à deux cycles;
et hétérocycle est un hydrocarbure aromatique ou non aromatique, mono- ou bicyclique de 5 à 8 chaînons, substitué ou non substitué, dans lequel 1 à 3 atomes de carbone sont remplacés par un hétéroatome choisi parmi les atomes d'azote, d'oxygène ou de soufre.

13. Formulation pharmaceutique selon la revendication 12, destinée au traitement du cancer, en particulier de tumeurs solides, plus particulièrement de tumeurs du sein, du côlon, du poumon et de la prostate.

14. Composés selon les revendications 1 à 11, à utiliser comme médicaments.

15. Composés selon les revendications 1 à 11, à utiliser comme médicaments pour le traitement du cancer, en particulier de tumeurs solides, plus particulièrement de tumeurs du sein, du côlon, du poumon et de la prostate.

16. Utilisation d'un composé selon les revendications 1 à 11 pour la fabrication de médicaments destinés au traitement du cancer, en particulier de tumeurs solides, plus particulièrement de tumeurs du sein, du côlon, du poumon et de la prostate.

17. Procédé de production d'un composé de formule 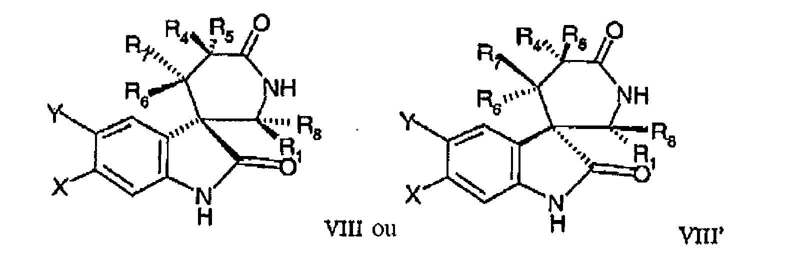 où
X est choisi dans le groupe constitué par l'hydrogène, halogène, cyano, nitro, cyclopropyle, méthyle, éthyle et isopropyle;
Y est l'hydrogène ou le fluor;
R₁ est l'hydrogène;
R₄ et R₅ sont l'hydrogène ou un alkyle en C₁-C₆,
R₈ est choisi dans le groupe constitué par alkyle en C₁-C₆, alkyle en C₁-C₆ substitué, alcényle en C₂-C₆, alcényle en C₂-C₆ substitué, aryle, aryle substitué, hétéroaryle, hétéroaryle substitué, hétérocycle, hétérocycle substitué, cycloalkyle, cycloalkyle substitué, cycloalcényle et cycloalcényle substitué;
l'un des R₆ et R₇ est choisi dans le groupe constitué par alkyle en C₁-C₆, alkyle en C₁-C₆ substitué, alcényle en C₂-C₆, alcényle en C₂-C₆ substitué, aryle, aryle substitué, hétéroaryle, hétéroaryle substitué, hétérocycle, hétérocycle substitué, cycloalkyle, cycloalkyle substitué, cycloalcényle et cycloalcényle substitué, et l'autre est l'hydrogène, cyano ou alkyle en C₁-C₆,
qui comprend la réaction d'un composé de formule 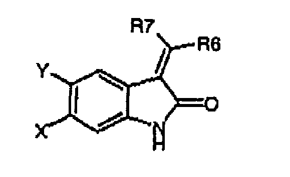 avec un composé de formule 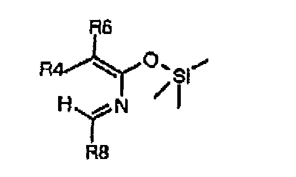 à environ 110-160°C et dans des conditions anhydres pour la production d'un composé de formule V ou V';
où
aryle est un radical hydrocarboné carbocyclique aromatique, monocyclique ou bicyclique, monovalent,
hétéroaiyle est un système cyclique hétérocyclique aromatique contenant jusqu'à deux cycles; et
hétérocycle est un hydrocarbure aromatique ou non aromatique, mono- ou bicyclique de 5 à 8 chaînons, substitué ou non substitué, dans lequel 1 à 3 atomes de carbone sont remplacés par un hétéroatome choisi parmi les atomes d'azote, d'oxygène ou de soufre.

18. Procédé de production d'un composé de formule 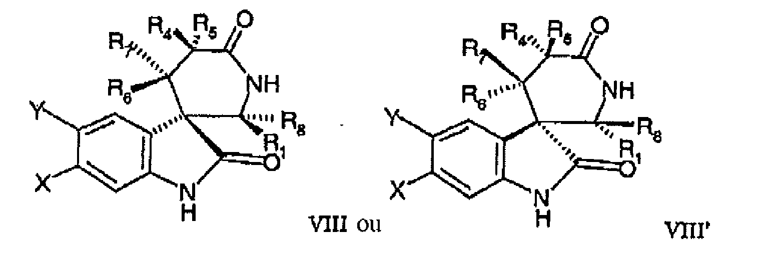 où
X est choisi dans le groupe constitué par l'hydrogène, halogène, cyano, nitro, cyclopropyle, méthyle, éthyle et isopropyle;
Y est l'hydrogène ou le fluor;
R₁ est l'hydrogène;
R₄ et R₅ sont l'hydrogène ou un alkyle en C₁-C₆,
R₈ est choisi dans le groupe constitué par alkyle en C₁-C₆₎, alkyle en C₁-C₆ substitué, alcényle en C₂-C₆, alcényle en C₂-C₆ substitué, aryle, aryle substitué, hétéroaryle, hétéroaryle substitué, hétérocycle, hétérocycle substitué, cycloalkyle, cycloalkyle substitué, cycloalcényle et cycloalcényle substitué;
l'un des R₆ et R₇ est choisi dans le groupe constitué par alkyle en C₁-C₆), alkyle en C₁-C₆ substitué, alcényle en C₂-C₆, alcényle en C₂-C₆ substitué, aryle, aryle substitué, hétéroaryle, hétéroaryle substitué, hétérocycle, hétérocycle substitué, cycloalkyle, cycloalkyle substitué, cycloalcényle et cycloalcényle substitué, et l'autre est l'hydrogène, cyano ou alkyle en C₁-C₆,
qui comprend la réaction d'un composé de formule 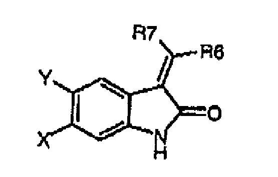 avec un groupe protecteur (Pg) approprié, donnant un composé de formule 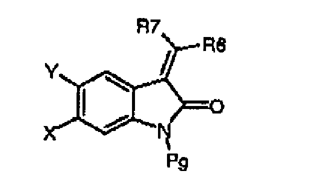 que l'on fait ensuite réagir avec un composé de formule 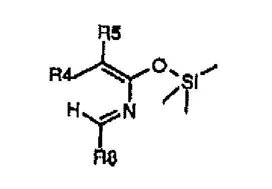 à environ 110-160°C et dans des conditions anhydres, puis on déprotège le produit obtenu pour produire un composé de formule V ou V';
où
aryle est un radical hydrocarboné carbocyclique aromatique, monocyclique ou bicyclique, monovalent,
hétéroaryle est un système cyclique hétérocyclique aromatique contenant jusqu'à deux cycles; et
hétérocycle est un hydrocarbure aromatique ou non aromatique, mono- ou bicyclique de 5 à 8 chaînons, substitué ou non substitué, dans lequel 1 à 3 atomes de carbone sont remplacés par un hétéroatome choisi parmi les atomes d'azote, d'oxygène ou de soufre.
